(19) Europäisches Patentamt
European Patent Office
Office européen des brevets

(11) EP 3 698 631 A2

(12) # EUROPEAN PATENT APPLICATION

(43) Date of publication:
**26.08.2020 Bulletin 2020/35**

(21) Application number: **20150010.5**

(22) Date of filing: **05.05.2010**

(51) Int Cl.:
*A01N 43/04* (2006.01) *A61K 31/70* (2006.01)
*A61K 9/127* (2006.01) *A61K 31/713* (2006.01)
*A61K 47/69* (2017.01) *A61P 19/02* (2006.01)
*A61P 43/00* (2006.01) *A61P 37/02* (2006.01)
*A61P 29/00* (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority:
**05.05.2009 US 17577709 P**
**14.08.2009 US 23404509 P**
**15.09.2009 US 24276109 P**
**15.10.2009 US 25199109 P**
**06.11.2009 US 25884809 P**

(62) Document number(s) of the earlier application(s) in accordance with Art. 76 EPC:
**18203606.1 / 3 504 967**
**10772775.2 / 2 416 652**

(71) Applicant: **Arbutus Biopharma Corporation**
**Burnaby, BC V5J 5J8 (CA)**

(72) Inventors:
- **NOVOBRANTSEVA, Tatiana**
  **Cambridge, MA Massachusetts 02142 (US)**
- **AKINC, Akin**
  **Cambridge, MA Massachusetts 02142 (US)**
- **SUGO, Tsukasa**
  **Cambridge, MA Massachusetts 02142 (US)**

(74) Representative: **Müller-Boré & Partner Patentanwälte PartG mbB**
**Friedenheimer Brücke 21**
**80639 München (DE)**

Remarks:
•This application was filed on 02.01.2020 as a divisional application to the application mentioned under INID code 62.
•Claims filed after the date of receipt of the divisional application (Rule 68(4) EPC).

(54) **METHODS OF DELIVERING OLIGONUCLEOTIDES TO IMMUNE CELLS**

(57) The invention relates to the field of delivery of nucleic acid-based agents to immune cells.

FIG. 1A

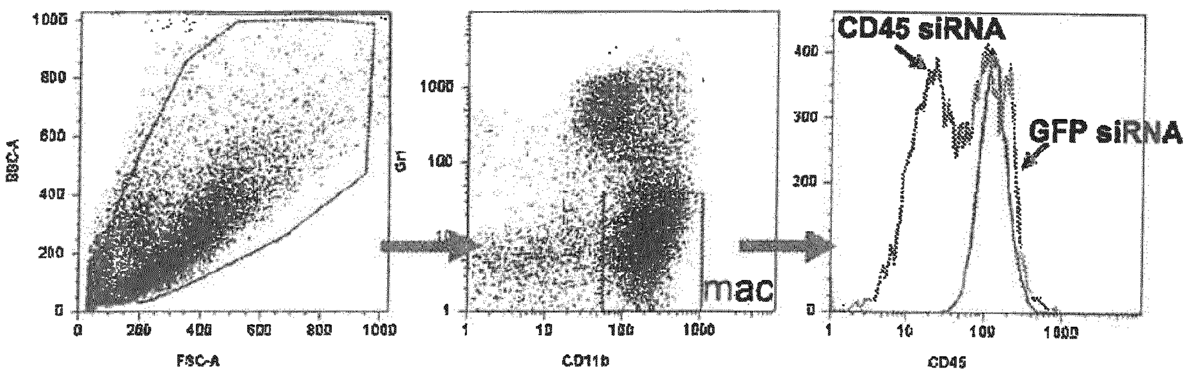


EP 3 698 631 A2

## Description

### RELATED APPLICATIONS

This application claims the benefit of U.S. Provisional Application No. 61/175,777, filed May 5, 2009; U.S. Provisional Application No. 61/234,045, filed August 14, 2009; U.S. Provisional Application No. 61/242,761, filed September 15, 2009; U.S. Provisional Application No. 61/251,991, filed October 15, 2009; and U.S. Provisional Application No. 61/258,848, filed November 6, 2009. Each of these prior applications is incorporated herein by reference in its entirety for all purposes.

### GOVERNMENT SUPPORT

The work described herein was carried out, at least in part, using funds from the U.S. Government under grant number HHSN266200600012C awarded by the National Institute of Allergy and Infectious Diseases. The government may therefore have certain rights in the invention.

### Technical Field

The invention relates to the field of delivery of nucleic acid-based agents to immune cells.

### Description of the Related Art

Therapeutic nucleic acids include, *e.g.*, small interfering RNA (siRNA), micro RNA (miRNA), antisense oligonucleotides, ribozymes, plasmids, and immune stimulating nucleic acids. These nucleic acids act via a variety of mechanisms. In the case of siRNA or miRNA, these nucleic acids can down-regulate intracellular levels of specific proteins through a process termed RNA interference (RNAi). Following introduction of siRNA or miRNA into the cell cytoplasm, these double-stranded RNA constructs can bind to a protein termed RISC. The sense strand of the siRNA or miRNA is displaced from the RISC complex providing a template within RISC that can recognize and bind mRNA with a complementary sequence to that of the bound siRNA or miRNA. Having bound the complementary mRNA the RISC complex cleaves the mRNA and releases the cleaved strands. RNAi can provide downregulation of specific proteins by targeting specific destruction of the corresponding mRNA that encodes for protein synthesis.

The therapeutic applications of RNAi are extremely broad, since siRNA and miRNA constructs can be synthesized with any nucleotide sequence directed against a target protein. To date, siRNA constructs have shown the ability to specifically down-regulate target proteins in both *in vitro* and *in vivo* models. In addition, siRNA constructs are currently being evaluated in clinical studies.

In spite of recent progress, there remains a need in the art for improved lipid-therapeutic nucleic acid compositions that are suitable for general therapeutic use. These compositions would, for example, encapsulate nucleic acids with high-efficiency, have high drug: lipid ratios, protect the encapsulated nucleic acid from degradation and clearance in serum, be suitable for systemic delivery, and provide intracellular delivery of the encapsulated nucleic acid. In addition, these lipid-nucleic acid particles should be well-tolerated and provide an adequate therapeutic index, such that patient treatment at an effective dose of the nucleic acid is not associated with significant toxicity and/or risk to the patient.

### Summary of Invention

The invention provides methods of delivering a nucleic acid-based agent to an immune cell (or silencing a gene in an immune cell) by, *e.g.*, providing a nucleic acid-based agent complexed with a formulation containing a lipid, and, for example, contacting the agent to the immune cell for a time sufficient to allow uptake of the agent into the immune cell. In one embodiment, immune cells of a selected compartment, *e.g.*, a selected tissue or organ of a subject, are targeted for agent delivery and gene silencing. In one embodiment the method includes selecting one or more of a subject, a nucleic acid-based agent, a lipid-containing formulation, or a route of delivery to provide for the cell type or compartment-based selectivity described herein.

The nucleic acid-based agent is, for example, an RNA-based construct, such as a double-stranded RNA (dsRNA), a single stranded RNA (ssRNA), an antisense RNA, a microRNA, or a ribozyme. In one embodiment, the nucleic acid-based agent is a dsRNA. The compositions described herein, for example, the nucleic acid-based agents complexed with lipid-containing formulations, have enhanced delivery to immune cells, particularly in immune cells of the peritoneal cavity of a subject. Thus, the featured compositions are particularly suited for use in the treatment of autoimmune and inflammatory disorders.

The featured method allows for selective delivery to a cell type or compartment (*e.g.*, a tissue or organ), or cell

type/compartment combination.

In one embodiment, the method includes confirming selective delivery or silencing, such as by measurement of entry into a cell, measurement of silencing, or detection of a therapeutic response in a subject.

Methods disclosed herein can be used *in vitro*, *in vivo* and *ex vivo.*

In one embodiment, the therapeutic agent, *e.g.,* the dsRNA, targets a gene expressed in an immune cell, *e.g.*, CD45, CD33, CD11, CD25, CD8, CD29, CD11 (*e.g.*, CD11a, b, or c), CD19, CD69, CD33, CD122, IL-2, or IL-6.

In another embodiment, a second dsRNA is administered to target a gene in an immune cell, such as to create a dominant effect, *e.g.*, to cause the cell carrying the silenced second target gene to affect the activity of other immune cells. In some embodiments, the second dsRNA targets a negative regulator of immune response (*e.g.*, PDL-1 (CD274 molecule), IL-10 (interleukin-10), or a TGF beta (transforming growth factor beta) gene, *e.g.*, a TGFbetal gene or a TGFbeta2 gene). In another embodiment, the second dsRNA targets an active pro-inflammatory stimuli (*e.g.*, TNF alpha (Tumor necrosis factor alpha), IL-18, *etc.*).

The immune cell can be in a localized tissue of a subject, such as in the peritoneal cavity, or bone marrow of the subject. The immune cell can be, for example, a leukocyte, such as a lymphocyte. The immune cell can be, for example, a macrophage, a dendritic cell, a monocyte, a neutrophil, a B cell, a T cell (*e.g.*, a regulatory T cell ("Treg"), or a natural killer (NK) cell In one embodiment, the immune cell is in the blood stream, and the immune cell is targeted to a localized tissue of the subject after the cell takes up the nucleic-acid based therapeutic agent.

In one embodiment, the nucleic acid-based agent is delivered to an immune cell of a subject (*e.g.*, a mammal, such as a human) by intravenous or intraperitoneal injection. In another embodiment, the nucleic acid-based agent is delivered to an immune cell in a particular tissue of the subject, such as to the peritoneal cavity or to the bone marrow of a subject. In another embodiment, the nucleic acid-based agent is delivered to an immune cell in the blood stream of the subject, and then the immune cell travels to and is taken up by a particular tissue, such as into the peritoneal cavity, or into the bone marrow or a site of inflammation.

In another embodiment, the nucleic acid-based agent is complexed to a formulation containing a lipid described in copending applications U.S.S.N 61/185,800, filed June 10,2009; PCT/US2009/063933, filed November 10, 2009; PCT/US2009/063931, filed November 10, 2009; PCT/US2009/063927, filed November 10, 2009; PCT/US2010/22614, filed January 29,2010; and U.S.S.N. 61/299291, filed January 28, 2010. The contents of each of these applications are incorporated by reference herein in their entirety for all purposes.

For example, the nucleic acid-based agent, *e.g.*, the dsRNA, can be complexed with a formulation having a sterol; a neutral lipid; a PEG or a PEG-modified lipid; and a cationic lipid selected from the group consisting of:

(i) a lipid having the structure of formula (I)

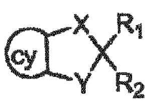 (I),

salts or isomers thereof, wherein: c

y is optionally substituted cyclic, optionally substituted heterocyclic or heterocycle, optionally substituted aryl or optionally substituted heteroaryl;
$R_1$ and $R_2$ are each independently for each occurrence optionally substituted $C_{10}$-$C_{30}$ alkyl, optionally substituted $C_{10}$-$C_{30}$ alkenyl, optionally substituted $C_{10}$-$C_{30}$ alkynyl, optionally substituted $C_{10}$-$C_{30}$ acyl or -linker-ligand;
X and Y are each independently O or S, alkyl or N(Q); and
Q is H, alkyl, acyl, co-aminoalkyl, ω-(substituted)aminoalky, ω-phosphoalkyl or ω-thiophosphoalkyl;

(ii) a lipid having the structure of formula (II)

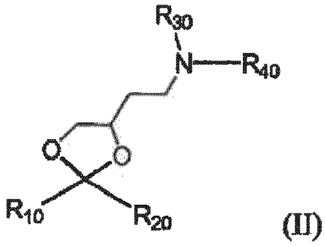 (II),

where $R_{10}$ and $R_{20}$ are independently alkyl, alkenyl or alkynyl, each can be optionally substituted, and $R_{30}$ and $R_{40}$ are independently lower alkyl or $R_{30}$ and $R_{40}$ can be taken together to form an optionally substituted heterocyclic ring;
(iii) a lipid having the structure

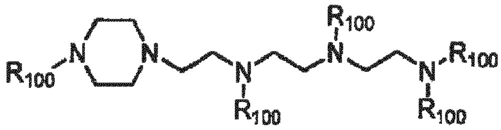

formula (III)

or

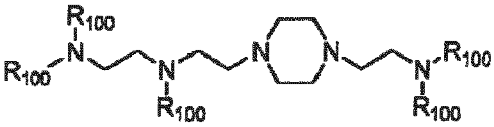

formula (IV),

wherein each R is independently H, alkyl,

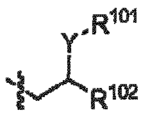, or 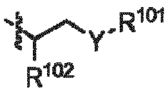 ;

provided that at least one R is

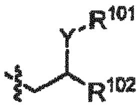, or 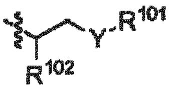 ;

wherein $R^{100}$, for each occurrence, is independently H, $R^{103}$,

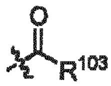, 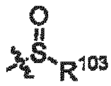, 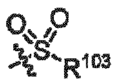, 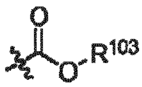,

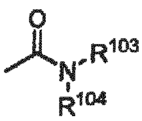, or 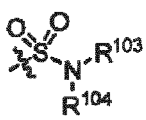 ;

wherein $R^{103}$ is optionally substituted with one or more substituent;
$R^{102}$, for each occurrence, is independently, alkyl, alkenyl, alkynyl, heteroalkyl, heteroalkenyl, or heteroalkynyl; each of which is optionally substituted with one or more substituent;
$R^{103}$, for each occurrence, is independently, alkyl, alkenyl, alkynyl, heteroalkyl, heteroalkenyl, or heteroalkynyl; each of which is optionally substituted with one or more substituent;
Y, for each occurrence, is independently O, $NR^{104}$, or S;
$R^{104}$, for each occurrence is independently H alkyl, alkenyl, alkynyl, heteroalkyl, heteroalkenyl, or heteroalkynyl; each of which is optionally substituted with one or more substituent, and

(iv) a lipid having the structure The compound of the following formula:

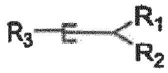 formula (V)

wherein:

$R_1$ and $R_2$ are each independently for each occurrence optionally substituted $C_{10}$-$C_{30}$ alkyl, optionally substituted $C_{10}$-$C_{30}$ alkoxy, optionally substituted $C_{10}$-$C_{30}$ alkenyl, optionally substituted $C_{10}$-$C_{30}$ alkenyloxy, optionally substituted $C_{10}$-$C_{30}$ alkynyl, optionally substituted $C_{10}$-$C_{30}$ alkynyloxy, or optionally substituted $C_{10}$-$C_{30}$ acyl;
E is -O-, -S-, -N(Q)-, -C(O)O-, -OC(O)-, -C(O)-, -N(Q)C(O)-, -C(O)N(Q)-, -N(Q)C(O)O-, -OC(O)N(Q)-, S(O), -N(Q)S(O)$_2$N(Q)-, -S(O)$_{2-}$, -N(Q)S(O)$_2$-, -SS-, - O-N=, =N-O-, -C(O)-N(Q)-N=, -N(Q)-N=, -N(Q)-O-, -C(O)S-, arylene, heteroarylene, cyclalkylene, or heterocyclylene; and
Q is H, alkyl, ω-aminoalkyl, ω-(substituted)aminoalkyl, ω-phosphoalkyl or ω-thiophosphoalkyl;
$R_3$ is H, optionally substituted $C_1$-$C_{10}$ alkyl, optionally substituted $C_2$-$C_{10}$ alkenyl, optionally substituted $C_2$-$C_{10}$ alkynyl, optionally substituted alkylheterocycle, optionally substituted heterocyclealkyl, optionally substituted alkylphosphate, optionally substituted phosphoalkyl, optionally substituted alkylphosphorothioate, optionally substituted phosphorothioalkyl, optionally substituted alkylphosphorodithioate, optionally substituted phosphorodithioalkyl, optionally substituted alkylphosphonate, optionally substituted phosphonoalkyl, optionally substituted amino, optionally substituted alkylamino, optionally substituted di(alkyl)amino, optionally substituted aminoalkyl, optionally substituted alkylaminoalkyl, optionally substituted di(alkyl)anoinoalkyl, optionally substituted hydroxyalkyl, optionally substituted polyethylene glycol (PEG, mw 100-40K), optionally substituted mPEG (mw 120-40K), optionally substituted heteroaryl, optionally substituted heterocycle, or linker-ligand.

In one embodiment, the lipid of formula (V) is 6Z,9Z,28Z,31Z)-heptatriaconta-6,9,28,31-tetraen-19-yl 4-(dimethylamino)butanoate (also called "DLin-M-C3-DMA," "MC3," and "Lipid M"), which has the following structure:

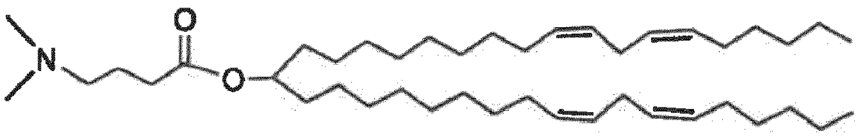

In this embodiment,
$R^1$ and $R^2$ are both linoleyl,
E is C(O)O; and
$R^3$ is a dimethylaminopropyl.

In one embodiment the method allows for one or more of the following:

a. preferential delivery of the nucleic acid-based agent or gene silencing in a peritoneal B cell, T cell, macrophage, or dendritic cell;
b. minimal delivery or gene silencing to a bone marrow B and or T cells;
c. preferential delivery or gene silencing in a bone marrow macrophage or dendritic cell;
d. preferential delivery or gene silencing in a splenic B cell or macrophage;
e. minimal delivery or gene silencing in a cell of Peyer's patches; or
f. minimal delivery to a liver cell.

In one embodiment, the method provides for delivery of a nucleic acid-based agent so that B cells, T cells, macrophages, or dendritic cells in the liver or Peyer's Patches are spared delivery of the agent complexed with the formulation or spared gene silencing.

In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter).

In some embodiments, the polydispersity index (PDI) of the particles is less than about 0.5 (e.g., less than about 0.4, less than about 0.3, less than about 0.2, or less than about 0.1).

In one aspect, a method of treating a subject having an autoimmune disorder, such as arthritis (*e.g.*, rheumatoid arthritis or artherosclerosis) is provided. The method includes administering to the subject a dsRNA complexed with a lipid-containing formulation, where the dsRNA targets a gene expressed in an immune cell, such as a CD45 gene in a

macrophage.

In another aspect, a method of preparing a liposome is provided. The method comprises providing a mixture comprising a sterol, a neutral lipid, and a cationic lipid, wherein the mixture is substantially free of a PEG or PEG-modified lipid; optionally, maintaining the mixture under conditions to allow the formation of liposomes, wherein the average diameter of the liposomes is at least 100 nm; and adding to said mixture a PEG or PEG-modified lipid; thereby preparing said liposome.

The details of one or more embodiments of the invention are set forth in the description below. Other features, objects, and advantages of the invention will be apparent from the description and the drawings, and from the claims.

## Brief Description of the Figures

FIGs. 1A and 1B show the results of LNP01 siRNA gene silencing *in vivo* in thioglycollate-activated macrophages. FIG. 1A is a panel depicting the results of fluorescence activated cell sorting of macrophages following uptake of LNP01-siRNAs. FIG. 1B is a graph depicting the downregulation of CD45 gene expression in macrophages by CD45 siRNAs.

FIG. 2 is a panel of FACS scans showing uptake of Alexa488-labeled siRNAs in B cells, myeloid cells and dendritic cells of the spleen.

FIGs. 3A and 3B show that LNP01 siRNAs were delivered to macrophages (FIG. 3A, third panel), but that there was no silencing of gene expression. AD-3176 siRNA targets ICAM2 RNAs and AD-1661 siRNA targets serum factor VII RNAs.

FIGs. 4A is a panel of FACS scans illustrating uptake of LNP08-formulated siRNAs when administered by i.v. (intravenous) or i.p. (intraperitoneal) injection. FIG. 4B is a bar graph showing downregulation ("knockdown" or KD) of CD45 gene expression in macrophages and dendritic cells isolated from the peritoneal cavity following administration of the LNP08-formulated siRNA by i.v. or i.p.

FIGs. 5A and 5B are FACS analyses showing the CD45 and luciferase LNP08 siRNAs were taken up by bone marrow leukocytes when administered by i.v. (FIG. 5A) or i.p. (FIG. 5B). FIG. 5C is a bar graph indicating that LNP08 CD45 siRNAs silenced gene expression in leukocytes following i.v. or i.p. administration.

FIG. 6 is a bar graph depicting CD45 levels in lymphocytes of the peritoneal cavity following injection of LNP08 siRNAs by i.p. or i.v.

FIGs. 7A and 7B are bar graphs depicting CD45 levels in lymphocytes (FIG. 7A) and leukocytes (FIG. 7B) of splenic cells following injection of LNP08 siRNAs by i.p. or i.v.

FIGs. 8A and 8B are bar graphs depicting CD45 levels in leukocytes from Peyer's Patches (FIG. 8A) or liver tissue (FIG. 8B) following injection of LNP08 siRNAs by i.p. or i.v.

FIG. 9 is a bar graph depicting the level of CD45 silencing in macrophages and dendritic cells in the peritoneal cavity, spleen, bone marrow (BM) and liver following i.v. administration of lipid A-formulated siRNAs.

FIGs. 10A and 10B are FACS analyses indicating uptake of lipid A-formulated CD45 siRNAs into macrophages (FIG. 10A) and dendritic cells (FIG. 10B) of the peritoneal cavity. FIG. 10C is a bar graph depicting CD45 silencing in macrophages and dendritic cells of the peritoneal cavity.

FIGs. 11A and 11B are FACS analyses indicating uptake of lipid A-formulated CD45 siRNAs into macrophages (FIG. 11A) and dendritic cells (FIG. 11B) of the peritoneal cavity at different dosage levels. FIG. 11C is a bar graph depicting CD45 silencing in macrophages and dendritic cells of the peritoneal cavity at various dosage levels.

FIG. 12 is a panel of FACS scans depicting a time course of uptake of Lipid A-formulated siRNAs by macrophages, monocytes, B cells and T cells in the peritoneal cavity, bone marrow, spleen and blood.

FIG. 13 is a graph illustrating the time course of uptake of lipid A-formulated siRNAs by blood monocytes, spleen macrophages, and large macrophages of the peritoneal cavity.

FIGs. 14A-14D are bar graphs depicting the silencing effect of CD45 siRNAs in monocytes and macrophages of bone marrow (FIG. 14A), spleen (FIG. 14B), blood (FIG. 14C) and the peritoneal cavity (FIG. 14D) following i.v. administration.

FIG. 15A is a panel of FACS scans demonstrating uptake of lipid A-formulated CD45 and luciferase siRNAs following i.v. or i.p. administration, and FIG. 15B is a bar graph illustrating downregulation of CD45 gene expression in leukocytes following i.v. or i.p. administration.

FIG. 16 is a bar graph depicting CD45 levels in lymphocytes of the peritoneal cavity following injection of Lipid T-formulated siRNAs by i.p. or i.v.

FIGs. 17A and B are FACS scans indicating that CD45 and luciferase Lipid T-formulated siRNAs were taken up by bone marrow leukocytes when injected by i.v. (FIG. 17A) or i.p. (FIG. 17B). FIG. 17C is a bar graph depicting silencing by Lipid T-formulated CD45 siRNAs in bone marrow leukocytes.

FIGs. 18A-18C are bar graphs depicting CD45 levels in leukocytes of the liver (FIG. 18A), spleen (FIG. 18B) or Peyer's patches following injection of Lipid T-formulated siRNAs into mice by i.p. or i.v.

FIGs. 19A and 19B illustrate dose-dependent uptake (FIG. 19A) and gene silencing (FIG. 19B) of lipid T-formulated siRNAs in macrophages of the peritoneal cavity following i.v. administration at various dosages.

FIGs. 20A and 20B illustrate uptake (FIG. 20A) and gene silencing (FIG. 20B) of lipid T-formulated siRNAs in macrophages and dendritic cells of the spleen following i.v. administration at various dosages.

FIG. 21A is a bar graph depicting CD45 silencing in macrophages and dendritic cells following injection of various lipid A-formulated CD45 siRNAs.

FIG. 21B is a bar graph depicting FVII silencing in liver following injection of various lipid A-formulated FVII siRNAs.

FIGs. 22A and 22B are correlation plots comparing FVII knockdown in liver and CD45 knockdown in macrophages (FIG. 22A) or dendritic cells (FIG. 22B) following injection of siRNAs formulated with various Lipid A compositions.

FIGs. 23A and 23B are graphs depicting the effect of incubation time on liposome size (FIG. 23A) and on size distribution as measured by polydispersity index (PDI) (FIG. 23B).

FIG 23C is a graph depicting the size distribution profiles of liposomes collected at the indicated times after initiation of the liposome fusion reaction.

FIG. 24A is a bar graph depicting FVII silencing in liver following injection of lipid A-formulated FVII siRNAs having various particle sizes.

FIG. 24B is a bar graph depicting CD45 silencing in peritoneal cells following injection of lipid A-formulated CD45 siRNAs having various particle sizes.

FIG. 24C is a bar graph depicting CD45 silencing in splenocytes following injection of lipid A-formulated CD45 siRNAs having various particle sizes.

FIG. 24D is a correlation plot comparing FVII silencing in the liver and CD45 silencing in macrophages following injection of lipid A-formulated siRNAs having various particle sizes.

FIGs. 25A and 25B are bar graphs depicting the dosage dependent silencing of CD45 in primary macrophages *in vitro* when formulated with LNP-01 (FIG. 25A) or with LNP08 (FIG. 25B).

FIG. 26 is a bar graph depicting the dosage dependent silencing of CD45 expression in macrophages and dendritic cells of the peritoneal cavity when siRNA is formulated with LNP08.

FIGs. 27A and 27B are FACS analyses depicting the uptake of lipid M formulated siRNAs by macrophages and dendritic cells. FIG. 27C is a bar graph indicating dosage dependent silencing by lipid M formulated siRNAs.

FIGs. 28A-28D are bar graphs depicting CD45 silencing following multi-dosing of lipid A- (XTC) or lipid M- (MC3-) formulated siRNAs by cells of the peritoneal cavity (FIG. 28A), spleen (FIG. 28B), bone marrow (FIG. 28C), or liver (FIG. 28D).

## Detailed Description

The invention provides methods of delivering a nucleic acid-based agent to an immune cell by, for example, providing a nucleic acid-based agent, *e.g.*, a therapeutic agent, complexed with a lipid-containing formulation, and contacting the agent to the immune cell for a time sufficient to allow uptake of the agent into the immune cell. The nucleic acid-based agent is, for example, an RNA-based construct, such as a double-stranded RNA (dsRNA), an antisense RNA, a microRNA, or a ribozyme.

### *Lipid formulations*

The compositions disclosed herein, *i.e.*, the compositions containing nucleic-acid based agents complexed with lipid formulations (also referred to as lipid-containing formulations), are suitable for delivering the nucleic acid-based agents to an immune cell, such as an immune cell in a subject. The delivery methods include administering the compositions containing an agent, *e.g.*, a dsRNA, to an animal, and, optionally, evaluating the expression of the target gene in the immune cell. Typically, the composition containing the nucleic acid-based agent and lipid formulation is taken up by an immune cell to a greater extent than if the nucleic acid was not complexed with the lipid formulation. For example, the uptake of the agent into the immune cell is at least 10% or greater (*e.g.*, at least 20%, 30%, 40%, 50%, 60%, 70%, 80%, 90% 100% or greater), than if the agent was not complexed with the lipid formulation.

Lipid formulations suitable for the compositions targeting immune cells, include formulations having a cationic lipid of formula A, a neutral lipid, a sterol and a PEG or PEG-modified lipid, wherein formula A is

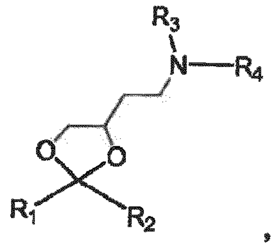,

where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally substituted heterocyclic ring. In one embodiment, $R_1$ and $R_2$ are independently selected from oleoyl, pamitoyl, steroyl, linoleyl and $R_3$ and $R_4$ are methyl. In another embodiment, $R_1$ and $R_2$ are independently selected from oleoyl, pamitoyl, steroyl, linoleyl and $R_3$ and $R_4$ are methyl.

In one embodiment, the lipid of formula A is 2,2-dilinoleyl-4-dimethylaminoethyl-[1,3]-dioxolane (also called LipidA or XTC,), which has the following structure:

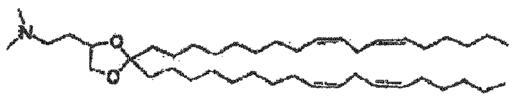

In one embodiment, the formulation includes 10-75% of cationic lipid of formula A, 0.5-50% of the neutral lipid, 5-60% of the sterol, and 0.1-20% of the PEG or PEG-modified lipid.

In another embodiment, the formulation includes 25-75% of cationic lipid of formula A, 0.5-15% of the neutral lipid, 5-50% of the sterol, and 0.5-20% of the PEG or PEG-modified lipid.

In another embodiment, the formulation includes 35-65% of cationic lipid of formula A, 3-12% of the neutral lipid, 15-45% of the sterol, and 0.5-10% of the PEG or PEG-modified lipid.

In yet another embodiment, the formulation includes 45-65% of cationic lipid of formula A, 5-10% of the neutral lipid, 25-40% of the sterol, and 0.5-5% of the PEG or PEG-modified lipid.

In one embodiment, the formulation includes 10-50% of cationic lipid of formula A, 10-50% of the neutral lipid, 20-50% of the sterol, and 0.5-15% of the PEG or PEG-modified lipid.

In one embodiment, the formulation includes 20-40% of cationic lipid of formula A, 20-40% of the neutral lipid, 25-45% of the sterol, and 0.5-5% of the PEG or PEG-modified lipid.

In one embodiment, the formulation includes 25-35% of cationic lipid of formula A, 25-35% of the neutral lipid, 35-45% of the sterol, and 1-2% of the PEG or PEG-modified lipid.

In one embodiment, the formulation includes about 30% of cationic lipid of formula A, 30% of the neutral lipid, 38.5% of the sterol, and 0.5% of the PEG or PEG-modified lipid. In one embodiment, the cationic lipid is Lipid A, the neutral lipid is DSPC (distearoylphosphatidylcholine), the sterol is cholesterol and the PEG (polyethylene glycol) lipid is PEG-DMG or PEG-DSG. In some embodiments, the PEG is PEG-Cerl4 or PEG-Cer18.

In one embodiment, the formulation includes 25-35% of cationic lipid of formula A, 25-35% of the neutral lipid, 25-35% of the sterol, and 5-15% of the PEG or PEG-modified lipid.

In one embodiment, the formulation includes about 30% of cationic lipid of formula A, 30% of the neutral lipid, 30% of the sterol, and 10% of the PEG or PEG-modified lipid. In one embodiment, the cationic lipid is Lipid A, the neutral lipid is DSPC (distearoylphosphatidylcholine), the sterol is cholesterol and the PEG (polyethylene glycol) lipid is PEG-CerC14 or PEG-Cerl8. In some embodiments, the PEG is PEG-Cer18.

In another embodiment, the formulation includes about 60% of cationic lipid of formula A, about 7.5% of the neutral lipid, about 31 % of the sterol, and about 1.5% of the PEG or PEG-modified lipid. In one embodiment, the cationic lipid of formula A is 2,2-Dilinoleyl-4-dimethylaminoethyl-[1,3]-dioxolane, the neutral lipid is DSPC (distearoylphosphatidylcholine), the sterol is cholesterol and the PEG (polyethylene glycol) lipid is PEG-DMG (1-(monomethoxy polyethyleneglycol)-2,3-dimyristoylglycerol), wherein the PEG has an average molecular weight of about 2,000.

In another embodiment, the formulation includes about 57.5% of cationic lipid of formula A, about 7.5% of the neutral lipid, about 31.5 % of the sterol, and about 3.5% of the PEG or PEG-modified lipid. In one embodiment, the cationic lipid of formula A is Lipid A (2,2-Dilinoleyl-4-dimethylaminoethyl-[1.3]-dioxolane), the neutral lipid is DSPC, the sterol is cholesterol and the PEG lipid is PEG-DMG.

In one embodiment, the ratio of lipid:dsRNA is at least about 0.5, at least about 1, at least about 2, at least about 3, at least about 4, at least about 5, at least about 6. In one embodiment, the ratio of lipid:siRNA ratio is between about 1 to about 20, about 3 to about 15, about 4 to about 15, about 5 to about 13. In one embodiment, the ratio of

lipid:siRNA ratio is between about 0.5 to about 12.

In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter).

In some embodiments, the polydispersity index (PDI) of the particles is less than about 0.5 (e.g., less than about 0.4, less than about 0.3, less than about 0.2, or less than about 0.1).

In one embodiment, the lipid formulations suitable for complexing with nucleic acid-based agents are produced via an extrusion method, an in-line mixing method, or any method described herein.

The extrusion method (also refer to as preformed method or batch process) is a method where the empty liposomes (*i.e.* no nucleic acid) are prepared first, followed by the the addition of nucleic acid to the empty liposome. Extrusion of liposome compositions through a small-pore polycarbonate membrane or an asymmetric ceramic membrane results in a relatively well-defined size distribution. Typically, the suspension is cycled through the membrane one or more times until the desired liposome complex size distribution is achieved. The liposomes may be extruded through successively smaller-pore membranes, to achieve a gradual reduction in liposome size. In some instances, the lipid-nucleic acid compositions which are formed can be used without any sizing. These methods are disclosed in the U.S. 5,008,050; US 4,927,637; US 4,737,323; Biochim Biophys Acta. 1979 Oct 19;557(1):9-23; Biochim Biophys Acta. 1980 Oct 2;601(3):559-7; Biochim Biophys Acta. 1986 Jun 13;858(1):161-8; and Biochim. Biophys. Acta 1985 812, 55-65, which are hereby incorporated by reference in their entirety.

The in-line mixing method is a method where both the lipids and the nucleic acid are added in parallel into a mixing chamber. The mixing chamber can be a simple T-connector or any other mixing chamber that is known to one skill in the art. These methods are disclosed in U.S. patent nos. 6,534,018 and U.S. 6,855,277; U.S. publication 2007/0042031 and Pharmaceuticals Research, Vol 22, No. 3, Mar. 2005, p. 362-372, which are hereby incorporated by reference in their entirety.

In some embodiments, a liposome can be prepared using a method that allows the formation of particles having a mean diameter of at least about 100 nm. The method comprises providing a mixture comprising a sterol, a neutral lipid, and a cationic lipid, wherein the mixture is substantially free of a PEG or PEG-modified lipid; optionally, maintaining the mixture under conditions to allow the formation of liposomes, wherein the average diameter of the liposomes is at least 100 nm; and adding to said mixture a PEG or PEG-modified lipid; thereby preparing said liposome.

In some embodiments, the method also includes incorporating a nucleic acid (e.g., a nucleic acid described herein) into the liposome to form a nucleic acid-containing agent. The nucleic acid can be a single stranded or double stranded nucleic acid. The nucleic acid can comprise RNA Interference Nucleic Acids as described herein.

In some embodiments, conditions which allow the formation of liposomes include adjustment of the pH, ionic strength and/or sodium concentration, temperature, among other parameters. In some embodiments, the pH of the mixture is acidic (e.g., the cationic lipid in the mixture is essentially protonated). In some embodiments, the pH of the mixture is less than the pKa of the cationic lipid (e.g., at least 1.0 less than the cationic lipid). In some embodiments, the pH is less than about 5.5 (e.g., about 5.2, about 4.8, about 3.2 or about 3.0).

In some embodiments, the mixture has a concentration of cation such as sodium of less than about 50 mM, (e.g., about 25 mM or less, about 15 mM or less, or about 10 mM or less).

In some embodiments, the mixture comprises a protic solvent such as ethanol. Exemplary cationic lipids include those described herein such as a cationic lipid of any of formulae I-IV. In some embodiments, the cationic lipid comprises lipid A. Exemplary neutral lipids include any neutral lipid described herein such as DSPC. Exemplary sterols include any sterol described herein such as cholesterol.

In some embodiments, the method includes including a PEG modified lipid, such as a PEG-modified lipid described herein (e.g., PEG-DMG, PEG-DSG, PEG-CerC14 or PEG-CerC18), for example, after maintaining the mixture under conditions to allow the formation of liposomes wherein the the average diameter of the liposomes is at least 100 nm (for example, at least 150nm, at least 200nm, at least 250nm, or at least 300nm).

The relative ratios of the sterol, neutral lipid, cationic lipid, and PEG or PEG-modified lipid are generally as descrbed herein. Where the liposome includes a nucleic acid (e.g., a nucleic acid-based agent), the ratios of components are also generally as described herein.

In one embodiment, the average particle size of the liposome (either containing or not containing a nucleic acid) is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter).

In some embodiments, the polydispersity index (PDI) of the liposome is less than about 0.5 (e.g., less than about 0.4, less than about 0.3, less than about 0.2, or less than about 0.1). In some embodiments, the average particle size of the liposome is at least about 100 nm in diameter e.g., at least about 110 nm in diameter, at least about 120 nm in diameter, at least about 150 nm in diameter, at least about 200 nm in diameter, at least about 250 nm in diameter, or

at least about 300 nm in diameter) and the PDI is less than about 0.5 (e.g., less than about 0.4, less than about 0.3, less than about 0.2, or less than about 0.1). It is further understood that the formulations of the invention can be prepared by any methods known to one of ordinary skill in the art.

In a further embodiment, representative formulations prepared *via* the extrusion method are delineated in Table 1, wherein Lipid A is a compound of formula A, where $R_1$ and $R_2$ are linoleyl and $R_3$ and $R_4$ are methyl:

Table 1

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 20 | 30 | 40 | 10 | 1955 | 2.13 | 1.12 | 12.82 | 39 | -0.265 | 85.3 | 0.109 |
| 20 | 30 | 40 | 10 | 1955 | 2.35 | 1.23 | 14.15 | 53 | -0.951 | 86.8 | 0.081 |
| 20 | 30 | 40 | 10 | 1955 | 2.37 | 1.25 | 14.29 | 70 | 0.374 | 79.1 | 0.201 |
| 20 | 30 | 40 | 10 | 1955 | 3.23 | 1.70 | 19.48 | 77 | 5.89 | 81.4 | 0.099 |
| 20 | 30 | 40 | 10 | 1955 | 3.91 | 2.05 | 23.53 | 85 | 10.7 | 80.3 | 0.105 |
| 30 | 20 | 40 | 10 | 1955 | 2.89 | 1.52 | 11.36 | 44 | -9.24 | 82.7 | 0.142 |
| 30 | 20 | 40 | 10 | 1955 | 3.34 | 1.76 | 13.16 | 57 | -4.32 | 76.3 | 0.083 |
| 30 | 20 | 40 | 10 | 1955 | 3.34 | 1.76 | 13.16 | 76 | -1.75 | 74.8 | 0.067 |
| 30 | 20 | 40 | 10 | 1955 | 4.10 | 2.15 | 16.13 | 93 | 3.6 | 72.8 | 0.082 |
| 30 | 20 | 40 | 10 | 1955 | 5.64 | 2.97 | 22.22 | 90 | 4.89 | 70.8 | 0.202 |
| 40 | 10 | 40 | 10 | 1955 | 3.02 | 1.59 | 8.77 | 57 | -12.3 | 63.3 | 0.146 |
| 40 | 10 | 40 | 10 | 1955 | 3.35 | 1.76 | 9.74 | 77 | 7.73 | 57 | 0.192 |
| 40 | 10 | 40 | 10 | 1955 | 3.74 | 1.97 | 10.87 | 92 | 13.2 | 56.9 | 0.203 |
| 40 | 10 | 40 | 10 | 1955 | 5.80 | 3.05 | 16.85 | 89 | 13.8 | 64 | 0.109 |
| 40 | 10 | 40 | 10 | 1955 | 8.00 | 4.20 | 23.26 | 86 | 14.7 | 65.2 | 0.132 |
| 45 | 5 | 40 | 10 | 1955 | 3.27 | 1.72 | 8.33 | 60 | -10.7 | 56.4 | 0.219 |
| 45 | 5 | 40 | 10 | 1955 | 3.30 | 1.74 | 8.43 | 89 | 12.6 | 40.8 | 0.238 |
| 45 | 5 | 40 | 10 | 1955 | 4.45 | 2.34 | 11.36 | 88 | 12.4 | 51.4 | 0.099 |
| 45 | 5 | 40 | 10 | 1955 | 7.00 | 3.68 | 17.86 | 84 | 13.2 | 78.1 | 0.055 |
| 45 | 5 | 40 | 10 | 1955 | 9.80 | 5.15 | 25.00 | 80 | 13.9 | 64.2 | 0.106 |
| 50 | 0 | 40 | 10 | 1955 | 27.03 | 14.21 | 68.97 | 29 | | 42.0 | 0.155 |
| 20 | 35 | 40 | 5 | 1955 | 3.00 | 1.58 | 16.13 | 31 | 8.14 | 76.8 | 0.068 |
| 20 | 35 | 40 | 5 | 1955 | 3.32 | 1.75 | 17.86 | 42 | -4.88 | 79.3 | 0.093 |
| 20 | 35 | 40 | 5 | 1955 | 3.05 | 1.60 | 16.39 | 61 | -4.48 | 64.4 | 0.12 |
| 20 | 35 | 40 | 5 | 1955 | 3.67 | 1.93 | 19.74 | 76 | 3.89 | 72.9 | 0.161 |
| 20 | 35 | 40 | 5 | 1955 | 4.71 | 2.48 | 25.32 | 79 | 10.7 | 76.6 | 0.067 |
| 30 | 25 | 40 | 5 | 1955 | 2.47 | 1.30 | 8.62 | 58 | -2.8 | 79.1 | 0.153 |
| 30 | 25 | 40 | 5 | 1955 | 2.98 | 1.57 | 10.42 | 72 | -2.73 | 74.1 | 0.046 |
| 30 | 25 | 40 | 5 | 1955 | 3.29 | 1.73 | 11.49 | 87 | 13.6 | 72.5 | 0.079 |
| 30 | 25 | 40 | 5 | 1955 | 4.99 | 2.62 | 17.44 | 86 | 14.6 | 72.3 | 0.057 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 30 | 25 | 40 | 5 | 1955 | 7.15 | 3.76 | 25.00 | 80 | 13.8 | 75.8 | 0.069 |
| 40 | 15 | 40 | 5 | 1955 | 2.79 | 1.46 | 7.14 | 70 | -3.52 | 65.4 | 0.068 |
| 40 | 15 | 40 | 5 | 1955 | 3.29 | 1.73 | 8.43 | 89 | 13.3 | 58.8 | 0.078 |
| 40 | 15 | 40 | 5 | 1955 | 4.33 | 2.28 | 11.11 | 90 | 14.9 | 62.3 | 0.093 |
| 40 | 15 | 40 | 5 | 1955 | 7.05 | 3.70 | 18.07 | 83 | 14.7 | 64.8 | 0.046 |
| 40 | 15 | 40 | 5 | 1955 | 9.63 | 5.06 | 24.69 | 81 | 15.4 | 63.2 | 0.06 |
| 45 | 10 | 40 | 5 | 1955 | 2.44 | 1.28 | 6.25 | 80 | -1.86 | 70.7 | 0.226 |
| 45 | 10 | 40 | 5 | 1955 | 3.21 | 1.69 | 8.24 | 91 | 8.52 | 59.1 | 0.102 |
| 45 | 10 | 40 | 5 | 1955 | 4.29 | 225 | 10.99 | 91 | 9.27 | 66.5 | 0.207 |
| 45 | 10 | 40 | 5 | 1955 | 6.50 | 3.42 | 16.67 | 90 | 9.3 | 59.6 | 0.127 |
| 45 | 10 | 40 | 5 | 1955 | 8.67 | 4.56 | 22.22 | 90 | 11.2 | 63.5 | 0.083 |
| 20 | 35 | 40 | 5 | 1661 | 4.10 | 2.16 | 22.06 | 68 | -3.94 (-2.95) | 85.6 | 0.041 |
| 20 | 35 | 40 | 5 | 1661 | 4.83 | 2.54 | 25.97 | 77 | 1.7 (1.73) | 81.5 | 0.096 |
| 30 | 25 | 40 | 5 | 1661 | 3.86 | 2.03 | 13.51 | 74 | 3.63 | 59.9 | 0.139 |
| 30 | 25 | 40 | 5 | 1661 | 5.38 | 2.83 | 18.75 | 80 | 12 | 67.3 | 0.106 |
| 30 | 25 | 40 | 5 | 1661 | 7.07 | 3.72 | 24.69 | 81 | 10.7 | 69.5 | 0,145 |
| 40 | 15 | 40 | 5 | 1661 | 3.85 | 2.02 | 9.87 | 76 | -3.79 | 63 | 0.166 |
| 40 | 15 | 40 | 5 | 1661 | 4.88 | 2.56 | 12.50 | 80 | 1.76 | 64.6 | 0.073 |
| 40 | 15 | 40 | 5 | 1661 | 7.22 | 3.80 | 18.52 | 81 | 5.87 | 69 | 0.094 |
| 40 | 15 | 40 | 5 | 1661 | 9.75 | 5.12 | 25.00 | 80 | 9.25 | 65.5 | 0.177 |
| 45 | 10 | 40 | 5 | 1661 | 2.83 | 1.49 | 7.25 | 69 | -10.2 | 67.8 | 0.036 |
| 45 | 10 | 40 | 5 | 1661 | 3.85 | 2.02 | 9.87 | 76 | 3.53 | 57.1 | 0.058 |
| 45 | 10 | 40 | 5 | 1661 | 4.88 | 2.56 | 12.50 | 80 | 6.22 | 57.9 | 0.096 |
| 45 | 10 | 40 | 5 | 1661 | 7.05 | 3.70 | 18.07 | 83 | 12.8 | 58.2 | 0.108 |
| 45 | 10 | 40 | 5 | 1661 | 9.29 | 4.88 | 23.81 | 84 | 9.89 | 55.6 | 0.067 |
| 45 | 20 | 30 | 5 | 1955 | 4.01 | 2.11 | 9.61 | 71 | 3.99 | 57.6 | 0.249 |
| 45 | 20 | 30 | 5 | 1661 | 3.70 | 1.95 | 8.86 | 77 | 4.33 | 74.4 | 0.224 |
| 50 | 15 | 30 | 5 | 1955 | 4.75 | 2.50 | 10.12 | 60 | 13 | 59.1 | 0.29 |
| 50 | 15 | 30 | 5 | 1661 | 3.80 | 2.00 | 8.09 | 75 | 5.48 | 82.5 | 0.188 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 55 | 10 | 30 | 5 | 1955 | 3.85 | 2.02 | 7.38 | 74 | 1.83 | 49.9 | 0.152 |
| 55 | 10 | 30 | 5 | 1661 | 4.13 | 2.17 | 7.91 | 69 | -6.76 | 53.9 | 0.13 |
| 60 | 5 | 30 | 5 | 1955 | 5.09 | 2.68 | 8.84 | 56 | -10.8 | 60 | 0.191 |
| 60 | 5 | 30 | 5 | 1661 | 4.67 | 2.46 | 8.11 | 61 | -11.5 | 63.7 | 0.254 |
| 65 | 0 | 30 | 5 | 1955 | 4.75 | 2.50 | 7.53 | 60 | 4.24 | 48.6 | 0.185 |
| 65 | 0 | 30 | 5 | 1661 | 6.06 | 3.19 | 9.62 | 47 | -8.3 | 45.7 | 0.147 |
| 56.5 | 10 | 30 | 3.5 | 1661 | 3.70 | 1.95 | 6.61 | 77 | -0.0189 | 54.3 | 0.096 |
| 56.5 | 10 | 30 | 3.5 | 1955 | 3.56 | 1.87 | 6.36 | 80 | 0.997 | 54.8 | 0.058 |
| 57.5 | 10 | 30 | 2.5 | 1661 | 3.48 | 1.83 | 5.91 | 82 | 2.63 | 70.1 | 0.049 |
| 57.5 | 10 | 30 | 2.5 | 1955 | 3.20 | 1.68 | 5.45 | 89 | 4.3 | 71.4 | 0.046 |
| 58.5 | 10 | 30 | 1.5 | 1661 | 3.24 | 1.70 | 5.26 | 88 | -1.91 | 81.3 | 0.056 |
| 58.5 | 10 | 30 | 1.5 | 1955 | 3.13 | 1.65 | 5.09 | 91 | 1.86 | 85.7 | 0.047 |
| 59.5 | 10 | 30 | 0.5 | 1661 | 3.24 | 1.70 | 5.01 | 88 | -10,7 | 138 | 0.072 |
| 59.5 | 10 | 30 | 0.5 | 1955 | 3.03 | 1.59 | 4.69 | 94 | -0.603 | 155 | 0.012 |
| 45 | 10 | 40 | 5 | 1661 | 7.57 | 3.98 | 17.05 | 88 | 6.7 | 59.8 | 0.196 |
| 45 | 10 | 40 | 5 | 1661 | 7.24 | 3.81 | 16.30 | 92 | 10.6 | 56.2 | 0.096 |
| 45 | 10 | 40 | 5 | 1661 | 7.48 | 3.93 | 16.85 | 89 | 1.2 | 55.3 | 0.151 |
| 45 | 10 | 40 | 5 | 1661 | 7.84 | 4.12 | 17.65 | 85 | 2.2 | 54.7 | 0.105 |
| 65 | 0 | 30 | 5 | 1661 | 4.01 | 2.11 | 6.37 | 71 | 13.2 | 57.3 | 0.071 |
| 60 | 5 | 30 | 5 | 1661 | 3.70 | 1.95 | 6.43 | 77 | 14 | 58.1 | 0.128 |
| 55 | 10 | 30 | 5 | 1661 | 3.65 | 1.92 | 7.00 | 78 | 5.54 | 63.1 | 0.278 |
| 50 | 10 | 35 | 5 | 1661 | 3.43 | 1.80 | 7.10 | 83 | 12.6 | 58.4 | 0.102 |
| 50 | 15 | 30 | 5 | 1661 | 3.80 | 2.00 | 8.09 | 75 | 15.9 (6.17) | 60.3 | 0.11 |
| 45 | 15 | 35 | 5 | 1661 | 3.70 | 1.95 | 8.60 | 77 | 10.7 | 48.5 | 0.327 |
| 45 | 20 | 30 | 5 | 1661 | 3.75 | 1.97 | 8.97 | 76 | 15.5 | 63.2 | 0.043 |
| 45 | 25 | 25 | 5 | 1661 | 3.85 | 2.02 | 9.49 | 74 | 14.2 (4.08) | 61.2 | 0.14 |
| 55 | 10 | 32.5 | 2.5 | 1661 | 3.61 | 1.90 | 6.35 | 79 | 0.0665 | 70.6 | 0.091 |
| 60 | 10 | 27.5 | 2.5 | 1661 | 3.65 | 1.92 | 6.03 | 78 | 5.8 | 72.2 | 0.02 |
| 60 | 10 | 25 | 5 | 1661 | 4.07 | 2.14 | 7.29 | 70 | 3.53 | 48.7 | 0.055 |
| 55 | 5 | 38.5 | 1.5 | 1661 | 3.75 | 1.97 | 6.17 | 76 | 4.05 | 87.7 | 0.066 |
| 60 | 10 | 28.5 | 1.5 | 1661 | 3.43 | 1.80 | 5.47 | 83 | 3.47 | 95.9 | 0.024 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 55 | 10 | 33.5 | 1.5 | 1661 | 3.48 | 1.83 | 5.91 | 82 | 7.58 | 76.6 | 0.09 |
| 60 | 5 | 33.5 | 1.5 | 1661 | 3.43 | 1.80 | 5.29 | 83 | 7.18 | 148 | 0.033 |
| 55 | 5 | 37.5 | 2.5 | 1661 | 3.75 | 1.97 | 6.39 | 76 | 4.32 | 61.9 | 0.065 |
| 60 | 5 | 32.5 | 2.5 | 1661 | 4.52 | 2.38 | 7.22 | 63 | 2.67 | 65.7 | 0.069 |
| 60 | 5 | 32.5 | 2.5 | 1661 | 3.52 | 1.85 | 5.62 | 81 | 4.98 | 73.2 | 0.101 |
| 45 | 15 (DMPC) | 35 | 5 | 1661 | 3.20 | 1.68 | 7.26 | 89 | 5.9 | 53 | 0.079 |
| 45 | 15 (DPPC) | 35 | 5 | 1661 | 3.43 | 1.80 | 7.88 | 83 | 7.5 | 50.6 | 0.119 |
| 45 | 15 (DOPC) | 35 | 5 | 1661 | 4.52 | 2.38 | 10.51 | 63 | 6 | 44.1 | 0.181 |
| 45 | 15 (POPC) | 35 | 5 | 1661 | 3.85 | 2.02 | 8.89 | 74 | 3.8 | 48 | 0.09 |
| 55 | 5 | 37.5 | 2.5 | 1661 | 3.96 | 2.08 | 6.75 | 72 | -11 | 53.9 | 0.157 |
| 55 | 10 | 325 | 2.5 | 1661 | 3.56 | 1.87 | 6.28 | 80 | -4.6 | 56.1 | 0.135 |
| 60 | 5 | 32.5 | 2.5 | 1661 | 3.80 | 2.00 | 6.07 | 75 | -5.8 | 82.4 | 0.097 |
| 60 | 10 | 27.5 | 2.5 | 1661 | 3.75 | 1.97 | 6.18 | 76 | -8.4 | 59.7 | 0.099 |
| 60 | 5 | 30 | 5 | 1661 | 4.19 | 2.20 | 7.28 | 68 | -4.8 | 45.8 | 0.235 |
| 60 | 5 | 33.5 | 1.5 | 1661 | 3.48 | 1.83 | 5.35 | 82 | -10.8 | 73.2 | 0.065 |
| 60 | 5 | 33.5 | 1.5 | 1661 | 6.64 | 3.49 | 10.21 | 86 | -1.8 | 77.8 | 0.090 |
| 60 | 5 | 30 | 5 | 1661 | 3.90 | 2.05 | 6.78 | 73 | 10.2 | 60.9 | 0.062 |
| 60 | 5 | 30 | 5 | 1661 | 4.65 | 2.44 | 8.05 | 82 | 12.6 | 65.9 | 0.045 |
| 60 | 5 | 30 | 5 | 1661 | 5.88 | 3.09 | 10.19 | 81 | 11.9 | 60.7 | 0.056 |
| 60 | 5 | 30 | 5 | 1661 | 7.51 | 3.95 | 13.03 | 76 | 9.4 | 59.6 | 0.065 |
| 60 | 5 | 30 | 5 | 1661 | 9.51 | 5.00 | 16.51 | 80 | 10.3 | 61.4 | 0.021 |
| 60 | 5 | 30 | 5 | 1661 | 11.06 | 5.81 | 19.20 | 86 | 12.8 | 62.0 | 0.037 |
| 62.5 | 2.5 | 50 | 5 | 1661 | 6.63 | 3.49 | 11.00 | 43 | 4.8 | 62.2 | 0.107 |
| 45 | 15 | 35 | 5 | 1661 | 3.31 | 1.74 | 7.70 | 86 | 8.6 | 63.0 | 0.077 |
| 45 | 15 | 35 | 5 | 1661 | 6.80 | 3.57 | 15.77 | 84 | 14.9 | 60.8 | 0.120 |
| 60 | 5 | 25 | 10 | 1661 | 6.48 | 3.41 | 13.09 | 44 | 5.6 | 40.6 | 0.098 |
| 60 | 5 | 32.5 | 2.5 | 1661 | 3.43 | 1.81 | 5.48 | 83 | 7.3 | 61.5 | 0.099 |
| 60 | 5 | 30 | 5 | 1661 | 3.90 | 2.05 | 6.78 | 73 | 5.6 | 59.7 | 0.090 |
| 60 | 5 | 30 | 5 | 1661 | 7.61 | 4.00 | 13.20 | 75 | 14.9 | 55.9 | 0.104 |
| 45 | 15 | 35 | 5 | 1955 | 3.13 | 1.65 | 7.27 | 91 | 8.5 | 64.1 | 0.091 |
| 45 | 15 | 35 | 5 | 1955 | 6.42 | 3.37 | 14.89 | 89 | 8 | 57.9 | 0.074 |
| 60 | 5 | 25 | 10 | 1955 | 6.48 | 3.41 | 13.09 | 44 | -12.5 | 34.2 | 0.153 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 60 | 5 | 32.5 | 2.5 | 1955 | 3.03 | 1.60 | 4.84 | 94 | 1.8 | 72.7 | 0.078 |
| 60 | 5 | 30 | 5 | 1955 | 3.43 | 1.81 | 5.96 | 83 | -0.7 | 61.8 | 0.074 |
| 60 | 5 | 30 | 5 | 1955 | 6.72 | 3.53 | 11.65 | 85 | 6.4 | 65.5 | 0.046 |
| 60 | 5 | 30 | 5 | 1661 | 4.13 | 2.17 | 7.17 | 69 | 1.3 | 47.8 | 0.142 |
| 70 | 5 | 20 | 5 | 1661 | 5.48 | 2.88 | 8.48 | 52 | 7.6 | 48.2 | 0.06 |
| 80 | 5 | 10 | 5 | 1661 | 5.94 | 3.13 | 8.33 | 48 | 8.7 | 51.6 | 0.056 |
| 90 | 5 | 0 | 5 | 1661 | 9.50 | 5.00 | 12.27 | 30 | 1.4 | 48.7 | 0.116 |
| 60 | 5 | 30 | 5 C12PEG | 1661 | 3.85 | 2.03 | 6.68 | 74 | 4.3 | 60.1 | 0.18 |
| 60 | 5 | 30 | 5 | 1661 | 3.70 | 1.95 | 6.43 | 77 | 5.1 | 53.7 | 0.212 |
| 60 | 5 | 30 | C16PEG | 1661 | 3.80 | 2.00 | 6.61 | 75 | 4.8 | 49.2 | 0.14 |
| 60 | 5 | 30 | 5 | 1661 | 4.19 | 2.21 | 7.28 | 68 | 14 | 58.3 | 0.095 |
| 60 | 5 | 29 | 5 | 1661 | 4.07 | 2.14 | 7.07 | 70 | 6.3 | 50.6 | 0.119 |
| 60 | 5 | 30 | 5 | 1955 | 3.56 | 1.88 | 6.19 | 80 | | 56.5 | 0.026 |
| 60 | 5 | 30 | 5 | 1955 | 3.39 | 1.79 | 5.89 | 84 | 9.9 | 70.5 | 0.025 |
| 60 | 5 | 30 | 5 | 1661 | 3.96 | 2.08 | 6.88 | 72 | 0.6 | 53.1 | 0.269 |
| 60 | 5 | 30 | 5 | 1661 | 4.01 | 2.11 | 6.97 | 71 | 0.1 | 50.4 | 0.203 |
| 60 | 5 | 30 | 5 | 1661 | 4.07 | 2.14 | 7.07 | 70 | 0.3 | 53.7 | 0.167 |
| 60 | 5 | 30 | 5 | 1661 | 4.25 | 2.24 | 7.39 | 67 | -0.4 | 56.8 | 0.216 |
| 60 | 5 | 30 | 5 | 1661 | 3.80 | 2.00 | 6.60 | 75 | 3.7 | 61.2 | 0.096 |
| 60 | 5 | 30 | 5 | 1661 | 3.31 | 1.74 | 5.76 | 86 | 4.1 | 111 | 0.036 |
| 60 | 5 | 30 | 5 | 1661 | 4.83 | 2.54 | 8.39 | 59 | -7.7 | 51.7 | 0.109 |
| 60 | 5 | 30 | 5 | 1661 | 4.67 | 2.46 | 8.11 | 61 | -4.2 | 46.3 | 0.122 |
| 60 | 5 | 30 | 5 | 1661 | 3.96 | 2.08 | 6.88 | 72 | -8.4 | 68.2 | 0.161 |
| 57.5 | 7.5 | 33.5 | 1.5 | 1661 | 3.39 | 1.79 | 5.49 | 84 | 1.1 | 79.5 | 0.093 |
| 57.5 | 7.5 | 32.5 | 2.5 | 1661 | 3.39 | 1.79 | 5.69 | 84 | 4.4 | 70.1 | 0.081 |
| 57.5 | 7.5 | 31.5 | 3.5 | 1661 | 3.52 | 1.85 | 6.10 | 81 | 6.8 | 59.3 | 0.098 |
| 57.5 | 7.5 | 30 | 5 | 1661 | 4.19 | 2.21 | 7.65 | 68 | 6.1 | 65.2 | 0.202 |
| 60 | 5 | 30 | 5 | 1661 | 3.96 | 2.08 | 6.88 | 72 | -4 | 60.7 | 0.338 |
| 60 | 5 | 30 | 5 | 1661 | 3.96 | 2.08 | 6.88 | 72 | -4.2 | 79.4 | 0.006 |
| 60 | 5 | 30 | 5 | 1661 | 3.56 | 1.88 | 6.19 | 80 | -1.9 | 69.4 | 0.214 |
| 60 | 5 | 33.5 | 1.5 | 1661 | 3.52 | 1.85 | 5.42 | 81 | 6.2 | 70.4 | 0.163 |
| 60 | 5 | 25 | 10 | 1661 | 5.18 | 2.73 | 10.47 | 55 | 0.7 | 43.3 | 0.351 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 60 | 5 (DPPC) | 30 | 5 | 1661 | 4.25 | 2.24 | 7.36 | 67 | 4.6 | 49.7 | 0.118 |
| 60 | 5 | 32.5 | 2.5 | 1661 | 3.70 | 1.95 | 5.91 | 77 | 9.7 | 88.1 | 0.064 |
| 57.5 | 7.5 | 31.5 | 3.5 | 1661 | 3.06 | 1.61 | 5.32 | 62 | -2.7 | 53.9 | 0.163 |
| 57.5 | 7.5 | 31.5 | 3.5 | 1661 | 3.65 | 1.92 | 6.33 | 78 | 9.1 | 65.9 | 0.104 |
| 57.5 | 7.5 | 31.5 | 3.5 | 1661 | 4.70 | 2.47 | 8.14 | 81 | 9 | 64.4 | 0.06 |
| 57.5 | 7.5 | 31.5 | 3.5 | 1661 | 6.56 | 3.45 | 11.37 | 87 | 10.5 | 68.8 | 0.066 |

In a further embodiment, representative formulations prepared via the in-line mixing method are delineated in Table 2, wherein Lipid A is a compound of formula A, where $R_1$ and $R_2$ are linoleyl and $R_3$ and $R_4$ are methyl:

Table 2

| Composition (mole%) | | | | siRNA | Lipid A/ siRNA | Charge ratio | Total Lipid/ siRNA | Entrapment (%) | Zeta potential | Particle Size (nm) | PDI |
|---|---|---|---|---|---|---|---|---|---|---|---|
| Lipid A | DSPC | Chol | PEG | | | | | | | | |
| 55 | 5 | 37.5 | 2.5 | 1661 | 3.96 | 2.08 | 6.75 | 72 | -11 | 53.9 | 0.157 |
| 55 | 10 | 32.5 | 2.5 | 1661 | 3.56 | 1.87 | 6.28 | 80 | -4.6 | 56.1 | 0.135 |
| 60 | 5 | 32.5 | 2.5 | 1661 | 3.80 | 2.00 | 6.07 | 75 | -5.8 | 82.4 | 0.097 |
| 60 | 10 | 27.5 | 2.5 | 1661 | 3.75 | 1.97 | 6.18 | 76 | -8.4 | 59.7 | 0.099 |
| 60 | 5 | 30 | 5 | 1661 | 4.19 | 2.20 | 7.28 | 68 | -4.8 | 45.8 | 0.235 |
| 60 | 5 | 33.5 | 1.5 | 1661 | 3.48 | 1.83 | 5.35 | 82 | -10.8 | 73.2 | 0.065 |
| 60 | 5 | 33.5 | 1.5 | 1661 | 6.64 | 3.49 | 10.21 | 86 | -1.8 | 77.8 | 0.090 |
| 60 | 5 | 25 | 10 | 1661 | 6.79 | 3.57 | 16.10 | 42 | -4.6 | 72.6 | 0.165 |
| 60 | 5 | 32.5 | 2.5 | 1661 | 3.96 | 2.08 | 6.32 | 72 | -3.9 | 57.6 | 0.102 |
| 60 | 5 | 34 | 1 | 1661 | 3.75 | 1.97 | 5.67 | 76 | -16.3 | 83.5 | 0.171 |
| 60 | 5 | 34.5 | 0.5 | 1661 | 3.28 | 1.72 | 4.86 | 87 | -7.3 | 126.0 | 0.08 |
| 50 | 5 | 40 | 5 | 1661 | 3.96 | 2.08 | 7.94 | 72 | 0.2 | 56.9 | 0.1 |
| 60 | 5 | 30 | 5 | 1661 | 4.75 | 2.50 | 8.25 | 60 | -1.8 | 44.3 | 0.296 |
| 70 | 5 | 20 | 5 | 1661 | 5.00 | 2.63 | 7.74 | 57 | -2.9 | 38.9 | 0.223 |
| 80 | 5 | 10 | 5 | 1661 | 5.18 | 2.73 | 7.27 | 55 | -5.1 | 45.3 | 0.170 |
| 60 | 5 | 30 | 5 | 1661 | 13.60 | 7.14 | 23.57 | 42 | 0.3 | 50.2 | 0.186 |
| 60 | 5 | 30 | 5 | 1661 | 14.51 | 7.63 | 25.19 | 59 | 0.5 | 74.6 | 0.156 |
| 60 | 5 | 30 | 5 | 1661 | 6.20 | 3.26 | 10.76 | 46 | -9.8 | 60.6 | 0.153 |
| 60 | 5 | 30 | 5 | 1661 | 4.60 | 2.42 | 7.98 | 62 | 7.7 | 88.7 | 0.177 |
| 60 | 5 | 30 | 5 | 1661 | 6.20 | 3.26 | 10.76 | 46 | -5 | 44.2 | 0.353 |
| 60 | 5 | 30 | 5 | 1661 | 5.82 | 3.06 | 10.10 | 49 | -14.2 | 50.3 | 0.232 |
| 40 | 5 | 54 | 1 | 1661 | 3.39 | 1.79 | 7.02 | 84 | 0.496 | 95.9 | 0.046 |
| 40 | 7.5 | 51.5 | 1 | 1661 | 3.39 | 1.79 | 7.15 | 84 | 3.16 | 81.8 | 0.002 |
| 40 | 10 | 49 | 1 | 1661 | 3.39 | 1.79 | 7.29 | 84 | 0.652 | 85.6 | 0.017 |
| 50 | 5 | 44 | 1 | 1661 | 3.39 | 1.79 | 5.88 | 84 | 9.74 | 94.7 | 0.030 |
| 50 | 7.5 | 41.5 | 1 | 1661 | 3.43 | 1.81 | 6.06 | 83 | 10.7 | 86.7 | 0.033 |
| 50 | 10 | 39 | 1 | 1661 | 3.35 | 1.76 | 6.02 | 85 | 11.9 | 81.1 | 0.069 |
| 60 | 5 | 34 | 1 | 1661 | 3.52 | 1.85 | 5.32 | 81 | -11.7 | 88.1 | 0.010 |
| 60 | 7.5 | 31.5 | 1 | 1661 | 3.56 | 1.88 | 5.475 | 80 | -10.4 | 81.5 | 0.032 |
| 60 | 10 | 29 | 1 | 1661 | 3.80 | 2.00 | 5.946667 | 75 | -12.6 | 81.8 | 0.021 |
| 70 | 5 | 24 | 1 | 1661 | 3.70 | 1.95 | 5.012987 | 77 | -9.6 | 103.0 | 0.091 |
| 70 | 7.5 | 21.5 | 1 | 1661 | 4.13 | 2.17 | 5.681159 | 69 | -12.8 | 90.3 | 0.073 |

(continued)

| Composition (mole%) | | | | | | | | | | | |
|---|---|---|---|---|---|---|---|---|---|---|---|
| Lipid A | DSPC | Chol | PEG | siRNA | Lipid A/ siRNA | Charge ratio | Total Lipid/ siRNA | Entrapment (%) | Zeta potential | Particle Size (nm) | PDI |
| 70 | 10 | 19 | 1 | 1661 | 3.85 | 2.03 | 5.378378 | 74 | -14 | 87.7 | 0.043 |
| 60 | 5 | 34 | 1 | 1661 | 3.52 | 1.85 | 5.320988 | 81 | -7 | 81.1 | 0.142 |
| 60 | 5 | 34 | 1 | 1661 | 3.70 | 1.95 | 5.597403 | 77 | -5 | 94.0 | 0.090 |
| 60 | 5 | 34 | 1 | 1661 | 3.52 | 1.85 | 5.320988 | 81 | -8.2 | 83.6 | 0.096 |
| 60 | 7.5 | 27.5 | 5 | 1661 | 5.18 | 2.73 | 9.145455 | 55 | -5.92 | 39.6 | 0.226 |
| 60 | 7.5 | 29 | 3.5 | 1661 | 4.45 | 2.34 | 7.484375 | 64 | -7.8 | 49.6 | 0.100 |
| 60 | 5 | 31.5 | 3 3.5 | 1661 | 4.83 | 2.54 | 7.983 051 | 59 | -4.61 | 46.9 | 0.187 |
| 60 | 7.5 | 31 | 1.5 | 1661 | 3.48 | 1.83 | 5.439024 | 82 | -6.74 | 77.6 | 0.047 |
| 57.5 | 7.5 | 30 | 5 | 1661 | 4.75 | 2.50 | 8.666667 | 60 | -6.19 | 40.5 | 0.207 |
| 57.5 | 7.5 | 31.5 | 3.5 | 1661 | 4.83 | 2.54 | 8.372881 | 59 | -4.34 | 50.7 | 0.171 |
| 57.5 | 5 | 34 | 3.5 | 1661 | 4.67 | 2.46 | 7.983607 | 61 | -6.49 | 45.7 | 0.107 |
| 57.5 | 7.5 | 33.5 | 1.5 | 1661 | 3.43 | 1.81 | 5.554217 | 83 | -5.46 | 76.6 | 0.069 |
| 55 | 7.5 | 32.5 | 5 | 1661 | 4.38 | 2.31 | 8.276923 | 65 | -3.01 | 42.4 | 0.132 |
| 55 | 7.5 | 34 | 3.5 | 1661 | 4.13 | 2.17 | 7.42029 | 69 | -4.57 | 47.3 | 0.137 |
| 55 | 5 | 36.5 | 3.5 | 1661 | 4.38 | 2.31 | 7.753846 | 65 | -4.73 | 49.5 | 0.116 |
| 55 | 7.5 | 36 | 1.5 | 1661 | 3.35 | 1.76 | 5.611765 | 85 | -4.45 | 76.2 | 0.048 |

In one embodiment, the lipid formulation is entrapped by at least 75%, at least 80% or at least 90%.

In some embodiments, the lipid A of the formulations in Table 1 or Table 2, is substituted with another lipid, such as a Lipid T or a Lipid M.

In yet another embodiment, the formulation complexed with a nucleic acid based agent contains LNP05, LNP06, LNP07, LNP08, or LNP09 as described below:

| Formulation | Molar % of Lipid A/ DSPC/ Cholesterol/ PEG-DMG Lipid: siRNA ratio |
|---|---|
| LNP05 | 57.5/7.5/31.5/3.5<br>lipid:siRNA ~ 6 |
| LNP06 | 57.5/7.5/31.5/3.5,<br>lipid:siRNA ~ 11 |
| LNP07 | 60/7.5/31/1.5,<br>lipid:siRNA ~ 6 |
| LNP08 | 60/7.5/31/1.5,<br>lipid:siRNA ~ 11 |
| LNP09 | 50/10/38.5/1.5<br>lipid:siRNA ~11 |

In one embodiment, the lipid-containing formulation further includes an apolipoprotein. As used herein, the term "apolipoprotein" or "lipoprotein" refers to apolipoproteins known to those of skill in the art and variants and fragments thereof and to apolipoprotein agonists, analogues or fragments thereof described below.

Other suitable embodiments of the lipid formulation are disclosed in copending applications U.S.S.N. 61/171,439, filed April 21, 2009; U.S.S.N. 61/156,851, filed March 2,2009, and U.S.S.N. 61/175,770, filed May 5, 2009. The entire contents of each of these provisional applications are incorporated herein by reference.

In one aspect, a nucleic acid-based agent is complexed with lipid particle having the structure

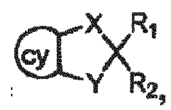

where cy is optionally substituted cyclic, optionally substituted heterocyclic or heterocycle, optionally substituted aryl or optionally substituted heteroaryl; $R_1$ and $R_2$ are each independently for each occurrence optionally substituted $C_{10}$-$C_{30}$ alkyl, optionally substituted $C_{10}$-$C_{30}$ alkenyl, optionally substituted $C_{10}$-$C_{30}$ alkynyl, optionally substituted $C_{10}$-$C_{30}$ acyl or-linker-ligand; X and Y are each independently O or S, alkyl or N(Q); and Q is H, alkyl, acyl, alkylamino or alkylphosphate.

In one embodiment, the nucleic acid-based agent is complexed with a lipid particle having a neutral lipid and a lipid capable of reducing particle aggregation. In one embodiment, the lipid particle consists essentially of (i) at least one lipid of the present invention; (ii) a neutral lipid selected from DSPC, DPPC, POPC, DOPE and SM; (iii) sterol, *e.g.* cholesterol; and (iv) peg-lipid, *e.g.* PEG-DMG or PEG-DMA, in a molar ratio of about 20-60% cationic lipid: 5-25% neutral lipid: 25-55% sterol; 0.5-15% PEG-lipid. In one embodiment, the lipid is optically pure.

In some embodiments, the lipid design has head groups with varying pKa, Cationic, 1°, 2° and 3°, monoamines, Di and triamines, Oligoamines/polyamines, Low pKa head groups - imidazoles and pyridine, guanidinium, anionic, zwitterionic and hydrophobic tails include symmetric and asymmetric chains, long and shorter, saturated and unsaturated chain the back bone includes backbone glyceride and other acyclic analogs, cyclic, spiro, bicyclic and polycyclic linkages with ethers, esters, phosphate and analogs, sulfonate and analogs, disulfides, pH sensitive linkages like acetals and ketals, imines and hydrazones, and oximes.

In one embodiment, the cationic lipid has the structure

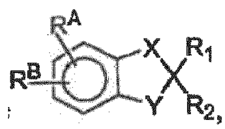

wherein:

$R_1$ and $R_2$ are each independently for each occurrence optionally substituted $C_{10}$-$C_{30}$ alkyl, optionally substituted $C_{10}$-$C_{30}$ alkenyl, optionally substituted $C_{10}$-$C_{30}$ alkynyl, optionally substituted $C_{10}$-$C_{30}$ acyl or -linker-ligand;
X and Y are each independently O or S, alkyl or N(Q);
Q is H, alkyl, acyl, alkylamino or alkylphosphate; and
$R^A$ and $R^B$ are each independently H, $R_3$, -Z'-$R_3$, -$(A_2)_j$-Z'-$R_3$, acyl, sulfonate or

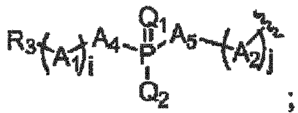
;

$Q_1$ is independently for each occurrence O or S;
$Q_2$ is independently for each occurrence O, S, N(Q), alkyl or alkoxy;
Q is H, alkyl, $\omega$-aminoalkyl, $\omega$-(substituted)aminoalky, $\omega$-phosphoalkyl or $\omega$-thiophosphoalkyl;
$A_1$, $A_4$, and $A_5$ are each independently 0, S, $CH_2$, CHF or $CF_2$;
Z' is O, S, N(Q) or alkyl;
i and j are independently 0 to 10; and
$R_3$ is H, optionally substituted $C_1$-$C_{10}$ alkyl, optionally substituted $C_2$-$C_{10}$ alkenyl, optionally substituted $C_2$-$C_{10}$ alkenyl, alkylheterocycle, alkylphosphate, alkylphosphorothioate, alkylphosphonates, alkylamines, hydroxyalkyls, $\omega$-aminoalkyls, $\omega$-(substituted)aminoalkyls, $\omega$-phosphoalkyls, $\omega$-thiophosphoalkyls, polyethylene glycol (PEG, mw 100-40K), mPEG (mw 120-40K), heteroaryl, heterocycle or linker-ligand.

Other suitable embodiments of the lipid formulation are disclosed in copending application U.S.S.N. 61/171,439, filed April 21, 2009, or U.S.S.N. 61/225,898, filed July 15, 2009, the entire contents of which are incorporated herein by reference.

In another embodiment, the formulation suitable for complexing with a nucleic acid based agent containing a Lipid T (also called LNP12, C12-200, or TechG1). Lipid T is described, *e.g.*, in Love et al. "Lipid-like materials for low-dose, in vivo gene silencing" Proc Natl Acad Sci U S A. 2010 107:1864-9 (incorporate by reference).

In a further embodiment, representative formulations prepared *via* the extrusion method or in-line mixing method for complexing with a nucleic acid-based agent are delineated in Table 3, where Lipid T is

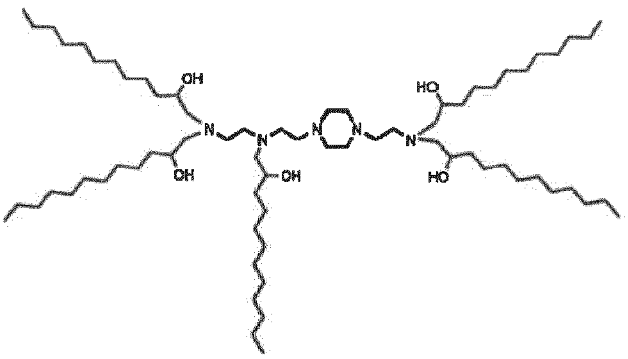

(Lipid T(V))

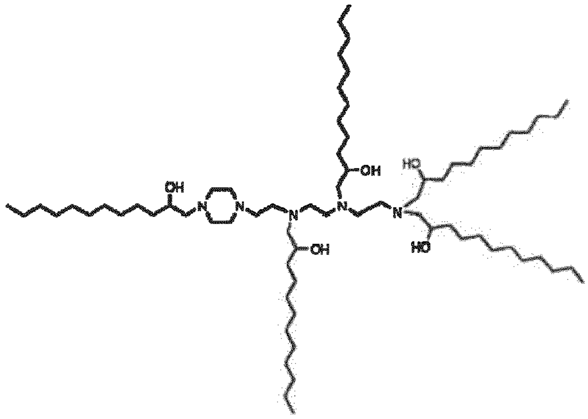

, (Lipid T(VI))

or combinations thereof:

Table 3

| Theoretical Composition (mole %) | | | | | Initial | | | Final (Entrapped) | | particle size (nm) | | |
|---|---|---|---|---|---|---|---|---|---|---|---|---|
| Lipid T | DSPC | Chol | PEG | siRNA | Lipid T/ siRNA | Total Lipid/ siRNA | Entrapment (%) | Lipid T/ siRNA | Total Lipid/ siRNA | Peak | width | PDI |
| 42 | 0 | 28 | 10 | 1661 | 4.75 | 9 | 58 | 8.19 | 15.52 | 89.6 | 31.7 | 0.133 |
| 42 | 0 | 28 | 10 | 1661 | 4.75 | 9 | 77 | 6.17 | 11.69 | 126 | 43.6 | 0.07 |
| 42 | 0 | 28 | 10 | 1661 | 4.75 | 9 | 24 | 19.79 | 37.50 | 37.3 | 13.4 | 0.194 |
| 50 | 0 | 40 | 10 | 1661 | 4.75 | 8.19 | 58 | 8.19 | 14.12 | 121 | 47.5 | 0.109 |
| 60 | 0 | 30 | 10 | 1661 | 4.75 | 7.35 | 43 | 11.05 | 17.09 | 117 | 48.1 | 0.095 |
| 55 | 0 | 40 | 5 | 1661 | 4.75 | 6.9 | 62 | 7.66 | 11.13 | 160 | 64.2 | 0.096 |
| 65 | 0 | 30 | 5 | 1661 | 4.75 | 6.32 | 41 | 11.59 | 15.41 | 164 | 59 | 0.086 |
| 40 | 10 | 40 | 10 | 1661 | 4.75 | 9.05 | 72 | 6.60 | 12.57 | 118 | 46.4 | 0.113 |
| 50 | 7.5 | 37.5 | 5 | 1661 | 4.75 | 7.03 | 79 | 6.01 | 8.90 | 131 | 61.1 | 0.126 |
| 50 | 0 | 40 | 10 | 1661 | 4.75 | 8.19 | 57 | 8.33 | 14.37 | 88.3 | 28.9 | 0.068 |
| 60 | 0 | 30 | 10 | 1661 | 4.75 | 7.35 | 35 | 13.57 | 21.00 | 84.7 | 33.6 | 0.099 |
| 55 | 0 | 40 | 5 | 1661 | 4.75 | 6.9 | 49 | 9.69 | 14.08 | 136 | 33.3 | 0.029 |
| 65 | 0 | 30 | 5 | 1661 | 4.75 | 6.32 | 26 | 18.27 | 24.31 | 98.3 | 33.2 | 0.096 |
| 40 | 10 | 40 | 10 | 1661 | 4.75 | 9.05 | 70 | 6.79 | 12.93 | 80.2 | 30.4 | 0.14 |
| 50 | 7.5 | 37.5 | 5 | 1661 | 4.75 | 7.03 | 68 | 6.99 | 10.34 | 103 | 33.9 | 0.082 |
| 57.5 | 7.5 | 31.5 | 3.5 | 1661 | 4.75 | 6.29 | 66 | 7.20 | 9.53 | 101 | 19.4 | 0.344 |
| 57.5 | 7.5 | 31.5 | 3.5 | 1661 | 4.75 | 6.29 | 83 | 5.72 | 7.58 | 144 | 58.4 | 0.087 |
| 57.5 | 7.5 | 31.5 | 3.5 | 1661 | 4.75 | 6.29 | 90 | 5.28 | 6.99 | 181 | 58.6 | 0.042 |
| 57.5 | 7.5 | 31.5 | 3.5 | 1661 | 4.75 | 6.29 | 60 | 7.92 | 10.48 | 95.2 | 33.1 | 0.153 |
| 40 | 7.5 | 32.5 | 20 | 1661 | 4.75 | 11.43 | 74 | 6.42 | 15.45 | 77.8 | 34.2 | 0.131 |
| 50 | 7.5 | 22.5 | 20 | 1661 | 4.75 | 9.77 | 48 | 9.90 | 20.35 | 96.5 | 37.7 | 0.152 |
| 57.5 | 7.5 | 31.5 | 3.5 | 1661 | 4.75 | 6.29 | 54 | 8.80 | 11.65 | 86.9 | 34.9 | 0.094 |
| 40 | 7.5 | 32.5 | 20 | 1661 | 4.75 | 11.43 | 76 | 6.25 | 15.04 | 85.3 | 33.6 | 0.096 |
| 57.5 | 7.5 | 31.5 | 3.5 | 1661 | 4.75 | 6.29 | 10 | 47.50 | 62.90 | 107 | 58.4 | 0.148 |

(continued)

| Theoretical Composition (mole %) | | | | | Initial | | | Final (Entrapped) | | particle size (nm) | | |
|---|---|---|---|---|---|---|---|---|---|---|---|---|
| Lipid T | DSPC | Chol | PEG | siRNA | Lipid T/ siRNA | Total Lipid/ siRNA | Entrapment (%) | Lipid T/ siRNA | Total Lipid/ siRNA | Peak | width | PDI |
| 57.5 | 7.5 | 31.5 | 3.5 | 1661 | 4.75 | 6.29 | 82 | 5.79 | 7.67 | 150 | 59.3 | 0.092 |
| 57.5 | 7.5 | 31.5 | 3.5 | 1661 | 4.75 | 6.29 | 73 | 6.51 | 8.62 | 113 | 37.1 | 0.094 |
| 57.5 | 7.5 | 31.5 | 3.5 | 1661 | 4.75 | 6.29 | 71 | 6.69 | 8.86 | 115 | 37.9 | 0.068 |
| 57.5 | 7.5 | 31.5 | 3.5 | 1661 | 4.75 | 6.72 | 13 | 36.54 | 51.69 | 39.9 | 12 | 0.265 |
| 57.5 | 7.5 | 31.5 | 3.5 | 1661 | 4.75 | 6.29 | 40 | 11.88 | 15.73 | 55.6 | 18.9 | 0.109 |
| 50 | 7.5 | 37.5 | 5 | 1955 | 4.75 | 7.03 | 93 | 5.11 | 7.56 | 122 | 45.7 | 0.083 |
| 50 | 7.5 | 37.5 | 5 | 3215 | 4.75 | 7.03 | 79 | 6.01 | 8.90 | 102 | 35 | 0.122 |
| 60 | 7.5 | 31 | 1.5 | 1661 | 4.75 | 6.26 | 79 | 6.01 | 7.92 | 191 | 70.5 | 0.096 |
| 55 | 7.5 | 32.5 | 5 | 1661 | 4.75 | 7.13 | 80 | 5.94 | 8.91 | 132 | 41 | 0.056 |
| 55 | 7.5 | 32.5 | 5 | 1661 | 4.75 | 7.13 | 40 | 11.88 | 17.83 | 73.2 | 24.6 | 0.096 |
| 55 | 7.5 | 32.5 | 5 | 1661 | 4.75 | 7.13 | 43 | 11.05 | 16.58 | 71.6 | 20 | 0.07 |
| 60 | 7.5 | 31 | 1.5 | 1661 | 4.75 | 6.26 | 60 | 7.92 | 10.43 | 61.9 | 19.7 | 0.064 |
| 60 | 7.5 | 31.5 | 1 | 1661 | 4.75 | 6.19 | 48 | 9.90 | 12.90 | 113 | 93.8 | 0.238 |
| 60 | 7.5 | 31 | 1.5 | 1661 | 4.75 | 6.26 | 41 | 11.59 | 15.27 | 156 | 81.1 | 0.132 |
| 60 | 7.5 | 31 | 1.5 | 1661 | 4.75 | 6.26 | 29 | 16.38 | 21.59 | 115 | 79.8 | 0.204 |
| 60 | 0 | 38.5 | 1.5 | 1661 | 4.75 | 6.05 | 17 | 27.94 | 35.59 | 139 | 77.8 | 0.184 |
| 60 | 7.5 | 31 | 1.5 | 1661 | 4.75 | 6.26 | 73 | 6.51 | 8.58 | 75.1 | 19.6 | 0.04 |
| 60 | 7.5 | 31 | 1.5 | 1661 | 4.75 | 6.26 | 74 | 6.42 | 8.46 | 71.3 | 25.7 | 0.091 |
| 60 | 7.5 | 31 | 1.5 | 1661 | 4.75 | 6.26 | 69 | 6.88 | 9.07 | 80.1 | 28 | 0.082 |
| 60 | 7.5 | 31 | 1.5 | 1661 | 9.5 | 12.53 | 70 | 13.57 | 17.90 | 69.8 | 22.5 | 0.09 |
| 50 | 10 | 38.5 | 1.5 | 1661 | 4.75 | 6.97 | 77 | 6.17 | 9.05 | 64 | 26.1 | 0.127 |
| 60 | 0 | 38.5 | 1.5 | 1661 | 4.75 | 6.05 | 51 | 9.31 | 11.86 | 64 | 21.9 | 0.088 |
| 40 | 20 | 38.5 | 1.5 | 1661 | 4.75 | 8.36 | 86 | 5.52 | 9.72 | 59.7 | 21.1 | 0.151 |
| 50 | 10 | 38.5 | 1.5 | 18747 | 4.75 | 6.97 | N/A | N/A | N/A | 70.3 | 22.6 | 0.034 |

(continued)

| Theoretical Composition (mole %) | | | | | Initial | | | Final (Entrapped) | | particle size (nm) | | |
|---|---|---|---|---|---|---|---|---|---|---|---|---|
| Lipid T | DSPC | Chol | PEG | siRNA | Lipid T/ siRNA | Total Lipid/ siRNA | Entrapment (%) | Lipid T/ siRNA | Total Lipid/ siRNA | Peak | width | PDI |
| 45 | 15 (DOPC) | 38.5 | 1.5 | 1661 | 4.75 | 7.58 | 82 | 5.79 | 9.24 | 70 | 19.4 | 0.043 |
| 45 | 15 (DMPC) | 38.5 | 1.5 | 1661 | 4.75 | 7.43 | 81 | 5.86 | 9.17 | 57.2 | 17.1 | 0.081 |
| 45 | 15 | 38.5 | 1.5 | 1661 | 4.75 | 7.59 | 81 | 5.86 | 9.37 | 54.4 | 17.3 | 0.118 |
| 50 | 10 | 38.5 | 1.5 (C10) | 1661 | 4.75 | 6.97 | 79 | 6.01 | 8.82 | 75.5 | 45.2 | 0.2 |
| 50 | 10 | 38.5 | 1.5 (C18) | 1661 | 4.75 | 6.98 | 81 | 5.86 | 8.62 | 64.1 | 18.4 | 0.069 |

In one embodiment, a formulation containing a lipid, and complexed with a nucleic acid-based agent can include: a sterol; a neutral lipid; a PEG or a PEG-modified lipid; and a cationic lipid of formula (I)

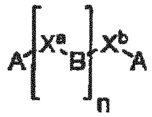

wherein,
formula (I)

each $X^a$ and $X^b$, for each occurrence, is independently $C_{1-6}$alkylene;
n is 0, 1, 2, 3, 4, or 5;
A for each occurrence is $NR_2$ or a cyclic moiety optionally substituted with 1-3R;
B is NR or a cyclic moiety optionally substituted with 1-2 R;
each R is independently H, alkyl,

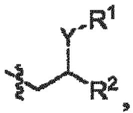, or 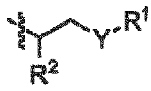 ;

provided that at least one R is

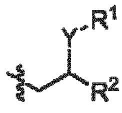, or 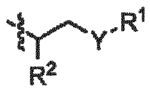 ;

$R^1$, for each occurrence, is independently H, $R^3$,

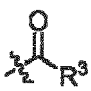, 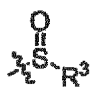, 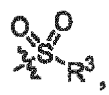,

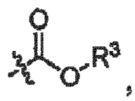, 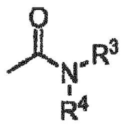, or 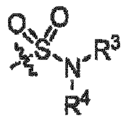 ;

$R^2$, for each occurrence, is independently, alkyl, alkenyl, alkynyl, heteroalkyl, heteroalkenyl, or heteroalkynyl; each of which is optionally substituted with one or more substituent;
$R^3$, for each occurrence, is independently, alkyl, alkenyl, alkynyl, heteroalkyl, heteroalkenyl, or heteroalkynyl; each of which is optionally substituted with one or more substituent (e.g., a hydrophilic substituent);
Y, for each occurrence, is independently O, $NR^4$, or S;
$R^4$, for each occurrence is independently H alkyl, alkenyl, alkynyl, heteroalkyl, heteroalkenyl, or heteroalkynyl; each of which is optionally substituted with one or more substituent.

In one embodiment, the compound of formula (I) includes at least 2 or three nitrogens, and in another embodiment, n is 1, 2, or 3. In another embodiment at least one A is a cyclic moiety, e.g, a nitrogen containing cyclic moiety, a piperidinyl or piperizinyl moiety. In another embodiment, at least one B is a cyclic moiety, e.g., a nitrogen containing cyclic moiety. In another embodiment, at least one B is a piperidinyl or piperizinyl moiety.

In one embodiment, the formulation includes a sterol; a PEG or a PEG-modified lipid, a neutral lipid and a cationic lipid of formula (II):

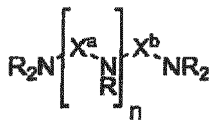

formula (II)

each $X^a$ and $X^b$, for each occurrence, is independently $C_{1-6}$ alkylene;
n is 0, 1, 2, 3, 4, or 5;
each R is independently H, alkyl,

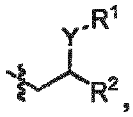, 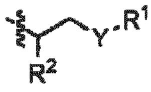,

or two Rs, together with the nitrogen to which they are attached form a ring; provided that at least one R

is 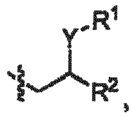, or 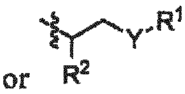;

$R^1$, for each occurrence, is independently H, $R^3$,

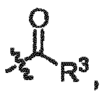, 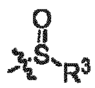, 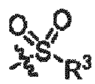,

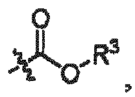, 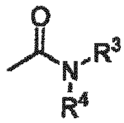, or 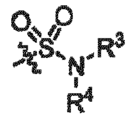;

wherein $R^3$ is optionally substituted with one or more substituent;
$R^2$, for each occurrence, is independently, alkyl, alkenyl, alkynyl, heteroalkyl, heteroalkenyl, or heteroalkynyl; each of which is optionally substituted with one or more substituent;
$R^3$, for each occurrence, is independently, alkyl, alkenyl, alkynyl, heteroalkyl, heteroalkenyl, or heteroalkynyl; each of which is optionally substituted with one or more substituent;
Y, for each occurrence, is independently O, $NR^4$, or S;
$R^4$, for each occurrence is independently H alkyl, alkenyl, alkynyl, heteroalkyl, heteroalkenyl, or heteroalkynyl; each of which is optionally substituted with one or more substituent.

In another embodiment, the formulation containing a lipid, and complexed with a nucleic acid based agent includes a sterol; a neutral lipid; a PEG or a PEG-modified lipid; and a compound of formula (III), (VI) or a mixture thereof,

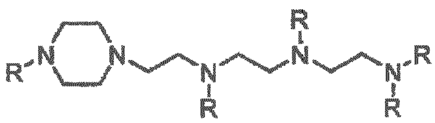 or 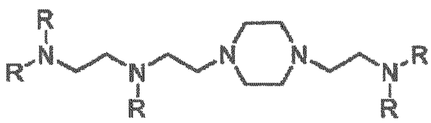

formula (III) formula (IV),

wherein each R is independently H, alkyl,

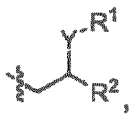, or 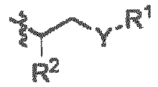 ;

provided that at least one R is

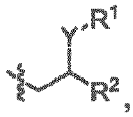, or 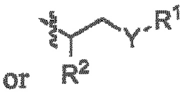 ;

wherein $R^1$, for each occurrence, is independently H, $R^3$,

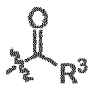, 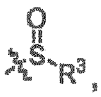, 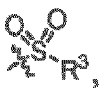, 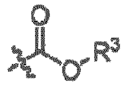, 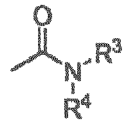,

or

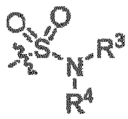 ;

wherein $R^3$ is optionally substituted with one or more substituent;

$R^2$, for each occurrence, is independently, alkyl, alkenyl, alkynyl, heteroalkyl, heteroalkenyl, or heteroalkynyl; each of which is optionally substituted with one or more substituent;

$R^3$, for each occurrence, is independently, alkyl, alkenyl, alkynyl, heteroalkyl, heteroalkenyl, or heteroalkynyl; each of which is optionally substituted with one or more substituent;

Y, for each occurrence, is independently O, $NR^4$, or S;

$R^4$, for each occurrence is independently H alkyl, alkenyl, alkynyl, heteroalkyl, heteroalkenyl, or heteroalkynyl; each of which is optionally substituted with one or more substituent.

In one embodiment, the formulation contains a Lipid T. Lipid T is a composition containing a sterol; a neutral lipid; a PEG or a PEG-modified lipid; and a compound of formula (III), (VI) or a mixture thereof,

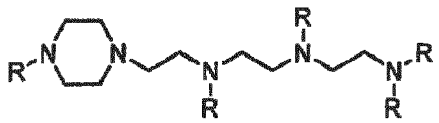 or 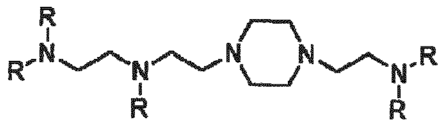

formula (III) formula (IV),

wherein each R is independently H, alkyl,

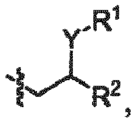, or 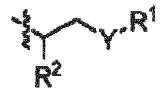 ;

provided that at least one R is

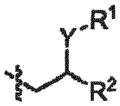, or 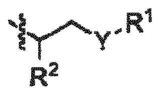 ;

wherein $R^1$, for each occurrence, is independently H, $R^3$,

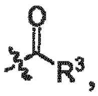, 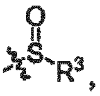, 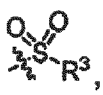, 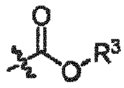, 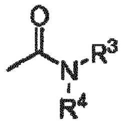,

or

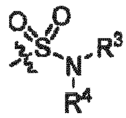 ;

wherein $R^3$ is optionally substituted with one or more substituent;
$R^2$, for each occurrence, is independently, alkyl, alkenyl, alkynyl, heteroalkyl, heteroalkenyl, or heteroalkynyl; each of which is optionally substituted with one or more substituent;
$R^3$, for each occurrence, is independently, alkyl, alkenyl, alkynyl, heteroalkyl, heteroalkenyl, or heteroalkynyl; each of which is optionally substituted with one or more substituent;
Y, for each occurrence, is independently O, $NR^4$, or S;
$R^4$, for each occurrence is independently H alkyl, alkenyl, alkynyl, heteroalkyl, heteroalkenyl, or heteroalkynyl; each of which is optionally substituted with one or more substituent; and
a compound of of formula (V) or formula (VI) below, or a mixture of Formulas (V) and (VI).

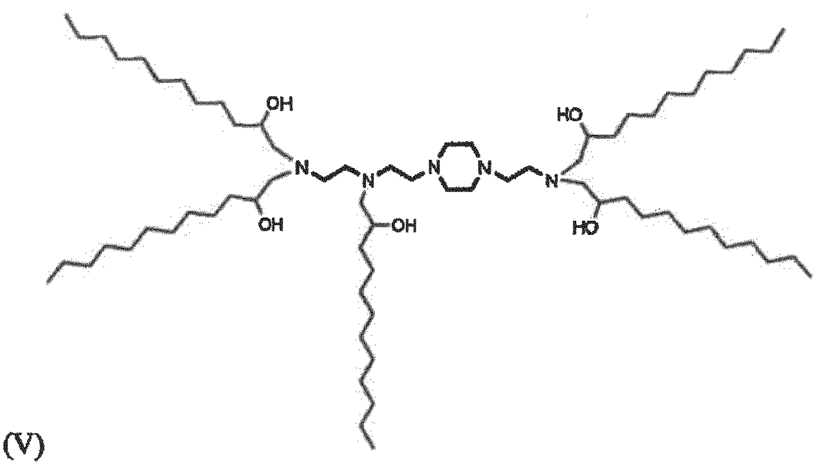

Formula (V)

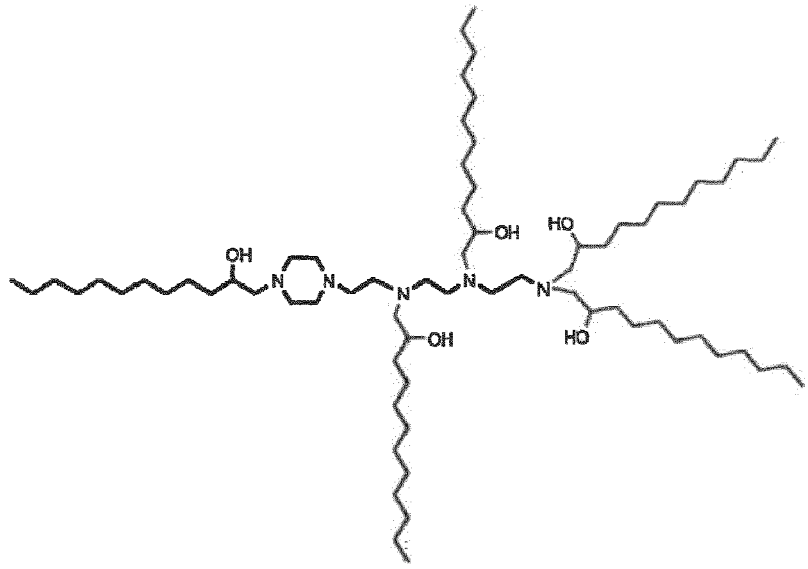

formula (VI).

In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter).

In another embodiment, the formulation containing a lipid includes a compound of formula (V), he compound of the following formula:

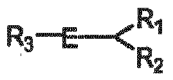 formula (V)

wherein:

$R_1$ and $R_2$ are each independently for each occurrence optionally substituted $C_{10}$-$C_{30}$ alkyl, optionally substituted $C_{10}$-$C_{30}$ alkoxy, optionally substituted $C_{10}$-$C_{30}$ alkenyl, optionally substituted $C_{10}$-$C_{30}$ alkenyloxy, optionally substituted $C_{10}$-$C_{30}$ alkynyl, optionally substituted $C_{10}$-$C_{30}$ alkynyloxy, or optionally substituted $C_{10}$-$C_{30}$ acyl;

E is -O-, -S-, -N(Q)-, -C(O)O-, -OC(O)-, -C(O)-, -N(Q)C(O)-, -C(O)N(Q)-, -N(Q)C(O)O-, -OC(O)N(Q)-, S(O), -N(Q)S(O)$_2$N(Q)-, -S(O)$_2$-, -N(Q)S(O)$_2$-, -SS-, - O-N=, =N-O-, -C(O)-N(Q)-N=, -N(Q)-N=, -N(Q)-O-, -C(O)S-, arylene, heteroarylene, cyclalkylene, or heterocyclylene; and

Q is H, alkyl, co-aminoalkyl, $\omega$-(substituted)aminoalkyl, $\omega$-phosphoalkyl or $\omega$-thiophosphoalkyl;

$R_3$ is H, optionally substituted $C_1$-$C_{10}$ alkyl, optionally substituted $C_2$-$C_{10}$ alkenyl, optionally substituted $C_2$-$C_{10}$ alkynyl, optionally substituted alkylheterocycle, optionally substituted heterocyclealkyl, optionally substituted alkylphosphate, optionally substituted phosphoalkyl, optionally substituted alkylphosphorothioate, optionally substituted phosphorothioalkyl, optionally substituted alkylphosphorodithioate, optionally substituted phosphorodithioalkyl, optionally substituted alkylphosphonate, optionally substituted phosphonoalkyl, optionally substituted amino, optionally substituted alkylamino, optionally substituted di(alkyl)amino, optionally substituted aminoalkyl, optionally substituted alkylaminoalkyl, optionally substituted di(alkyl)aminoalkyl, optionally substituted hydroxyalkyl, optionally substituted polyethylene glycol (PEG, mw 100-40K), optionally substituted mPEG (mw 120-40K), optionally substituted heteroaryl, optionally substituted heterocycle, or linker-ligand.

In one embodiment, the lipid of formula (V) is 6Z,9Z,28Z,31Z)-heptatriaconta-6,9,28,31-tetraen-19-yl 4-(dimethylamino)butanoate (also called "DLin-M-C3-DMA," "MC3," and "Lipid M"), which has the following structure:

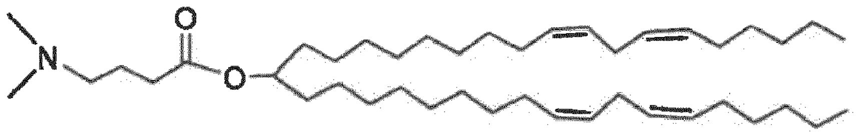

In this embodiment,
$R^1$ and $R^2$ are both linoleyl, and
E is C(O)O;
$R^3$ is a dimethylaminopropyl.

In one embodiment, the lipid is a racemic mixture.

In one embodiment, the lipid is enriched in one diastereomer, e.g. the lipid has at least 95%, at least 90%, at least 80% or at least 70% diastereomeric execess.

In one embodiment, the lipid is chirally pure, e.g. is a single isomer.

In one embodiment, the lipid is enriched for one isomer.

In one embodiment, the formulations of the invention are entrapped by at least 75%, at least 80% or at least 90%.

*Target Genes Expressed in Immune Cells*

The compositions described herein, *e.g.*, the nucleic acid-based agents complexed with lipid-containing formulations, are characterized by having enhanced uptake into immune cells. Thus, the target gene of the nucleic acid-based agent (*e.g.*, the dsRNA) is typically a gene expressed in an immune cell. For example, the target gene can be CD33, CD4, CD25, CD8, CD29, CD11 (*e.g.*, CD11a, b, and c), CD19, CD40, CD31, CD45, CD38, CD116, CD28, NK1.1, TCR-beta, GR-1, CD69, CD122, IL-2, or IL-6

The effect of the expression of the target gene, *e.g.*, CD45, is evaluated by measuring CD45 levels in a biological sample, such as a blood, serum, urine or tissue sample. In one embodiment, the level of target gene expression from the synovial fluid of a patient, *e.g.*, a patient who has arthritis, is assayed.

In one embodiment, the level of mRNA in cells from the peritoneal cavity is evaluated. In another embodiment, at least two types of evaluation are made, *e.g.,* an evaluation of protein level (*e.g.*, in blood), and a measure of mRNA level (*e.g.*, in cells from the peritoneal cavity) are both made.

In another embodiment, the composition containing the nucleic acid-based agent and lipid-containing formulation is taken up by an immune cell, such as a leukocyte, *e.g.*, a lymphocyte, such as a B cell or a T cell. The composition is absorbed, for example, by a macrophage, a dendritic cell, a T regulatory cell (Treg), an NK (natural killer) cell, a monocyte, a myeloid cell, a granulocyte, or a neutrophil. In other embodiments, the composition is taken up by, for example, a [$CD5^+$ $CD11^-$ cell] (*e.g.*, a T cell); a [$CD19^+$ IgM cell] or [$CD19^+$ IgD cell] (*e.g.,* a B cell); a $CD5^-$ $CD11b^+$ $CD11c^-$ cell] (*e.g.*, a myeloid cell); or a [$CD5^-$ $CD11b^+$ $CD11c^+$ cell] (*e.g.*, a dendritic cell). In some embodiments, the composition is taken up by a $CD11b^+$ cell, *e.g.*, a macrophage or granulocyte, or a $CD11c^+$ cell. In another embodiment, the nucleic acid-based agent of the construct, *e.g.*, the dsRNA, inhibits expression of a gene expressed in the immune cell, *e.g.*, a CD45 gene.

The immune cells having enhanced uptake of the compositions described herein can be the peritoneal cavity or in the bone marrow. In some embodiments, the immune cells are circulating cells, such as *in* plasma or blood, and in other embodiments, or in addition, the target immune cells are in the spleen, or liver. In other embodiments, the immune cells having enhanced uptake of the lipid compositions are at a site of inflammation, e.g., at an arthritic joint. Typically, the compositions display enhanced uptake in immune cells, *e.g.*, macrophages and dendritic cells, in the peritoneal cavity.

In one embodiment, at various time points after administration of a candidate nucleic-acid based agent, a biological sample, such as a fluid sample, *e.g.*, blood, plasma, or serum, or a tissue sample, is taken from the test subject and tested for an effect of the agent on target protein or mRNA expression levels. For example, in one embodiment, the candidate agent is a dsRNA that targets a CD45, and the biological sample is tested for an effect on CD45 protein or mRNA levels. In one embodiment, plasma levels of CD45 protein are assayed, such as by using an immunohistochemistry assay or a chromogenic assay. In another embodiment, levels of CD45 mRNA, *e.g.*, from cells of the peritoneal cavity or bone marrow, are tested by an assay, such as a branched DNA assay, or a Northern blot or RT-PCR assay.

In one embodiment, the composition, *e.g.*, a nucleic acid-based agent complexed with a lipid formulation, is evaluated for toxicity. In yet another embodiment, a subject treated with the composition can be monitored for physical effects, such as by a change in weight or cageside behavior. In one embodiment the synovial fluid of a patient having arthritis is monitored for a decrease in the number of macrophages in the synovial fluid of affected tissues.

*Nucleic Acid-based agents*

Nucleic acid-based agents suitable for use in the compositions described herein, *e.g.*, the lipid formulated compositions described herein, include single-stranded DNA or RNA, or double-stranded DNA or RNA, or DNA-RNA hybrid. For example, a double-stranded DNA can be a structural gene, a gene including control and termination regions, or a self-replicating system such as a viral or plasmid DNA. A double-stranded RNA can be, *e.g.*, a dsRNA or another RNA interference reagent. A single-stranded nucleic acid can be, *e.g.,* an antisense oligonucleotide, ribozyme, microRNA, or triplex-forming oligonucleotide. Immunostimulatory oligonucleotides, or triplex-forming oligonucleotides are also suitable for use in the compositions usefule for enhanced targeting to immune cells. These agents are also described in greater detail below.

As used herein "Alkyl" means a straight chain or branched, noncyclic or cyclic, saturated aliphatic hydrocarbon containing from 1 to 24 carbon atoms. Representative saturated straight chain alkyls include methyl, ethyl, n-propyl, n-butyl, n-pentyl, n-hexyl, and the like; while saturated branched alkyls include isopropyl, *sec*-butyl, isobutyl, *tert*-butyl, isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like.

"Alkenyl" means an alkyl, as defined above, containing at least one double bond between adjacent carbon atoms. Alkenyls include both cis and trans isomers. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

"Alkynyl" means any alkyl or alkenyl, as defined above, which additionally contains at least one triple bond between adjacent carbons. Representative straight chain and branched alkynyls include acetylenyl, propynyl, 1-butynyl, 2-butynyl, 1-pentynyl, 2-pentynyl, 3-methyl-1 butynyl, and the like.

"Acyl" means any alkyl, alkenyl, or alkynyl wherein the carbon at the point of attachment is substituted with an oxo group, as defined below. For example, - C(=O)alkyl, -C(=O)alkenyl, and -C(=O)alkynyl are acyl groups.

"Heterocycle" means a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined below. Heterocycles include morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" means that, when substituted, at least one hydrogen atom is replaced with a substituent In the case of an oxo substituent (=O) two hydrogen atoms are replaced. In this regard, substituents include oxo, halogen, heterocycle, - CN, $-OR^x$, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^y$, wherein n is 0, 1 or 2, $R^x$ and $R^y$ are the same or different and independently hydrogen, alkyl or heterocycle, and each of said alkyl and heterocycle substituents may be further substituted with one or more of oxo, halogen, -OH, -CN, alkyl, $-OR^x$, heterocycle, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^y$.

"Halogen" means fluoro, chloro, bromo and iodo.

In some embodiments, the lipid formulations for use with nucleic acid-based agents may require the use of protecting groups. Protecting group methodology is well known to those skilled in the art (*see*, *for example*, PROTECTIVE GROUPS IN ORGANIC SYNTHESIS, Green, T.W. et. al., Wiley-Interscience, New York City, 1999). Briefly, protecting groups within the context of this invention are any group that reduces or eliminates unwanted reactivity of a functional group. A protecting group can be added to a functional group to mask its reactivity during certain reactions and then removed to reveal the original functional group. In some embodiments an "alcohol protecting group" is used. An "alcohol protecting group" is any group which decreases or eliminates unwanted reactivity of an alcohol functional group. Protecting groups can be added and removed using techniques well known in the art.

*Nucleic Acid-Lipid Particles*

In certain embodiments, the compositions featured herein include a nucleic acid-based agent complexed with a lipid particle. In particular embodiments, the nucleic acid is fully encapsulated in the lipid particle. As used herein, the term "nucleic acid" is meant to include any oligonucleotide or polynucleotide. Fragments containing up to 50 nucleotides are generally termed oligonucleotides, and longer fragments are called polynucleotides. In particular embodiments, oligonucletoides are 20-50 nucleotides in length.

In the context of this invention, the terms "polynucleotide" and "oligonucleotide" refer to a polymer or oligomer

of nucleotide or nucleoside monomers consisting of naturally occurring bases, sugars and intersugar (backbone) linkages. The terms "polynucleotide" and "oligonucleotide" also includes polymers or oligomers comprising non-naturally occurring monomers, or portions thereof, which function similarly. Such modified or substituted oligonucleotides are often substituted for the native forms because of properties such as, for example, enhanced cellular uptake and increased stability in the presence of nucleases.

Oligonucleotides are classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose.

The nucleic acid that is present in a lipid-nucleic acid particle according to this invention includes any form of nucleic acid that is known. The nucleic acids used herein can be single-stranded DNA or RNA, or double-stranded DNA or RNA, or DNA-RNA hybrids. Examples of double-stranded DNA include structural genes, genes including control and termination regions, and self-replicating systems such as viral or plasmid DNA. Examples of double-stranded RNA include siRNA and other RNA interference reagents. Single-stranded nucleic acids include, *e.g.*, antisense oligonucleotides, ribozymes, microRNA, and triplex-forming oligonucleotides.

Nucleic acid-based agent can be of various lengths, and the length generally depends on the particular form of nucleic acid. For example, in particular embodiments, plasmids or genes may be from about 1,000 to 100,000 nucleotide residues in length. In particular embodiments, oligonucleotides may range from about 10 to 100 nucleotides in length. In various related embodiments, oligonucleotides (including single-stranded, double-stranded, and triple-stranded), may range in length from about 10 to about 50 nucleotides, from about 20 o about 50 nucleotides, from about 15 to about 30 nucleotides, from about 20 to about 30 nucleotides in length.

In particular embodiments, an oligonucleotide (or a strand thereof) present in the composition specifically hybridizes to or is complementary to a target polynucleotide. "Specifically hybridizable" and "complementary" are terms that are used to indicate a sufficient degree of complementarity such that stable and specific binding occurs between the DNA or RNA target and the oligonucleotide. It is understood that an oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility or expression therefrom, and there is a sufficient degree of complementarity to avoid non-specific binding of the oligonucleotide to non-target sequences under conditions in which specific binding is desired, *i.e.*, under physiological conditions in the case of *in vivo* assays or therapeutic treatment, or, in the case of *in vitro* assays, under conditions in which the assays are conducted. Thus, in other embodiments, this oligonucleotide includes 1, 2, or 3 base substitutions as compared to the region of a gene or mRNA sequence that it is targeting or to which it specifically hybridizes.

In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter).

In some embodiments, the polydispersity index (PDI) of the particles is less than about 0.5 (e.g., less than about 0.4, less than about 0.3, less than about 0.2, or less than about 0.1).

## *Method of Use*

The compositions featured herein, *e.g.*, having a nucleic acid-based agent complexed with a lipid-containing formulation, are used to deliver the agent to an immune cell, *e.g.*, *in vitro* or *in vivo.* Typical nucleic acids for introduction into cells are dsRNA, immune-stimulating oligonucleotides, plasmids, antisense and ribozymes. These methods may be carried out by contacting the particles or compositions featured herein with the cells for a period of time sufficient for intracellular delivery to occur.

The compositions described herein can be used to treat a disorder characterized by overexpression or unwanted expression of a gene expressed in an immune cell. For example, a composition containing a nucleic acid-based agent, such as a dsRNA, complexed with a lipid-containing formulation, can be used to treat an autoimmune disorder, such as arthritis, artheroslerosis, psoriasis, lupus or IBD (*e.g.*, Crohn's disease or ulcerative colitis). For example, a composition featured herein can have enhanced uptake into a dendritic cell, where, for example, the nucleic acid-based agent targets CD45 expression, and the result can relieve one or more symptoms of IBD.

In another embodiment, a composition containing a nucleic acid-based agent, such as a dsRNA, complexed with a lipid formulation, can be used to treat an inflammatory disorder, such as arthritis. In yet another embodiment, a composition containing a nucleic acid-based agent, such as a dsRNA, complexed with a lipid formulation, is used to treat a cancer, such as a hematological malignancy, *e.g.*, acute myeloid leukemia (AML) or myelodysplastic syndrome. In other embodiments, enhanced uptake of the featured compositions into immune cells is useful for the treatment of non-Hodgkin's lymphoma, prostate cancer, colorectal cancer, multiple myeloma, or non-small cell lung cancer.

In one embodiment, the compositions featured herein are used in *ex vivo* therapy. For example, a composition containing a nucleic acid-based agent complexed with a lipid formulation can be contacted with an immune cell *(e.g.,* a dendritic cell) *in vitro,* such that that the agent is taken up by the cell, and expression of the target gene is decreased. The cell is then transferred to a patient (*e.g.*, by injection) to treat a disorder, *e.g.*, a cancer or autoimmune disease. In one embodiment, immune cells (*e.g.*, dendritic cells) are extracted from the patient, contacted with the nucleic acid based agent in lipid formulation such that the agent is taken up into the cells where it decreases gene expression, and then the cells are reintroduced into the patient. This *ex vivo* therapy is effective to treat a disorder in the patient, such as a cancer, *e.g.*, non-Hodgkin's lymphoma.

The compositions featured herein can be adsorbed to almost any cell type, but are particularly targeted to and adsorbed by immune cells. Once adsorbed, the nucleic acid-lipid particles can either be endocytosed by a portion of the cells, exchange lipids with cell membranes, or fuse with the cells. Transfer or incorporation of the nucleic acid portion of the complex can take place via any one of these pathways. In some embodiments, where particles are taken up into a cell by endocytosis, the particles can interact with the endosomal membrane, resulting in destabilization of the endosomal membrane, possibly by the formation of non-bilayer phases, resulting in introduction of the encapsulated nucleic acid into the cytoplasm of the immune cell. Similarly, in the case of direct fusion of the particles with the cell plasma membrane, when fusion takes place, the liposome membrane is integrated into the immune cell membrane and the contents of the liposome combine with the intracellular fluid. Contact between the cells and the lipid-nucleic acid compositions, when carried out *in vitro,* will take place in a biologically compatible medium. The concentration of compositions can vary widely depending on the particular application, but is generally between about 1 μmol and about 10 mmol. In certain embodiments, treatment of the cells with the lipid-nucleic acid compositions will generally be carried out at physiological temperatures (about 37°C) for periods of time from about 1 to 24 hours, such as from about 2 to 8 hours. For *in vitro* applications, the delivery of nucleic acids can be to an immune cell (*e.g.*, a macrophage or dendritic cell) grown in culture, whether of plant or animal origin, vertebrate or invertebrate, and of any tissue or type. In certain embodiments, the cells will be animal cells, *e.g.*, mammalian cells, such as human cells.

Typical applications include using well known procedures to provide intracellular delivery of dsRNA to knock down or silence specific cellular targets. Alternatively applications include delivery of DNA or mRNA sequences that code for therapeutically useful polypeptides. In this manner, therapy is provided for genetic diseases by supplying deficient or absent gene products (*i.e.*, for Duchenne's dystrophy, see Kunkel, et al., Brit. Med Bull. 45(3):630-643 (1989), and for cystic fibrosis, see Goodfellow, Nature 341:102-103 (1989)). Other uses for the compositions featured herein include introduction of antisense oligonucleotides in cells (see, Bennett, et al., Mol. Pharm. 41:1023-1033 (1992)).

Alternatively, the compositions containing a nucleic acid-based agent complexed with a lipid formulation can also be used for delivery of nucleic acids to cells *in vivo,* using methods which are known to those of skill in the art. With respect to delivery of DNA or mRNA sequences, Zhu, et al., Science 261:209-211 (1993), incorporated herein by reference, describes the intravenous delivery of cytomegalovirus (CMV)-chloramphenicol acetyltransferase (CAT) expression plasmid using DOTMA-DOPE complexes. Hyde, et al., Nature 362:250-256 (1993), incorporated herein by reference, describes the delivery of the cystic fibrosis transmembrane conductance regulator (CFTR) gene to epithelia of the airway and to alveoli in the lung of mice, using liposomes. Brigham, et al., Am. J. Med. Sci. 298:278-281 (1989), incorporated herein by reference, describes the *in vivo* transfection of lungs of mice with a functioning prokaryotic gene encoding the intracellular enzyme, chloramphenicol acetyltransferase (CAT). Thus, the compositions containing nucleic acid-based agents complexed with lipid formulations can be used in the treatment of infectious diseases.

For *in vivo* administration, the pharmaceutical compositions are typically administered parenterally, *i.e.*, intraarticularly, intravenously, intraperitoneally, subcutaneously, intramuscularly, or subdermally, such as by an implanted device. In particular embodiments, the pharmaceutical compositions are administered intravenously or intraperitoneally by a bolus injection. For one example, see Stadler, et al., U.S. Patent No. 5,286,634, which is incorporated herein by reference. Intracellular nucleic acid delivery has also been discussed in Straubringer, et al., METHODS IN ENZYMOLOGY, Academic Press, New York. 101:512-527 (1983); Mannino, et al., Biotechniques 6:682-690 (1988); Nicolau, et al., Crit. Rev. Ther. Drug Carrier Syst. 6:239-271 (1989), and Behr, Acc. Chem. Res. 26:274-278 (1993). Still other methods of administering lipid-based therapeutics are described in, for example, Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179; Lenk et al., U.S. Patent No. 4,522,803; and Fountain et al., U.S. Patent No. 4,588,578.

In other methods, the pharmaceutical preparations may be contacted with the target tissue by direct application of the preparation to the tissue. The application may be made by topical, "open" or "closed" procedures. By "topical," it is meant the direct application of the pharmaceutical preparation to a tissue exposed to the environment, such as the skin, oropharynx, external auditory canal, and the like. "Open" procedures are those procedures which include incising the skin of a patient and directly visualizing the underlying tissue to which the pharmaceutical preparations are applied. This is generally accomplished by a surgical procedure, such as a thoracotomy to access the lungs, abdominal laparotomy to access abdominal viscera, or other direct surgical approach to the target tissue. "Closed" procedures are invasive procedures in which the internal target tissues are not directly visualized, but accessed via inserting instruments through

small wounds in the skin. For example, the preparations may be administered to the peritoneum by needle lavage. Likewise, the pharmaceutical preparations may be administered to the meninges or spinal cord by infusion during a lumbar puncture followed by appropriate positioning of the patient as commonly practiced for spinal anesthesia or metrazamide imaging of the spinal cord. Alternatively, the preparations may be administered through endoscopic devices.

The lipid-nucleic acid compositions can also be administered in an aerosol inhaled into the lungs (*see*, Brigham, et al., Am. J. Sci. 298(4):278-281 (1989)) or by direct injection at the site of disease (Culver, Human Gene Therapy, MaryAnn Liebert, Inc., Publishers, New York. pp.70-71 (1994)).

The methods of using the compositions for enhanced uptake into immune cells can be practiced in a variety of hosts, including mammalian hosts, such as humans, non-human primates, dogs, cats, cattle, horses, sheep, and the like.

Dosages for lipid-therapeutic agent particles will depend on the ratio of therapeutic agent to lipid and the administrating physician's opinion based on age, weight, and condition of the patient.

In one embodiment, the invention provides a method of modulating the expression of a target polynucleotide or polypeptide. These methods generally include contacting a cell with a lipid particle that is associated with a nucleic acid capable of modulating the expression of a target polynucleotide or polypeptide. As used herein, the term "modulating" refers to altering the expression of a target polynucleotide or polypeptide. In different embodiments, modulating can mean increasing or enhancing, or it can mean decreasing or reducing. Methods of measuring the level of expression of a target polynucleotide or polypeptide are known and available in the arts and include, e.g., methods employing reverse transcription-polymerase chain reaction (RT-PCR) and immunohistochemical techniques. In particular embodiments, the level of expression of a target polynucleotide or polypeptide is increased or reduced by at least 10%, 20%, 30%, 40%, 50%, or greater than 50% as compared to an appropriate control value. For example, if increased expression of a polypeptide is desired, the nucleic acid may be an expression vector that includes a polynucleotide that encodes the desired polypeptide. On the other hand, if reduced expression of a polynucleotide or polypeptide is desired, then the nucleic acid may be, *e.g.,* an antisense oligonucleotide, dsRNA, or microRNA that comprises a polynucleotide sequence that specifically hybridizes to a polnucleotide that encodes the target polypeptide, thereby disrupting expression of the target polynucleotide or polypeptide. Alternatively, the nucleic acid may be a plasmid that expresses such an antisense oligonucletoide, dsRNA, or microRNA.

In one particular embodiment, the invention provides a method of modulating the expression of a polypeptide by a cell, comprising providing to a cell a lipid particle that consists of or consists essentially of a cationic lipid of formula A, a neutral lipid, a sterol, a PEG of PEG-modified lipid, *e.g.,* in a molar ratio of about 35-65% of cationic lipid of formula A, 3-12% of the neutral lipid, 15-45% of the sterol, and 0.5-10% of the PEG or PEG-modified lipid, wherein the lipid particle is associated with a nucleic acid capable of modulating the expression of the polypeptide. In particular embodiments, the molar lipid ratio is approximately 60/7.5/31/1.5 or 57.5/7.5/31.5/3.5 (mol% LIPID A/DSPC/Chol/PEG-DMG) or approximately 50/10/30/10, or 50/10/38.5/1.5 (mol% LIPID A/DSPC/Chol/PEG-CerC14 or PEG-CerC18). In another group of embodiments, the neutral lipid in these compositions is replaced with DPPC, POPC, DOPE or SM. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

In one embodiment, the invention provides a method of modulating the expression of a polypeptide by a cell, comprising providing to a cell a lipid particle that consists of or consists essentially of a cationic lipid of formula A, a neutral lipid, a sterol, a PEG of PEG-modified lipid, *e.g.*, in a molar ratio of about 10-50% of cationic lipid of formula A, 10-50% of the neutral lipid, 20-50% of the sterol, and 0.5-15% of the PEG or PEG-modified lipid, wherein the lipid particle is associated with a nucleic acid capable of modulating the expression of the polypeptide. In particular embodiments, the molar lipid ratio is approximately 30/30/30/10 or 30/30/38.5/1.5 (mol% LIPID A/DSPC/Chol/PEG-DMG or PEG-DSG). In another group of embodiments, the neutral lipid in these compositions is replaced with DPPC, POPC, DOPE or SM. In some embodiments, the PEG modified lipid is PEG-CerC18. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

In particular embodiments, the therapeutic agent is selected from a dsRNA, a microRNA, an antisense oligonucleotide, and a plasmid capable of expressing a dsRNA, a microRNA, or an antisense oligonucleotide, and wherein the dsRNA, microRNA, or antisense RNA comprises a polynucleotide that specifically binds to a polynucleotide that encodes the polypeptide, or a complement thereof, such that the expression of the polypeptide is reduced.

In other embodiments, the nucleic acid is a plasmid that encodes the polypeptide or a functional variant or

fragment thereof, such that expression of the polypeptide or the functional variant or fragment thereof is increased.

In related embodiments, the invention provides a method of treating a disease or disorder characterized by overexpression of a polypeptide in a subject, by for example, providing to the subject a pharmaceutical composition havine a nucleic acid-based agent complexed with a lipid-containing formulation, where the agent is selected from a dsRNA, a microRNA, an antisense oligonucleotide, and a plasmid capable of expressing a dsRNA, a microRNA, or an antisense oligonucleotide, and wherein the dsRNA, microRNA, or antisense RNA includes a polynucleotide that specifically binds to a polynucleotide that encodes the polypeptide, or a complement thereof.

In one embodiment, the pharmaceutical composition comprises a lipid particle that consists of or consists essentially of Lipid A, DSPC, Chol and PEG-DMG, PEG-C-DOMG or PEG-DMA, e.g., in a molar ratio of about 35-65% of cationic lipid of formula A, 3-12% of the neutral lipid, 15-45% of the sterol, and 0.5-10% of the PEG or PEG-modified lipid PEG-DMG, PEG-C-DOMG or PEG-DMA, wherein the lipid particle is associated with the therapeutic nucleic acid. In particular embodiments, the molar lipid ratio is approximately 60/7.5/31/1.5, or 57.5/7.5/31.5/3.5 (mol% LIPID A/DSPC/Chol/PEG-DMG) or approximately 50/10/30/10, or 50/10/38.5/1.5 (mol% LIPID A/DSPCVChol/PEG-CerC14 or PEG-CerC18. In another group of embodiments, the neutral lipid in these compositions is replaced with DPPC, POPC, DOPE or SM. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

In one embodiment, the pharmaceutical composition comprises a lipid particle that consists of or consists essentially of a cationic lipid of formula A, a neutral lipid, a sterol, a PEG of PEG-modified lipid, e.g., in a molar ratio of about 10-50% of cationic lipid of formula A, 10-50% of the neutral lipid, 20-50% of the sterol, and 0.5-15% of the PEG or PEG-modified lipid, wherein the lipid particle is associated with the therapeutic nucleic acid. In particular embodiments, the molar lipid ratio is approximately 30/30/30/10 or 30/30/38.5/1.5 (mol% LIPID A/DSPC/Chol/PEG-DMG or PEG-DSG). In another group of embodiments, the neutral lipid in these compositions is replaced with DPPC, POPC, DOPE or SM. In some embodiments, the PEG modified lipid is PEG-CerC18. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

In another related embodiment, the invention includes a method of treating a disease or disorder characterized by underexpression of a polypeptide in a subject, by, for example, providing to the subject a pharmaceutical composition as described herein, where the therapeutic agent is a plasmid that encodes the polypeptide or a functional variant or fragment thereof. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

The invention further provides a method of inducing an immune response in a subject, comprising providing to the subject a pharmaceutical composition described herein, where the nucleic acid-based agent is an immunostimulatory oligonucleotide. In certain embodiments, the immune response is a humoral or mucosal immune response consists of or consists essentially of Lipid A, DSPC, Chol and PEG-DMG, PEG-C-DOMG or PEG-DMA, e.g., in a molar ratio of about 35-65% of cationic lipid of formula A, 3-12% of the neutral lipid, 15-45% of the sterol, and 0.5-10% of the PEG or PEG-modified lipid PEG-DMG, PEG-C-DOMG or PEG-DMA, wherein the lipid particle is associated with the therapeutic nucleic acid. In particular embodiments, the molar lipid ratio is approximately 60/7.5/31/1.5 or 57.5/7.5/31.5/3.5, (mol% LIPID A/DSPC/Chol/PEG-DMG) or approximately 50/10/30/10, or 50/10/38.5/1.5 (mol% LIPID A/DSPC/Chol/PEG-CerC14 or PEG-CerC18. In another group of embodiments, the neutral lipid in these compositions is replaced with DPPC, POPC, DOPE or SM. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

The invention further provides a method of inducing an immune response in a subject, comprising providing to the subject a pharmaceutical composition described herein, where the nucleic acid-based agent is an immunostimulatory oligonucleotide. In certain embodiments, the immune response is a humoral or mucosal immune response that consists of or consists essentially of a cationic lipid of formula A, a neutral lipid, a sterol, a PEG or PEG-modified lipid, *e.g.*, in a molar ratio of about 10-50% of cationic lipid of formula A, 10-50% of the neutral lipid, 20-50% of the sterol, and 0.5-15%

of the PEG or PEG-modified lipid, wherein the lipid particle is associated with the therapeutic nucleic acid. In particular embodiments, the molar lipid ratio is approximately 30/30/30/10 or 30/30/38.5/1.5 (mol% LIPID A/DSPC/Chol/PEG-DMG or PEG-DSG). In another group of embodiments, the neutral lipid in these compositions is replaced with DPPC, POPC, DOPE or SM. In some embodiments, the PEG modified lipid is PEG-CerC18. In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter). In some embodiments, the polydispersity index (PDI) of the particles is less than about 0.5 (e.g., less than about 0.4, less than about 0.3, less than about 0.2, or less than about 0.1).

In some embodiments, pharmaceutical compositions containing a nucleic acid-based agent complexed to a liposome formulation can be administered in combination with a second nucleic acid-based agent (e.g., a second dsRNA) and/or one or more additional therapy. For example, for treatment of a cancer a composition featured herein can be administered with a chemotherapeutic agent or in combination with radiotherapy. Examplary chemotherapeutic agents include but are not limited to temozolomide, daunorubicin, daunomycin, dactinomycin, doxorubicin, epirubicin, idarubicin, esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5-azacytidine, hydroxyurea, deoxycoformycin, 4-hydroxyperoxycyclophosphor- amide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin and diethylstilbestrol (DES). See, generally, The Merck Manual of Diagnosis and Therapy, 15th Ed. 1987, pp. 1206-1228, Berkow et al., eds., Rahway, N.J. When used with the dsRNAs featured in the invention, such chemotherapeutic agents may be used individually (*e.g.*, 5-FU and oligonucleotide), sequentially (*e.g.*, 5-FU and oligonucleotide for a period of time followed by MTX and oligonucleotide), or *in* combination with one or more other such chemotherapeutic agents (*e.g.*, 5-FU, MTX and oligonucleotide, or 5-FU, radiotherapy and oligonucleotide).

For treatment of an inflammatory disease, a composition containing a nucleic acid-based agent and a lipid formulation can be administered in combination with an anti-inflammatory drug, such as a nonsteroidal anti-inflammatory drug or corticosteroid, or antiviral drug, such as ribivirin, vidarabine, acyclovir or ganciclovir. See, generally, The Merck Manual of Diagnosis and Therapy, 15th Ed., Berkow et al., eds., 1987, Rahway, NJ., pages 2499-2506 and 46-49, respectively). Other non-RNAi chemotherapeutic agents are also within the scope of this invention. Two or more combined compounds may be used together or sequentially.

In further embodiments, the pharmaceutical composition is provided to the subject in combination with a vaccine or antigen. Thus, the invention itself provides vaccines having a lipid particle complexed with an immunostimulatory oligonucleotide, and also associated with an antigen to which an immune response is desired. In particular embodiments, the antigen is a tumor antigen or is associated with an infective agent, such as, *e.g.,* a virus, bacteria, or parasiste.

A variety of tumor antigens, infections agent antigens, and antigens associated with other disease are well known in the art and examples of these are described in references cited herein. Examples of antigens suitable for use in the invention include, but are not limited to, polypeptide antigens and DNA antigens. Specific examples of antigens are Hepatitis A, Hepatitis B, small pox, polio, anthrax, influenza, typhus, tetanus, measles, rotavirus, diphtheria, pertussis, tuberculosis, and rubella antigens. In one embodiment, the antigen is a Hepatitis B recombinant antigen. In other aspects, the antigen is a Hepatitis A recombinant antigen. In another aspect, the antigen is a tumor antigen. Examples of such tumor-associated antigens are MUC-1, EBV antigen and antigens associated with Burkitt's lymphoma. In a further aspect, the antigen is a tyrosinase-related protein tumor antigen recombinant antigen. Those of skill in the art will know of other antigens suitable for use in the invention.

Tumor-associated antigens suitable for use in the subject invention include both mutated and non-mutated molecules that may be indicative of single tumor type, shared among several types of tumors, and/or exclusively expressed or overexpressed in tumor cells in comparison with normal cells. In addition to proteins and glycoproteins, tumor-specific patterns of expression of carbohydrates, gangliosides, glycolipids and mucins have also been documented. Exemplary tumor-associated antigens for use in the subject cancer vaccines include protein products of oncogenes, tumor suppressor genes and other genes with mutations or rearrangements unique to tumor cells, reactivated embryonic gene products, oncofetal antigens, tissue-specific (but not tumor-specific) differentiation antigens, growth factor receptors, cell surface carbohydrate residues, foreign viral proteins and a number of other self proteins.

Specific embodiments of tumor-associated antigens include, e.g., mutated antigens such as the protein products of the Ras p21 protooncogenes, tumor suppressor p53 and BCR-abl oncogenes, as well as CDK4, MUM1, Caspase 8, and Beta catenin; overexpressed antigens such as galectin 4, galectin 9, carbonic anhydrase, Aldolase A, PRAME, Her2/neu, ErbB-2 and KSA, oncofetal antigens such as alpha fetoprotein (AFP), human chorionic gonadotropin (hCG); self antigens such as carcinoembryonic antigen (CEA) and melanocyte differentiation antigens such as Mart 1/Melan A, gp100, gp75, Tyrosinase, TRP1 and TRP2; prostate associated antigens such as PSA, PAP, PSMA, PSM-P1 and

PSM-P2; reactivated embryonic gene products such as MAGE 1, MAGE 3, MAGE 4, GAGE 1, GAGE 2, BAGE, RAGE, and other cancer testis antigens such as NY-ESO1, SSX2 and SCP1; mucins such as Muc-1 and Muc-2; gangliosides such as GM2, GD2 and GD3, neutral glycolipids and glycoproteins such as Lewis (y) and globo-H; and glycoproteins such as Tn, Thompson-Freidenreich antigen (TF) and sTn. Also included as tumor-associated antigens herein are whole cell and tumor cell lysates as well as immunogenic portions thereof, as well as immunoglobulin idiotypes expressed on monoclonal proliferations of B lymphocytes for use against B cell lymphomas.

Pathogens include, but are not limited to, infectious agents, *e.g.*, viruses, that infect mammals, and more particularly humans. Examples of infectious virus include, but are not limited to: Retroviridae (*e.g.*, human immunodeficiency viruses, such as HIV-1 (also referred to as HTLV-III, LAV or HTLV-III/LAV, or HIV-III; and other isolates, such as HIV-LP; Picornaviridae (*e.g.*, polio viruses, hepatitis A virus; enteroviruses, human Coxsackie viruses, rhinoviruses, echoviruses); Calciviridae (*e.g.,* strains that cause gastroenteritis); Togaviridae (*e.g.*, equine encephalitis viruses, rubella viruses); Flaviridae (*e.g.*, dengue viruses, encephalitis viruses, yellow fever viruses); Coronoviridae (*e.g.*, coronaviruses); Rhabdoviradae (*e.g.*, vesicular stomatitis viruses, rabies viruses); Coronaviridae (*e.g.*, coronaviruses); Rhabdoviridae (*e.g.*, vesicular stomatitis viruses, rabies viruses); Filoviridae (*e.g.*, ebola viruses); Paramyxoviridae (*e.g.*, parainfluenza viruses, mumps virus, measles virus, respiratory syncytial virus); Orthomyxoviridae (*e.g.*, influenza viruses); Bungaviridae (*e.g.*, Hantaan viruses, bunga viruses, phleboviruses and Nairo viruses); Arena viridae (hemorrhagic fever viruses); Reoviridae (*e.g.*, reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae herpes simplex virus (HSV) 1 and 2, varicella zoster virus, cytomegalovirus (CMV), herpes virus; Poxviridae (variola viruses, vaccinia viruses, pox viruses); and Iridoviridae (*e.g.*, African swine fever virus); and unclassified viruses (*e.g.*, the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (*i.e.,* Hepatitis C); Norwalk and related viruses, and astroviruses).

Also, gram negative and gram positive bacteria serve as antigens in vertebrate animals. Such gram positive bacteria include, but are not limited to Pasteurella species, Staphylococci species, and Streptococcus species. Gram negative bacteria include, but are not limited to, Escherichia coli, Pseudomonas species, and Salmonella species. Specific examples of infectious bacteria include but are not limited to: Helicobacterpyloris, Borelia burgdorferi, Legionella pneumophilia, Mycobacteria sps (*e.g.*, M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcusfaecalis, Streptococcus bovis, Streptococcus (anaerobic sps.), Streptococcus pneumoniae, pathogenic Campylobacter sp., Enterococcus sp., Haemophilus infuenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia, and Actinomyces israelli.

Additional examples of pathogens include, but are not limited to, infectious fungi that infect mammals, and more particularly humans. Examples of infectious fingi include, but are not limited to: Cryptococcus neoformans, Histoplasma capsulatum, Coccidioides immitis, Blastomyces dermatitidis, Chlamydia trachomatis, Candida albicans. Examples of infectious parasites include Plasmodium such as Plasmodium falciparum, Plasmodium malariae, Plasmodium ovale, and Plasmodium vivax. Other infectious organisms (*i.e.*, protists) include Toxoplasma gondii.

*RNA Interference Nucleic Acids*

In particular embodiments, nucleic acid-based agents used in compositions for targeting immune cells are associated with RNA interference (RNAi) molecules. RNA interference methods using RNAi molecules may be used to disrupt the expression of a gene or polynucleotide of interest. In the last 5 years small interfering RNA (siRNA, or dsRNA) has essentially replaced antisense ODN and ribozymes as the next generation of targeted oligonucleotide drugs under development. DsRNAs are RNA duplexes typically having a region of complementarity less than 30 nucleotides in length, generally 19 to 24 nucleotides in length, *e.g.*, 19 to 21 nucleotides in length. In some embodiments, the dsRNA is from about 10 to about 15 basepairs, and in other embodiments the dsRNA is from about 25 to about 30 basepairs in length. In another embodiment, the dsRNA is at least 15 basepairs in length. In one embodiment, one or both of the sense and antisense strands of the dsRNA is from about 10 to 15 nucleotides in length, and in other embodiments, one of both of the strands is from about 25 to about 30 nucleotides in length. In one embodiment, one or both strands of the dsRNA is 19 to 24 nucleotides in length, e.g., 19 to 21 nucleotides in length. The dsRNA can associate with a cytoplasmic multiprotein complex known as RNAi-induced silencing complex (RISC). RISC loaded with dsRNA mediates the degradation of homologous mRNA transcripts, therefore dsRNA can be designed to knock down protein expression with high specificity. Unlike other antisense technologies, dsRNA function through a natural mechanism evolved to control gene expression through non-coding RNA. This is generally considered to be the reason why their activity is more potent *in*

*vitro* and *in vivo* than either antisense ODN or ribozymes. A variety of RNAi reagents, including dsRNAs targeting clinically relevant targets, are currently under pharmaceutical development, as described, *e.g.,* in de Fougerolles, A. et al., Nature Reviews 6:443-453 (2007).

While the first described RNAi molecules were RNA:RNA hybrids comprising both an RNA sense and an RNA antisense strand, it has now been demonstrated that DNA sense:RNA antisense hybrids, RNA sense:DNA antisense hybrids, and DNA:DNA hybrids are capable of mediating RNAi (Lamberton, J.S. and Christian, A.T., (2003) Molecular Biotechnology 24:111-119). Thus, the invention includes the use of RNAi molecules comprising any of these different types of double-stranded molecules. In addition, it is understood that RNAi molecules may be used and introduced to cells in a variety of forms. Accordingly, as used herein, RNAi molecules encompasses any and all molecules capable of inducing an RNAi response in cells, including, but not limited to, double-stranded polynucleotides comprising two separate strands, *i.e.* a sense strand and an antisense strand, *e.g.*, small interfering RNA (siRNA); polynucleotides comprising a hairpin loop of complementary sequences, which forms a double-stranded region, *e.g.*, shRNAi molecules, and expression vectors that express one or more polynucleotides capable of forming a double-stranded polynucleotide alone or in combination with another polynucleotide.

RNA interference (RNAi) may be used to specifically inhibit expression of target polynucleotides. Double-stranded RNA-mediated suppression of gene and nucleic acid expression may be accomplished according to the invention by introducing dsRNA, siRNA or shRNA into cells or organisms. SiRNA may be double-stranded RNA, or a hybrid molecule comprising both RNA and DNA, *e.g.,* one RNA strand and one DNA strand. It has been demonstrated that the direct introduction of dsRNAs to a cell can trigger RNAi in mammalian cells (Elshabir, S.M., et al. Nature 411:494-498 (2001)). Furthermore, suppression in mammalian cells occurred at the RNA level and was specific for the targeted genes, with a strong correlation between RNA and protein suppression (Caplen, N. et al., Proc. Natl. Acad. Sci. USA 98:9746-9747 (2001)). In addition, it was shown that a wide variety of cell lines, including HeLa S3, COS7, 293, NIH/3T3, A549, HT-29, CHO-KI and MCF-7 cells, are susceptible to some level of siRNA silencing (Brown, D. et al. TechNotes 9(1):1-7, available on the worldwide web ambion.com/techlib/tn/91/912.html (September 1, 2002)).

RNAi molecules targeting specific polynucleotides can be readily prepared according to procedures known in the art. Structural characteristics of effective siRNA molecules have been identified. Elshabir, S.M. et al. (2001) Nature 411:494-498 and Elshabir, S.M. et al. (2001), EMBO 20:6877-6888. Accordingly, one of skill in the art would understand that a wide variety of different siRNA molecules may be used to target a specific gene or transcript. In certain embodiments, siRNA molecules according to the invention are double-stranded and 16 - 30 or 18 - 25 nucleotides in length, including each integer in between. In certain embodiments, an siRNA is 19, 20, 21, 22, or 23 basepairs in length. In certain embodiments, dsRNAs have 0-7 nucleotide 3' overhangs or 0-4 nucleotide 5' overhangs. In one embodiment, an siRNA molecule has a two nucleotide 3' overhang. In one embodiment, an siRNA has sense and antisense strands 21 nucleotides in length, with two nucleotide 3' overhangs (*i.e.* there is a 19 nucleotide complementary region between the sense and antisense strands). In certain embodiments, the overhangs are UU or dTdT 3' overhangs.

In one embodiment, at least one end of a dsRNA (e.g., an siRNA) has a single-stranded nucleotide overhang of 1 to 4, generally 1 or 2 nucleotides. dsRNAs having at least one nucleotide overhang have unexpectedly superior inhibitory properties than their blunt-ended counterparts. Moreover, the presence of only one nucleotide overhang can strengthen the interference activity of the dsRNA without affecting its overall stability. dsRNA having only one overhang has proven particularly stable and effective *in vivo,* as well as in a variety of cells, cell culture mediums, blood, and serum. Generally, the single-stranded overhang is located at the 3'-terminal end of the antisense strand or, alternatively, at the 3'-tenmnal end of the sense strand. The dsRNA may also have a blunt end, generally located at the 5'-end of the antisense strand. Such dsRNAs have improved stability and inhibitory activity, thus allowing administration at low dosages, *i.e.,* less than 5 mg/kg body weight of the recipient per day. In one embodiment, the antisense strand of the dsRNA has a 1-10 nucleotide overhang at the 3' end and/or the 5' end. In one embodiment, the sense strand of the dsRNA has a 1-10 nucleotide overhang at the 3' end and/or the 5' end. In another embodiment, one or more of the nucleotides in the overhang is replaced with a nucleoside thiophosphate.

Generally, dsRNA molecules are completely complementary to one strand of a target DNA molecule, since even single base pair mismatches have been shown to reduce silencing. In other embodiments, dsRNAs may have a modified backbone composition, such as, for example, 2'-deoxy- or 2'-O-methyl modifications. However, in certain embodiments, the entire strand of the dsRNA is not made with either 2' deoxy or 2'-O-modified bases.

In another embodiment, the invention provides a cell including a vector for inhibiting the expression of a gene in a cell. The vector includes a regulatory sequence operably linked to a nucleotide sequence that encodes at least one strand of a dsRNA that targets a gene in an immune cell.

In one embodiment, dsRNA target sites are selected by scanning the target mRNA transcript sequence for the occurrence of AA dinucleotide sequences. Each AA dinucleotide sequence in combination with the 3' adjacent approximately 19 nucleotides are potential dsRNA target sites. In one embodiment, dsRNA target sites are preferentially not located within the 5' and 3' untranslated regions (UTRs) or regions near the start codon (within approximately 75 bases), since proteins that bind regulatory regions may interfere with the binding of the siRNP endonuclease complex (Elshabir,

S. et al. Nature 411:494-498 (2001); Elshabir, S. et al. EMBO J. 20:6877-6888 (2001)). In addition, potential target sites may be compared to an appropriate genome database, such as BLASTN 2.0.5, available on the NCBI server at www.ncbi.nlm, and potential target sequences with significant homology to other coding sequences eliminated.

In particular embodiments, short hairpin RNAs constitute the nucleic acid component of a nucleic acid-lipid particle. Short Hairpin RNA (shRNA) is a form of hairpin RNA capable of sequence-specifically reducing expression of a target gene. Short hairpin RNAs may offer an advantage over dsRNAs in suppressing gene expression, as they are generally more stable and less susceptible to degradation in the cellular environment. It has been established that such short hairpin RNA-mediated gene silencing works in a variety of normal and cancer cell lines, and in mammalian cells, including mouse and human cells. Paddison, P. et al., Genes Dev. 16(8):948-58 (2002). Furthermore, transgenic cell lines bearing chromosomal genes that code for engineered shRNAs have been generated. These cells are able to constitutively synthesize shRNAs, thereby facilitating long-lasting or constitutive gene silencing that may be passed on to progeny cells. Paddison, P. et al., Proc. Natl. Acad. Sci. USA 99(3):1443-1448 (2002).

ShRNAs contain a stem loop structure. In certain embodiments, they may contain variable stem lengths, typically from 19 to 29 nucleotides in length, or any number in between. In certain embodiments, hairpins contain 19 to 21 nucleotide stems, while in other embodiments, hairpins contain 27 to 29 nucleotide stems. In certain embodiments, loop size is between 4 to 23 nucleotides in length, although the loop size may be larger than 23 nucleotides without significantly affecting silencing activity. ShRNA molecules may contain mismatches, for example G-U mismatches between the two strands of the shRNA stem without decreasing potency. In fact, in certain embodiments, shRNAs are designed to include one or several G-U pairings in the hairpin stem to stabilize hairpins during propagation in bacteria, for example. However, complementarity between the portion of the stem that binds to the target mRNA (antisense strand) and the mRNA is typically required, and even a single base pair mismatch is this region may abolish silencing. 5' and 3' overhangs are not required, since they do not appear to be critical for shRNA function, although they may be present (Paddison et al. (2002) Genes & Dev. 16(8):948-58).

*MicroRNAs*

Micro RNAs (miRNAs) are a highly conserved class of small RNA molecules that are transcribed from DNA in the genomes of plants and animals, but are not translated into protein. Processed miRNAs are single stranded ~17-25 nucleotide (nt) RNA molecules that become incorporated into the RNA-induced silencing complex (RISC) and have been identified as key regulators of development, cell proliferation, apoptosis and differentiation. They are believed to play a role in regulation of gene expression by binding to the 3 '-untranslated region of specific mRNAs.RISC mediates down-regulation of gene expression through translational inhibition, transcript cleavage, or both. RISC is also implicated in transcriptional silencing in the nucleus of a wide range of eukaryotes.

The number of miRNA sequences identified to date is large and growing, illustrative examples of which can be found, for example, in: "miRBase: microRNA sequences, targets and gene nomenclature" Griffiths-Jones S, Grocock RJ, van Dongen S, Bateman A, Enright AJ. NAR, 2006, 34, Database Issue, D140-D144; "The microRNA Registry" Griffiths-Jones S. NAR, 2004, 32, Database Issue, D109-D111; and also on the worldwide web at microrna.dot.sanger.dot.ac-dot.uk/sequences/.

*Antisense Oligonucleotides*

In one embodiment, a nucleic acid is an antisense oligonucleotide directed to a target polynucleotide. The term "antisense oligonucleotide" or simply "antisense" is meant to include oligonucleotides that are complementary to a targeted polynucleotide sequence. Antisense oligonucleotides are single strands of DNA or RNA that are complementary to a chosen sequence. In the case of antisense RNA, they prevent translation of complementary RNA strands by binding to it. Antisense DNA can be used to target a specific, complementary (coding or non-coding) RNA. If binding takes places this DNA/RNA hybrid can be degraded by the enzyme RNase H. In particular embodiment, antisense oligonucleotides contain from about 10 to about 50 nucleotides, *e.g.*, about 15 to about 30 nucleotides. The term also encompasses antisense oligonucleotides that may not be exactly complementary to the desired target gene. Thus, the invention can be utilized in instances where non-target specific-activities are found with antisense, or where an antisense sequence containing one or more mismatches with the target sequence is typical for a particular use.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal $GABA_A$ receptor and human EGF (Jaskulski et al., Science. 1988 Jun 10;240(4858):1544-6; Vasanthakumar

and Ahmed, Cancer Commun. 1989;1(4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Furthermore, antisense constructs have also been described that inhibit and can be used to treat a variety of abnormal cellular proliferations, e.g. cancer (U. S. Patent 5,747,470; U. S. Patent 5,591,317 and U. S. Patent 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, $T_m$, binding energy, and relative stability. Antisense oligonucleotides may be selected based upon their relative inability to form dimers, hairpins, or other secondary structures that would reduce or prohibit specific binding to the target mRNA in a host cell. In some embodiments, the target regions of the mRNA are selected to include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al., Nucleic Acids Res. 1997,25(17):3389-402).

*Ribozymes*

According to another embodiment, nucleic acid-lipid particles are associated with ribozymes. Ribozymes are RNA-protein complexes having specific catalytic domains that possess endonuclease activity (Kim and Cech, Proc Nat1 Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

At least six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, a hepatitis $\delta$ virus, group I intron or RNaseP RNA (in association with an RNA guide sequence) or Neurospora VS RNA motif, for example. Specific examples of hammerhead motifs are described by Rossi et al. Nucleic Acids Res. 1992 Sep 11;20(17):4559-65. Examples of hairpin motifs are described by Hampel *et al.* (Eur. Pat. Appl. Publ. No. EP 0360257), Hampel and Tritz, Biochemistry 1989 Jun 13;28(12):4929-33; Hampel et al., Nucleic Acids Res. 1990 Jan 25;18(2):299-304 and U. S. Patent 5,631,359. An example of the hepatitis $\delta$ virus motif is described by Perrotta and Been, Biochemistry. 1992 Dec 1;31(47):11843-52; an example of the RNaseP motif is described by Guerrier-Takada et al., Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci U S A. 1991 Oct 1;88(19):8826-30; Collins and Olive, Biochemistry. 1993 Mar 23;32(11):2795-9); and an example of the Group I intron is described in U. S. Patent 4,987,071. Important characteristics of enzymatic nucleic acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus the ribozyme constructs need not be limited to specific motifs mentioned herein.

Methods of producing a ribozyme targeted to any polynucleotide sequence are known in the art. Ribozymes may be designed as described in Int. Pat. Appl. Publ. No. WO 93/23569 and Int. Pat. Appl. Publ. No. WO 94/02595, each specifically incorporated herein by reference, and synthesized to be tested *in vitro* and *in vivo,* as described therein.

Ribozyme activity can be optimized by altering the length of the ribozyme binding arms or chemically synthesizing ribozymes with modifications that prevent their degradation by serum ribonucleases (see *e.g.,* Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298A; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Additional specific nucleic acid sequences of oligonucleotides (ODNs) suitable for use in the compositions and

methods featured herein are described in U.S. Patent Appln. 60/379,343, U.S. patent application Ser. No. 09/649,527, Int. Publ. WO 02/069369, Int. Publ. No. WO 01/15726, U.S. Pat. No. 6,406,705, and Raney et al., Journal of Pharmacology and Experimental Therapeutics, 298:1185-1192 (2001). In certain embodiments, an ODN has a phosphodiester ("PO") backbone or a phosphorothioate ("PS") backbone, and/or at least one methylated cytosine residue in a CpG motif.

*Nucleic Acid Modifications*

In the 1990's DNA-based antisense oligodeoxynucleotides (ODN) and ribozymes (RNA) represented an exciting new paradigm for drug design and development, but their application *in vivo* was prevented by endo- and exo- nuclease activity as well as a lack of successful intracellular delivery. The degradation issue was effectively overcome following extensive research into chemical modifications that prevented the oligonucleotide (oligo) drugs from being recognized by nuclease enzymes but did not inhibit their mechanism of action. This research was so successful that antisense ODN drugs in development today remain intact *in vivo* for days compared to minutes for unmodified molecules (Kurreck, J. 2003. Antisense technologies. Improvement through novel chemical modifications. Eur J Biochem 270:1628-44). However, intracellular delivery and mechanism of action issues have so far limited antisense ODN and ribozymes from becoming clinical products.

RNA duplexes are inherently more stable to nucleases than single stranded DNA or RNA, and unlike antisense ODN, unmodified dsRNA show good activity once they access the cytoplasm. Even so, the chemical modifications developed to stabilize antisense ODN and ribozymes have also been systematically applied to dsRNA to determine how much chemical modification can be tolerated and if pharmacokinetic and pharmacodynamic activity can be enhanced. RNA interference by dsRNA duplexes requires an antisense and sense strand, which have different functions. Both are necessary to enable the dsRNA to enter RISC, but once loaded the two strands separate and the sense strand is degraded whereas the antisense strand remains to guide RISC to the target mRNA. Entry into RISC is a process that is structurally less stringent than the recognition and cleavage of the target mRNA. Consequently, many different chemical modifications of the sense strand are possible, but only limited changes are tolerated by the antisense strand (Zhang *et al*., 2006).

As is known in the art, a nucleoside is a base-sugar combination. Nucleotides are nucleosides that further include a phosphate group covalently linked to the sugar portion of the nucleoside. For those nucleosides that include a pentofuranosyl sugar, the phosphate group can be linked either to the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure. Within the oligonucleotide structure, the phosphate groups are commonly referred to as forming the internucleoside backbone of the oligonucleotide. The normal linkage or backbone of RNA and DNA is a 3' to 5' phosphodiester linkage.

The nucleic acid that is used in a lipid-nucleic acid particle according to this invention includes any form of nucleic acid that is known. Thus, the nucleic acid may be a modified nucleic acid of the type used previously to enhance nuclease resistance and serum stability. Surprisingly, however, acceptable therapeutic products can also be prepared by formulating lipid-nucleic acid particles from nucleic acids that have no modification to the phosphodiester linkages of natural nucleic acid polymers. Thus, in some embodiments, a nucleic acid-based agent includes unmodified phosphodiester linkages (*i.e*., nucleic acids in which all of the linkages are phosphodiester linkages).

*Backbone Modifications*

Antisense, dsRNA and other oligonucleotides useful *in* this invention include, but are not limited to, oligonucleotides containing modified backbones or non-natural internucleoside linkages. Oligonucleotides having modified backbones include those that retain a phosphorus atom in the backbone and those that do not have a phosphorus atom in the backbone. Modified oligonucleotides that do not have a phosphorus atom in their internucleoside backbone can also be considered to be oligonucleosides. Modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, phosphoroselenate, methylphosphonate, or O-alkyl phosphotriester linkages, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'.

Various salts, mixed salts and free acid forms are also included. Representative United States patents that teach the preparation of the above linkages include, but are not limited to, U.S. Patent Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253;

5,571,799; 5,587,361; and 5,625,050.

In certain embodiments, modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include, *e.g.*, those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts. Representative United States patents that describe the above oligonucleosides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

The phosphorothioate backbone modification, where a non-bridging oxygen in the phosphodiester bond is replaced by sulfur, is one of the earliest and most common means deployed to stabilize nucleic acid drugs against nuclease degradation. In general, it appears that PS modifications can be made extensively to both dsRNA strands without much impact on activity (Kurreck, Eur. J. Biochem. 270:1628-44, 2003). However, PS oligos are known to avidly associate non-specifically with proteins resulting in toxicity, especially upon i.v. administration. Therefore, the PS modification is usually restricted to one or two bases at the 3' and 5' ends. The boranophosphate linker (Table 3, #2) is a recent modification that is apparently more stable than PS, enhances dsRNA activity and has low toxicity (Hall et al., Nucleic Acids Res. 32:5991-6000, 2004).

Other useful nucleic acids derivatives include those nucleic acids molecules in which the bridging oxygen atoms (those forming the phosphoester linkages) have been replaced with -S-, -NH-, $-CH_2-$ and the like. In certain embodiments, the alterations to the antisense, dsRNA, or other nucleic acids used will not completely affect the negative charges associated with the nucleic acids. Thus, the invention contemplates the use of antisense, dsRNA, and other nucleic acids in which a portion of the linkages are replaced with, for example, the neutral methyl phosphonate or phosphoramidate linkages. When neutral linkages are used, in certain embodiments, less than 80% of the nucleic acid linkages are so substituted, or less than 50% of the linkages are so substituted.

*Base Modifications*

Base modifications are less common than those to the backbone and sugar. The modifications shown in 0.3-6 all appear to stabilize dsRNA against nucleases and have little effect on activity (Zhang et al., Curr. Top. Med. Chem. 6:893-900, 2006).

Accordingly, oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C or m5c), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine.

Certain nucleobases are particularly useful for increasing the binding affinity of oligomeric compounds. These nucleobases include, *e.g.*, 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2° C. (Sanghvi, Y. S., Crooke, S. T. and Lebleu, B., eds., Antisense Research and Applications 1993, CRC Press, Boca Raton, pages 276-278). These may be combined, in particular embodiments, with 2'-O-methoxyethyl sugar modifications. United States patents that teach the preparation of certain of these modified nucleobases as well as other modified nucleobases include, but are not limited to, the above noted U.S. Pat. No. 3,687,808, as well as U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941.

*Sugar Modifications*

Most modifications on the sugar group occur at the 2'-OH of the RNA sugar ring, which provides a convenient chemically reactive site (Manoharan, Curr. Opin. Chem. Biol. 8:570-9, 2004; Zhang et al., Curr. Top. Med. Chem.

6:893-900,2006). The 2'-F and 2'-OME (0.7 and 8) are common and both increase stability, the 2'-OME modification does not reduce activity as long as it is restricted to less than 4 nucleotides per strand (Holen et al., Nucleic Acids Res. 31:2401-7, 2003). The 2'-O-MOE (0.9) is most effective in dsRNA when modified bases are restricted to the middle region of the molecule (Prakash et al., J. Med. Chem 48:4247-53, 2005). Other modifications found to stabilize dsRNA without loss of activity are shown in 0.10-14.

Modified oligonucleotides may also contain one or more substituted sugar moieties. For example, the invention includes oligonucleotides that comprise one of the following at the 2' position: OH; F; O-, S-, or N-alkyl, O-alkyl-O-alkyl, O-, S-, or N-alkenyl, or O-, S- or N-alkynyl, wherein the alkyl, alkenyl and alkynyl may be substituted or unsubstituted $C_1$ to $C_{10}$ alkyl or $C_2$ to $C_{10}$ alkenyl and alkynyl. Typical embodiments include, *e.g.*, $O[(CH_2)_nO]_mCH_3$, $O(CH_2)_nOCH_3$, $O(CH_2)_2ON(CH_3)_2$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other oligonucleotides include one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. One modification includes 2'-methoxyethoxy (2'-O-$CH_2CH_2OCH_3$, also known as 2'-O-(2-methoxyethyl) or 2'-MOE) (Martin et al., Helv. Chim. Acta 1995, 78, 486-504), *i.e.,* an alkoxyalkoxy group. Other modifications include 2'-dimethylaminooxyethoxy, *i.e.,* a $O(CH_2)_2ON(CH_3)_2$ group, also known as 2'-DMAOE, and 2'-dimethylaminoethoxyethoxy (2'-DMAEOE).

Additional modifications include 2'-methoxy (2'-O-$CH_3$), 2'-aminopropoxy (2'-$OCH_2CH_2CH_2NH_2$) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugars structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

In other oligonucleotide mimetics, both the sugar and the internucleoside linkage, *i.e.,* the backbone, of the nucleotide units are replaced with novel groups, although the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos. 5,539,082; 5,714,331; and 5,719,262. Further teaching of PNA compounds can be found in Nielsen et al. (Science, 1991, 254, 1497-1500).

In some embodiments, an oligonucleotide includes a phosphorothioate backbone and an oligonucleoside includes a heteroatom backbone, such as --$CH_2$-NH--O--$CH_2$--, --$CH_2$--$N(CH_3)$ --O--$CH_2$- (referred to as a methylene (methylimino) or MMI backbone) --$CH_2$--O--$N(CH_3)$ --$CH_2$--, --$CH_2$--$N(CH_3)$--$N(CH_3)$--$CH_2$-- and --O--$N(CH_3)$--$CH_2$--$CH_2$--(where the native phosphodiester backbone is represented as --O--P--O--$CH_2$--) of the above referenced U.S. Pat. No. 5,489,677, and an amide backbone of the above referenced U.S. Pat. No. 5,602,240. In other embodiments, an oligonucleotide includes a morpholino backbone structure of the above-referenced U.S. Pat. No. 5,034,506.

*Chimeric Oligonucleotides*

It is not necessary for all positions in a given compound to be uniformly modified, and in fact more than one of the aforementioned modifications may be incorporated in a single compound or even at a single nucleoside within an oligonucleotide. In some embodiments, an oligonucleotide is a chimeric oligonucleotide. A "chimeric oligonucleotide" or "chimera," in the context of this invention, is an oligonucleotide that contains two or more chemically distinct regions, each made up of at least one nucleotide. These oligonucleotides typically contain at least one region of modified nucleotides that confers one or more beneficial properties (such as, *e.g.*, increased nuclease resistance, increased uptake into cells, increased binding affinity for the RNA target) and a region that is a substrate for RNase H cleavage.

In one embodiment, a chimeric oligonucleotide comprises at least one region modified to increase target binding affinity. Affinity of an oligonucleotide for its target is routinely determined by measuring the Tm of an oligonucleotide/target pair, which is the temperature at which the oligonucleotide and target dissociate; dissociation is detected spectrophotometrically. The higher the Tm, the greater the affinity of the oligonucleotide for the target. In some embodiments, the region of the oligonucleotide modified to increase target mRNA binding affinity includes at least one nucleotide modified at the 2' position of the sugar, such as a 2'-O-alkyl, 2'-O-alkyl-O-alkyl or 2'-fluoro-modified nucleotide. Such modifications

are routinely incorporated into oligonucleotides and these oligonucleotides have been shown to have a higher Tm (*i.e.,* higher target binding affinity) than 2'-deoxyoligonucleotides against a given target. The effect of such increased affinity is to greatly enhance oligonucleotide inhibition of target gene expression.

In another embodiment, a chimeric oligonucletoide comprises a region that acts as a substrate for RNAse H. Of course, it is understood that oligonucleotides may include any combination of the various modifications described herein

Another suitable modification of an oligonucleotide involves chemically linking to the oligonucleotide one or more moieties or conjugates which enhance the activity, cellular distribution or cellular uptake of the oligonucleotide. Such conjugates and methods of preparing the same are known in the art.

Those skilled in the art will realize that for *in vivo* utility, such as therapeutic efficacy, a reasonable rule of thumb is that if a thioated version of the sequence works in the free form, that encapsulated particles of the same sequence, of any chemistry, will also be efficacious. Encapsulated particles may also have a broader range of *in vivo* utilities, showing efficacy in conditions and models not known to be otherwise responsive to antisense therapy. Those skilled in the art know that applying this invention they may find old models which now respond to antisense therapy. Further, they may revisit discarded antisense sequences or chemistries and find efficacy by employing the invention.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of the routineer. It is also well known to use similar techniques to prepare other oligonucleotides such as the phosphorothioates and alkylated derivatives.

*Lipid Particles*

The agents and/or amino lipids can be formulated in lipid particles. Lipid particles include, but are not limited to, liposomes. As used herein, a liposome is a structure having lipid-containing membranes enclosing an aqueous interior. Liposomes may have one or more lipid membranes. The invention contemplates both single-layered liposomes, which are referred to as unilamellar, and multi-layered liposomes, which are referred to as multilamellar. When complexed with nucleic acids, lipid particles may also be lipoplexes, which are composed of cationic lipid bilayers sandwiched between DNA layers, as described, *e.g.,* in Felgner, Scientific American.

Lipid particles may further include one or more additional lipids and/or other components such as cholesterol. Other lipids may be included in the liposome compositions for a variety of purposes, such as to prevent lipid oxidation or to attach ligands onto the liposome surface. Any of a number of lipids may be present, including amphipathic, neutral, cationic, and anionic lipids. Such lipids can be used alone or in combination. Specific examples of additional lipid components that may be present are described below.

Additional components that may be present in a lipid particle include bilayer stabilizing components such as polyamide oligomers (*see, e.g.,* U.S. Patent No. 6,320,017), peptides, proteins, detergents, lipid-derivatives, such as PEG coupled to phosphatidylethanolamine and PEG conjugated to ceramides (*see,* U.S. Patent No. 5,885,613).

A lipid particle can include one or more of a second amino lipid or cationic lipid, a neutral lipid, a sterol, and a lipid selected to reduce aggregation of lipid particles during formation, which may result from steric stabilization of particles which prevents charge-induced aggregation during formation.

Examples of lipids suitable for conjugation to nucleic acid agents are polyethylene glycol (PEG)-modified lipids, monosialoganglioside Gml, and polyamide oligomers ("PAO") such as (described in U.S. Pat. No. 6,320,017). Other compounds with uncharged, hydrophilic, steric-barrier moieties, which prevent aggregation during formulation, like PEG, Gml or ATTA, can also be coupled to lipids. ATTA-lipids are described, *e.g.*, in U.S. Patent No. 6,320,017, and PEG-lipid conjugates are described, *e.g.*, in U.S. Patent Nos. 5,820,873, 5,534,499 and 5,885,613. Typically, the concentration of the lipid component selected to reduce aggregation is about 1 to 15% (by mole percent of lipids).

Specific examples of PEG-modified lipids (or lipid-polyoxyethylene conjugates) that are useful in the invention can have a variety of "anchoring" lipid portions to secure the PEG portion to the surface of the lipid vesicle. Examples of suitable PEG-modified lipids include PEG-modified phosphatidylethanolamine and phosphatidic acid, PEG-ceramide conjugates (*e.g.*, PEG-CerC14 or PEG-CerC20) which are described in co-pending U.S.S.N. 08/486,214, incorporated herein by reference, PEG-modified dialkylamines and PEG-modified 1,2-diacyloxypropan-3-amines. PEG-modified diacylglycerols and dialkylglycerols are typical.

In embodiments where a sterically-large moiety such as PEG or ATTA are conjugated to a lipid anchor, the selection of the lipid anchor depends on what type of association the conjugate is to have with the lipid particle. It is well known that mePEG (mw2000)-diastearoylphosphatidylethanolamine (PEG-DSPE) will remain associated with a liposome until the particle is cleared from the circulation, possibly a matter of days. Other conjugates, such as PEG-CerC20 have similar staying capacity. PEG-CerC14, however, rapidly exchanges out of the formulation upon exposure to serum, with a $T_{1/2}$ less than 60 minutes in some assays. As illustrated in U.S. Pat. Application S.N. 08/486,214, at least three

characteristics influence the rate of exchange: length of acyl chain, saturation of acyl chain, and size of the steric-barrier head group. Compounds having suitable variations of these features may be useful for the invention. For some therapeutic applications it may be preferable for the PEG-modified lipid to be rapidly lost from the nucleic acid-lipid particle *in vivo* and hence the PEG-modified lipid will possess relatively short lipid anchors. In other therapeutic applications it may be preferable for the nucleic acid-lipid particle to exhibit a longer plasma circulation lifetime and hence the PEG-modified lipid will possess relatively longer lipid anchors. Exemplary lipid anchors include those having lengths of from about $C_{14}$ to about $C_{22}$, such as from about $C_{14}$ to about $C_{16}$. In some embodiments, a PEG moiety, for example an mPEG-$NH_2$, has a size of about 1000, 2000, 5000, 10,000, 15,000 or 20,000 daltons.

It should be noted that aggregation preventing compounds do not necessarily require lipid conjugation to function properly. Free PEG or free ATTA in solution may be sufficient to prevent aggregation. If the particles are stable after formulation, the PEG or ATTA can be dialyzed away before administration to a subject.

Neutral lipids, when present in the lipid particle, can be any of a number of lipid species which exist either in an uncharged or neutral zwitterionic form at physiological pH. Such lipids include, for example diacylphosphatidylcholine, diacylphosphatidylethanolamine, ceramide, sphingomyelin, dihydrosphingomyelin, cephalin, and cerebrosides. The selection of neutral lipids for use in the particles described herein is generally guided by consideration of, *e.g.,* liposome size and stability of the liposomes in the bloodstream. Typically, the neutral lipid component is a lipid having two acyl groups (*i.e.*, diacylphosphatidylcholine and diacylphosphatidylethanolamine). Lipids having a variety of acyl chain groups of varying chain length and degree of saturation are available or may be isolated or synthesized by well-known techniques. In one group of embodiments, lipids containing saturated fatty acids with carbon chain lengths in the range of $C_{14}$ to $C_{22}$ are used. In another group of embodiments, lipids with mono or diunsaturated fatty acids with carbon chain lengths in the range of $C_{14}$ to $C_{22}$ are used. Additionally, lipids having mixtures of saturated and unsaturated fatty acid chains can be used. Typically, the neutral lipids used in the invention are DOPE, DSPC, POPC, or any related phosphatidylcholine. The neutral lipids useful in the invention may also be composed of sphingomyelin, dihydrosphingomyeline, or phospholipids with other head groups, such as serine and inositol.

The sterol component of the lipid mixture, when present, can be any of those sterols conventionally used in the field of liposome, lipid vesicle or lipid particle preparation. A typical sterol is cholesterol.

Other cationic lipids, which carry a net positive charge at about physiological pH, in addition to those specifically described above, may also be included in the lipid particles. Such cationic lipids include, but are not limited to, N,N-dioleyl-N,N-dimethylammonium chloride ("DODAC"); N-(2,3-dioleyloxy)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.Cl"); 3β-(N-(N',N'-dimethylammoethane)-carbamoyl)cholesterol ("C-Chol"), N-(1-(2,3-dioleyloxy)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N, N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic lipids can be used, such as, *e.g.,* LIPOFECTIN (including DOTMA and DOPE, available from GIBCO/BRL), and LIPOFECTAMINE (comprising DOSPA and DOPE, available from GIBCO/BRL). In particular embodiments, a cationic lipid is an amino lipid.

Anionic lipids suitable for use in the lipid particles include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

In numerous embodiments, amphipathic lipids are included in the lipid particles. "Amphipathic lipids" refer to any suitable material, wherein the hydrophobic portion of the lipid material orients into a hydrophobic phase, while the hydrophilic portion orients toward the aqueous phase. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine, dioleoylphosphatidylcholine, distearoylphosphatidylcholine, or dilinoleylphosphatidylcholine. Other phosphorus-lacking compounds, such as sphingolipids, glycosphingolipid families, diacylglycerols, and β-acyloxyacids, can also be used. Additionally, such amphipathic lipids can be readily mixed with other lipids, such as triglycerides and sterols.

Also suitable for inclusion in the lipid particles are programmable fusion lipids. Such lipid particles have little tendency to fuse with cell membranes and deliver their payload until a given signal event occurs. This allows the lipid particle to distribute more evenly after injection into an organism or disease site before it starts fusing with cells. The signal event can be, for example, a change in pH, temperature, ionic environment, or time. In the latter case, a fusion delaying or "cloaking" component, such as an ATTA-lipid conjugate or a PEG-lipid conjugate, can simply exchange out of the lipid particle membrane over time. Exemplary lipid anchors include those having lengths of from about $C_{14}$ to about $C_{22}$, such as from about $C_{14}$ to about $C_{16}$. In some embodiments, a PEG moiety, for example an mPEG-$NH_2$, has

a size of about 1000, 2000, 5000, 10,000, 15,000 or 20,000 daltons.

In one embodiment, the average particle size of the nucleic acid-based agent complexed with the lipid formulation described herein is at least about 100 nm in diameter (e.g., at least about 110 nm in diameter, at least about 120 nm in diameter, at least about 150 nm in diameter, at least about 200 nm in diameter, at least about 250 nm in diameter, or at least about 300 nm in diameter).

In some embodiments, the polydispersity index (PDI) of the particles is less than about 0,5 (e.g., less than about 0.4, less than about 0.3, less than about 0.2, or less than about 0.1).

By the time the lipid particle is suitably distributed in the body, it has lost sufficient cloaking agent so as to be fusogenic. With other signal events, it is desirable to choose a signal that is associated with the disease site or target cell, such as increased temperature at a site of inflammation.

A lipid particle conjugated to a nucleic acid agent can also include a targeting moiety, *e.g.,* a targeting moiety that is specific to a cell type or tissue. Targeting of lipid particles using a variety of targeting moieties, such as ligands, cell surface receptors, glycoproteins, vitamins (*e.g.*, riboflavin), folate and monoclonal antibodies (*e.g.*, antibodies to $\beta_7$ integrin ($\beta_7$I)), has been previously described (see, *e.g.,* U.S.

Patent Nos. 4,957,773 and 4,603,044). The targeting moieties can include the entire protein or fragments thereof. Targeting mechanisms generally require that the targeting agents be positioned on the surface of the lipid particle in such a manner that the targeting moiety is available for interaction with the target, for example, a cell surface receptor. A variety of different targeting agents and methods are known and available in the art, including those described, *e.g.,* in Sapra, P. and Allen, TM, Prog. Lipid Res. 42(5):439-62 (2003); and Abra, RM et al., J. Liposome Res. 12:1-3, (2002).

The use of Lipid particles, *i.e.,* liposomes, with a surface coating of hydrophilic polymer chains, such as polyethylene glycol (PEG) chains, for targeting has been proposed (Allen, et al., Biochimica et Biophysica Acta 1237: 99-108 (1995); DeFrees, et al., Journal of the American Chemistry Society 118: 6101-6104 (1996); Blume, et al., Biochimica et Biophysica Acta 1149: 180-184 (1993); Klibanov, et al., Journal of Liposome Research 2: 321-334 (1992); U.S. Patent No. 5,013556; Zalipsky, Bioconjugate Chemistry 4: 296-299 (1993); Zalipsky, FEBS Letters 353: 71-74 (1994); Zalipsky, in Stealth Liposomes Chapter 9 (Lasic and Martin, Eds) CRC Press, Boca Raton F1 (1995). In one approach, a ligand, such as an antibody, for targeting the lipid particle is linked to the polar head group of lipids forming the lipid particle. In another approach, the targeting ligand is attached to the distal ends of the PEG chains forming the hydrophilic polymer coating (Klibanov, et al., Journal of Liposome Research 2: 321-334 (1992); Kirpotin et al., FEBS Letters 388: 115-118 (1996)).

Standard methods for coupling the target agents can be used. For example, phosphatidylethanolamine, which can be activated for attachment of target agents, or derivatized lipophilic compounds, such as lipid-derivatized bleomycin, can be used. Antibody-targeted liposomes can be constructed using, for instance, liposomes that incorporate protein A (*see,* Renneisen, et al., J. Bio. Chem., 265:16337-16342 (1990) and Leonetti, et al., Proc. Natl Acad. Sci. (USA), 87:2448-2451 (1990). Other examples of antibody conjugation are disclosed in U.S. Patent No. 6,027,726, the teachings of which are incorporated herein by reference. Examples of targeting moieties can also include other proteins, specific to cellular components, including antigens associated with neoplasms or tumors. Proteins used as targeting moieties can be attached to the liposomes via covalent bonds (*see,* Heath, Covalent Attachment of Proteins to Liposomes, 149 Methods in Enzymology 111-119 (Academic Press, Inc. 1987)). Other targeting methods include the biotin-avidin system.

## *Definitions*

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A," "T" and "U" each generally stand for a nucleotide that contains guanine, cytosine, adenine, thymidine and uracil as a base, respectively. However, it will be understood that the term "ribonucleotide" or "nucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences of dsRNA featured in the invention by a nucleotide containing, for example, inosine. In another example, adenine and cytosine anywhere in the oligonucleotide can be replaced with guanine and uracil, respectively to form G-U Wobble base pairing with the target mRNA. Sequences containing such replacement moieties are suitable for the compositions and methods featured in the invention.

As used herein, "target sequence" refers to a contiguous portion of the nucleotide sequence of an mRNA molecule formed during the transcription of the gene, including mRNA that is a product of RNA processing of a primary transcription product.

As used herein, the term "strand including a sequence" refers to an oligonucleotide including a chain of nucleotides that is described by the sequence referred to using the standard nucleotide nomenclature.

As used herein, and unless otherwise indicated, the term "complementary," when used in the context of a nucleotide pair, means a classic Watson-Crick pair, *i.e.,* GC, AT, or AU. It also extends to classic Watson-Crick pairings where one or both of the nuclotides has been modified as decribed herein, *e.g.,* by a rbose modification or a phosphate backpone modification. It can also include pairing with an inosine or other entity that does not substantially alter the base pairing properties.

As used herein, and unless otherwise indicated, the term "complementary," when used to describe a first nucleotide sequence in relation to a second nucleotide sequence, refers to the ability of an oligonucleotide or polynucleotide including the first nucleotide sequence to hybridize and form a duplex structure under certain conditions with an oligonucleotide or polynucleotide including the second nucleotide sequence, as will be understood by the skilled person. Complementarity can include, full complementarity, substantial complementarity, and sufficient complementarity to allow hybridization under physiological conditions, e.g, under physiologically relevant conditions as may be encountered inside an organism. Full complementarity refers to complementarity, as defined above for an individual pair, at all of the pairs of the first and second sequence. When a sequence is "substantially complementary" with respect to a second sequence herein, the two sequences can be fully complementary, or they may form one or more, but generally not more than 4, 3 or 2 mismatched base pairs upon hybridization, while retaining the ability to hybridize under the conditions most relevant to their ultimate application. Substantial complementarity can also be defined as hybridization under stringent conditions, where stringent conditions may include: 400 mM NaCl, 40 mM PIPES pH 6.4, 1 mM EDTA, 50°C or 70°C for 12-16 hours followed by washing. The skilled person will be able to determine the set of conditions most appropriate for a test of complementarity of two sequences in accordance with the ultimate application of the hybridized nucleotides.

However, where two oligonucleotides are designed to form, upon hybridization, one or more single stranded overhangs, such overhangs shall not be regarded as mismatches with regard to the determination of complementarity. For example, a dsRNA including one oligonucleotide 21 nucleotides in length and another oligonucleotide 23 nucleotides in length, wherein the longer oligonucleotide includes a sequence of 21 nucleotides that is fully complementary to the shorter oligonucleotide, may yet be referred to as "fully complementary."

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs include, but are not limited to, G:U Wobble or Hoogstein base pairing.

The terms "complementary," "fully complementary," "substantially complementary" and sufficient complementarity to allow hybridization under physiological conditions, e.g, under physiologically relevant conditions as may be encountered inside an organism, may be used herein with respect to the base matching between the sense strand and the antisense strand of a dsRNA, or between the antisense strand of a dsRNA and a target sequence, as will be understood from the context of their use.

As used herein, a polynucleotide which is "complementary," *e.g.,* substantially complementary to at least part of" a messenger RNA (mRNA) refers to a polynucleotide which is complementary, *e.g.,* substantially complementary, to a contiguous portion of the mRNA of interest (*e.g.*, encoding CD45). For example, a polynucleotide is complementary to at least a part of a CD45 mRNA if the sequence is substantially complementary to a non-interrupted portion of an mRNA encoding CD45.

The term "double-stranded RNA" or "dsRNA", as used herein, refers to a ribonucleic acid molecule, or complex of ribonucleic acid molecules, having a duplex structure including two anti-parallel and substantially complementary, as defined above, nucleic acid strands. The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5'end of the respective other strand forming the duplex structure, the connecting RNA chain is referred to as a "hairpin loop". Where the two strands are connected covalently by means other than an uninterrupted chain of nucleotides between the 3'-end of one strand and the 5'end of the respective other strand forming the duplex structure, the connecting structure is referred to as a "linker." The RNA strands may have the same or a different number of nucleotides. The maximum number of base pairs is the number of nucleotides in the shortest strand of the dsRNA. In addition to the duplex structure, a dsRNA may comprise one or more nucleotide overhangs. A dsRNA as used herein is also refered to as a "small inhibitory RNA," "siRNA," "siRNA agent," "iRNA agent" or "RNAi agent."

As used herein, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. "Blunt" or "blunt end" means that there are no unpaired nucleotides at that end of the dsRNA, *i,e.,* no nucleotide overhang. A "blunt ended" dsRNA is a dsRNA that is double-stranded over its entire length, *i.e.,* no nucleotide overhang at either end of the molecule.

The term "antisense strand" refers to the strand of a dsRNA which includes a region that is substantially complementary to a target sequence. As used herein, the term "region of complementarity" refers to the region on the antisense strand that is substantially complementary to a sequence, for example a target sequence, as defined herein. Where the region of complementarity is not fully complementary to the target sequence, the mismatches may be in the internal or terminal regions of the molecule. Generally, the most tolerated mismatches are in the terminal regions, *e.g.*, within 6, 5, 4, 3, or 2 nucleotides of the 5' and/or 3' terminus.

The term "sense strand," as used herein, refers to the strand of a dsRNA that includes a region that is substantially complementary to a region of the antisense strand.

The term "identity" is the relationship between two or more polynucleotide sequences, as determined by comparing the sequences. Identity also means the degree of sequence relatedness between polynucleotide sequences, as determined by the match between strings of such sequences. While there exist a number of methods to measure identity between two polynucleotide sequences, the term is well known to skilled artisans (see, *e.g.,* Sequence Analysis in Molecular Biology, von Heinje, G., Academic Press (1987); and Sequence Analysis Primer, Gribskov., M. and Devereux, J., eds., M. Stockton Press, New York (1991)). "Substantially identical," as used herein, means there is a very high degree of homology (*e.g.*, 100% sequence identity) between the sense strand of the dsRNA and the corresponding part of the target gene. However, dsRNA having greater than 90%, or 95% sequence identity may be used in the invention, and thus sequence variations that might be expected due to genetic mutation, strain polymorphism, or evolutionary divergence can be tolerated. Although 100% identity is typical, the dsRNA may contain single or multiple base-pair random mismatches between the RNA and the target gene.

"Introducing into a cell," when referring to a dsRNA, means facilitating uptake or absorption into the cell, as is understood by those skilled in the art. Absorption or uptake of dsRNA can occur through unaided diffusive or active cellular processes, or by auxiliary agents or devices. The meaning of this term is not limited to cells *in vitro*; a dsRNA may also be "introduced into a cell," wherein the cell is part of a living organism. In such instance, introduction into the cell will include the delivery to the organism. For example, for *in vivo* delivery, dsRNA can be injected into a tissue site or administered systemically. *In vivo* delivery can also be by a beta-glucan delivery system, such as those described in U.S. Patent Nos. 5,032,401 and 5,607,677, and U.S. Publication No. 2005/0281781. U.S. Patent Nos. 5,032,401 and 5,607,677, and U.S. Publication No. 2005/0281781 are hereby incorporated by reference in their entirety. *In vitro* introduction into a cell includes methods known in the art such as electroporation and lipofection.

The terms "silence" and "inhibit the expression of," "down-regulate the expression of," "suppress the expression of," and the like, in as far as they refer to a gene expressed in an immune cell, *e.g.,* CD45, expressed, *e.g.,* in a macrophage, herein refer to the at least partial suppression of the expression of the CD45 gene, as manifested by a reduction of the amount of CD45 mRNA which may be isolated from a first cell or group of cells in which the CD45 gene is transcribed and which has or have been treated such that the expression of the CD45 gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to CD45 gene expression, *e.g.,* the amount of protein encoded by the CD45 gene, which is expressed in or secreted by a cell, or the number of cells displaying a certain phenotype, *e.g.,* apoptosis. In principle, CD45 gene silencing may be determined in any cell expressing CD45, either constitutively or by genomic engineering, and by any appropriate assay. However, when a reference is needed in order to determine whether a given dsRNA inhibits the expression of the CD45 gene by a certain degree and therefore is encompassed by the instant invention, the assays provided in the Examples below shall serve as such reference.

For example, in certain instances, expression of the CD45 gene is suppressed by at least about 20%, at least about 25%, at least about 30%, at least about 35%, at least about 40%, at least about 45%, or at least about 50% by administration of a nucleic acid-based agent, *e.g.,* a dsRNA, and where the gene expression is measured by an assay as described below in the Examples. In one embodiment, the CD45 gene is suppressed by at least about 60%, at least about 70%, or at least about 80%. In another embodiment, the CD45 gene is suppressed by at least about 85%, at least about 90%, or at least about 95%.

As used herein, the term "SNALP" refers to a stable nucleic acid-lipid particle. A SNALP represents a vesicle of lipids coating a reduced aqueous interior comprising a nucleic acid such as an iRNA agent or a plasmid from which an iRNA agent is transcribed. SNALPs are described, *e.g.*, in U.S. Patent Application Publication Nos. 20060240093, 20070135372, and USSN 61/045,228 filed April 15, 2008. These applications are hereby incorporated by reference.

The terms "treat," "treatment," and the like, refer to relief from or alleviation of a disease or disorder. In the

context insofar as it relates to any of the other conditions recited herein below (*e.g.*, a CD45-mediated condition, such as autoimmune or inflammatory disorder), the terms "treat," "treatment," and the like mean to relieve or alleviate at least one symptom associated with such condition, or to slow or reverse the progression of such condition.

A "therapeutically relevant" composition can alleviate a disease or disorder, or a symptom of a disease or disorder when administered at an appropriate dose.

As used herein, the term "CD45-mediated condition or disease" and related terms and phrases refer to a condition or disorder characterized by inappropriate, *e.g.,* greater than normal, CD45 activity. Inappropriate CD45 functional activity might arise as the result of CD45 expression in cells which normally do not express CD45, or increased CD45 expression (leading to, *e.g.,* a symptom of an inflammatory disorder or autoimmune disease). A CD45-mediated condition or disease may be completely or partially mediated by inappropriate CD45 functional activity. However, a CD45-mediated condition or disease is one in which modulation of CD45 results in some effect on the underlying condition or disorder (*e.g.*, a CD45 inhibitor results in some improvement in patient well-being in at least some patients).

As used herein, an "autoimmune disease" is any disorder that arises from an overactive response of the body against substances and tissues in the body. Exemplary autoimmune diseases suitable for treatment with the compositions described herein include arthritis (*e.g.*, rheumatoid arthritis), atherosclerosis, lupus, psoriasis, inflammatory bowel disease (IBD) (*e.g.*, Crohn's disease or ulcerative colitis), diabetes (*e.g.*, diabetes mellitus type I), chronic immune deficiency syndrome and autoimmune deficiency syndrome (AIDS).

As used herein, an "inflammatory disorder" is any disorder associated with inflammation. Inflammatory disorders may also be autoimmune disorders. Exemplary inflammatory disorders suitable for treatment with the compositions described herein include arthritis (*e.g.*, rheumatoid arthritis), inflammatory bowel disease (IBD) (*e.g.*, Crohn's disease or ulcerative colitis).

As used herein, the phrases "therapeutically effective amount" and "prophylactically effective amount" refer to an amount that provides a therapeutic benefit in the treatment, prevention, or management of an autoimmune or inflammatory disease, or an overt symptom of such disorder, *e.g.*, joint or muscle pain, swelling, weakness, or inflammation. The specific amount that is therapeutically effective can be readily determined by an ordinary medical practitioner, and may vary depending on factors known in the art, such as, *e.g.,* the type of autoimmune disorder, the patient's history and age, the stage of the disease, and the administration of other agents.

As used herein, a "pharmaceutical composition" includes a pharmacologically effective amount of a dsRNA and a pharmaceutically acceptable carrier. As used herein, "pharmacologically effective amount," "therapeutically effective amount" or simply "effective amount" refers to that amount of an RNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, a "transformed cell" is a cell into which a vector has been introduced from which a dsRNA molecule may be expressed.

*Pharmaceutical compositions*

The composition provided herein, *e.g.,* including a nucleic acid-based agent *e.g.,* a dsRNA, complexed with a lipid formulatin, can also include a pharmaceutically acceptable carrier, to provide a pharmaceutical composition. The pharmaceutical composition is useful for treating a disease or disorder associated with the expression or activity of the gene. Such pharmaceutical compositions are formulated based on the mode of delivery. One example is compositions that are formulated for systemic administration via parenteral delivery.

Pharmaceutical compositions including the identified agent are administered in dosages sufficient to inhibit expression of the target gene, *e.g.*, the CD45 gene. In general, a suitable dose of dsRNA agent will be in the range of 0.01 to 200.0 milligrams per kilogram body weight of the recipient per day, generally in the range of 0.02 to 50 mg per kilogram body weight per day. For example, the dsRNA can be administered at 0.01, 0.1, 0.05 mg/kg, 0.5 mg/kg, 1 mg/kg, 2 mg/kg, 3 mg/kg, 5 mg/kg, 10 mg/kg, 20 mg/kg, 30 mg/kg, 40 mg/kg, or 50 mg/kg per single dose. The pharmaceutical composition may be administered once daily, or the dsRNA may be administered as two, three, or more sub-doses at appropriate intervals throughout the day or even using continuous infusion or delivery through a controlled release formulation. In that case, the dsRNA contained in each sub-dose must be correspondingly smaller in order to achieve the total daily dosage. The dosage unit can also be compounded for delivery over several days, *e.g.*, using a conventional sustained release formulation which provides sustained release of the dsRNA over a several day period. Sustained release formulations are well known in the art and are particularly useful for vaginal delivery of agents, such as could be used with the nucleic acid-based agents described herein. In this embodiment, the dosage unit contains a corresponding multiple of the daily dose.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and *in vivo* half-lives for the individual dsRNAs encompassed by the invention can be made using conventional methodologies or on the basis of in *vivo* testing using an appropriate animal model, as described elsewhere herein.

In particular embodiments, pharmaceutical compositions containing the featured lipid-nucleic acid-based particles are prepared according to standard techniques and further include a pharmaceutically acceptable carrier. Generally, normal saline will be employed as the pharmaceutically acceptable carrier. Other suitable carriers include, *e.g.,* water, buffered water, 0.9% saline, 0.3% glycine, and the like, including glycoproteins for enhanced stability, such as albumin, lipoprotein, globulin, *etc.* In compositions containing saline or other salt containing carriers, the carrier is typically added following lipid particle formation. Thus, after the lipid-nucleic acid compositions are formed, the compositions can be diluted into pharmaceutically acceptable carriers such as normal saline.

The resulting pharmaceutical preparations may be sterilized by conventional, well known sterilization techniques. The aqueous solutions can then be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, *etc.* Additionally, the lipidic suspension may include lipid-protective agents which protect lipids against free-radical and lipid-peroxidative damages on storage. Lipophilic free-radical quenchers, such as $\alpha$-tocopherol and water-soluble iron-specific chelators, such as ferrioxamine, are suitable.

The concentration of lipid particle or lipid-nucleic acid particle in the pharmaceutical formulations can vary widely, *i.e.,* from less than about 0.01%, usually at or at least about 0.05-5% to as much as 10 to 30% by weight and will be selected primarily by fluid volumes, viscosities, *etc.,* in accordance with the particular mode of administration selected. For example, the concentration may be increased to lower the fluid load associated with treatment. This may be particularly desirable in patients having atherosclerosis-associated congestive heart failure or severe hypertension. Alternatively, complexes composed of irritating lipids may be diluted to low concentrations to lessen inflammation at the site of administration. In one group of embodiments, the nucleic acid will have an attached label and will be used for diagnosis (by indicating the presence of complementary nucleic acid). In this instance, the amount of complexes administered will depend upon the particular label used, the disease state being diagnosed and the judgement of the clinician but will generally be between about 0.01 and about 50 mg per kilogram of body weight, such as between about 0.1 and about 5 mg/kg of body weight.

As noted above, a lipid-therapeutic agent (*e.g.*, nucleic acid) particle may include polyethylene glycol (PEG)-modified phospholipids, PEG-ceramide, or ganglioside $G_{M1}$-modified lipids or other lipids effective to prevent or limit aggregation. Addition of such components does not merely prevent complex aggregation. Rather, it may also provide a means for increasing circulation lifetime and increasing the delivery of the lipid-nucleic acid composition to the target tissues.

The invention also provides lipid-therapeutic agent compositions in kit form. The kit will typically include a container that is compartmentalized for holding the various elements of the kit. The kit will contain the particles or pharmaceutical compositions, such as in dehydrated or concentrated form, with instructions for their rehydration or dilution and administration. In certain embodiments, the particles include the active agent, while in other embodiments, they do not.

The pharmaceutical compositions containing a nucleic acid-based agent complexed with a lipid formulation may be administered in a number of ways depending upon whether local or systemic treatment is desired and upon the area to be treated. Administration may be topical, pulmonary, *e.g.*, by inhalation or insufflation of powders or aerosols, including by nebulizer; intratracheal, intranasal, epidermal and transdermal), oral or parenteral. Administration may also be designed to result in preferential localization to particular tissues through local delivery, such as by direct intraarticular injection into joints, by rectal administration for direct delivery to the gut and intestines, by intravaginal administration for delivery to the cervix and vagina, by intravitreal administration for delivery to the eye. Parenteral administration includes intravenous, intraarterial, intraarticular, subcutaneous, intraperitoneal or intramuscular injection or infusion; or intracranial, *e.g.,* intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Typical topical formulations include those in which the nucleic acid-based agents, *e.g.*, the dsRNAs, are in admixture with a topical delivery component, such as a lipid, liposome, fatty acid, fatty acid ester, steroid, chelating agent or surfactant. Typical lipids and liposomes include neutral (*e.g.* dioleoylphosphatidyl DOPE ethanolamine, dimyristoylphosphatidyl choline DMPC, distearolyphosphatidyl choline) negative (*e.g.* dimyristoylphosphatidyl glycerol DMPG) and cationic (*e.g.* dioleoyltetramethylaminopropyl DOTAP and dioleoylphosphatidyl ethanolamine DOTMA). DsRNAs may be encapsulated within liposomes or may form complexes thereto, in particular to cationic liposomes. Alternatively, dsRNAs may be complexed to lipids, in particular to cationic lipids. Typical fatty acids and esters include but are not limited arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a $C_{1-10}$ alkyl ester (*e.g.* isopropylmyristate IPM), monoglyceride, diglyceride or pharmaceutically acceptable salt thereof. Topical formulations are described in detail in U.S. patent application Ser. No. 09/315,298 filed on May 20, 1999, which is incorporated herein by reference in its entirety.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Typical oral formulations are those in which the nucleic acid-based agents, *e.g.,* the dsRNAs, are administered in conjunction with one or more penetration enhancers surfactants and chelators. Typical surfactants include fatty acids and/or esters or salts thereof, bile acids and/or salts thereof. Typical bile acids/salts include chenodeoxycholic acid (CDCA) and ursodeoxychenodeoxycholic acid (UDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate and sodium glycodihydrofusidate. Typical fatty acids include arachidonic acid, undecanoic acid, oleic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a monoglyceride, a diglyceride or a pharmaceutically acceptable salt thereof (*e.g.* sodium). Combinations of penetration enhancers, for example, fatty acids/salts in combination with bile acids/salts are also common. A typical combination is the sodium salt of lauric acid, capric acid and UDCA. Other penetration enhancers include polyoxyethylene-9-lauryl ether, polyoxyethylene-20-cetyl ether. Nucleic acid-based agents, *e.g.,* dsRNAs, complexed with lipid formulations may be delivered orally, in granular form including sprayed dried particles, or complexed to form micro or nanoparticles. Complexing agents for use with nucleic acid-based agents include, *e.g.*, poly-amino acids; polyimines; polyacrylates; polyalkylacrylates, polyoxethanes, polyalkylcyanoacrylates; cationized gelatins, albumins, starches, acrylates, polyethyleneglycols (PEG) and starches; polyalkylcyanoacrylates; DEAE-derivatized polyimines, pollulans, celluloses and starches. Typical complexing agents include, *e.g.*, chitosan, N-trimethylchitosan, poly-L-lysine, polyhistidine, polyornithine, polyspermines, protamine, polyvinylpyridine, polythiodiethylaminomethylethylene P(TDAE), polyaminostyrene (*e.g.* p-amino), poly(methylcyanoacrylate), poly(ethylcyanoacrylate), poly(butylcyanoacrylate), poly(isobutylcyanoacrylate), poly(isohexylcynaoacrylate), DEAE-methacrylate, DEAE-hexylacrylate, DEAE-acrylamide, DEAE-albumin and DEAE-dextran, polymethylacrylate, polyhexylacrylate, poly(D,L-lactic acid), poly(DL-lactic-co-glycolic acid (PLGA), alginate, and polyethyleneglycol (PEG). Oral formulations for dsRNAs and their preparation are described in detail in U.S. application. Ser. No. 08/886,829 (filed Jul. 1,1997), Ser. No. 09/108,673 (filed Jul. 1, 1998), Ser. No. 09/256,515 (filed Feb. 23,1999), Ser. No. 09/082,624 (filed May 21, 1998) and Ser. No. 09/315,298 (filed May 20,1999), each of which is incorporated herein by reference in their entirety.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions featured herein may be formulated into any of many possible dosage forms such as, but not limited to, tablets, capsules, gel capsules, liquid syrups, soft gels, suppositories, and enemas. The compositions may

also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

In one embodiment, the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product. The preparation of such compositions and formulations is generally known to those skilled in the pharmaceutical and formulation arts and may be applied to the formulation of the compositions featured herein.

This invention is not limited in its application to the details of construction and the arrangement of components set forth in the following description. The invention is capable of other embodiments and of being practiced or of being carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

## EXAMPLES

The following examples are offered to illustrate, but not to limit the claimed invention.

**Example 1**: CD45 siRNAs complexed with LNP01 silenced CD45 gene expression in thioglycollate activated macrophages.

Mice (n=4) were administered thioglycollate by IP injection to activate macrophages. At three and five days after administration of thioglycollate, the mice were administered 10 mg/kg CD45, ICAM2 or GFP siRNA formulated with LNP01 by IP injection, and then mice were sacrificed at day 4 (LNP01 formulations are described, for example, in International Application publication WO2008/042973. Macrophages were isolated and analyzed by flow cytometry to determine uptake of siRNA and to assess the effect of the siRNAs on gene expression. CD45 and GFP LNP01-siRNAs, but not ICAM2 siRNAs were taken up by macrophages. Uptake of the CD45 siRNA resulted in a 65% reduction of CD45 gene expression. See FIGs. 1A and 1B.

**Example 2.** Alexa488-labeled siRNA in LNP01 was taken up by immune cells.

Mice were injected with 5 mg/kg Alexa488-siRNA in LNP01, and sacrificed two hours later. Leukocytes from spleen, liver and bone marrow were analyzed by flow cytometry. T cells were identified as being $CD5^+$, $CD11^-$; B cells were identified as being $CD19^+$, IgM/IgD; myeloid cells were identified as $CD5^-$, $CD11b^+$, $CD11c^-$; and dendritic cells were identified as $CD5^-$, $CD11b^+$, $CD11c^+$. Myeloid $CD11b^+$ cells include macrophages and granulocytes. The results indicated that the Alexa488-siRNA was taken up by B cells, myeloid cells, and dendritic cells. B cells bound the siRNA more efficiently than T cells (FIG. 2).

**Example 3**. No silencing was observed in liver macrophages with systemically delivered LNP01-formulated siRNA.

Balb/c mice (n=4 per group) were administered ICAM2 (AD3176) or Factor VII (AD-1661) LNP01 formulated siRNAs at 7.5mg/kg by intravenous injection. Mice were injected by i.v. at days 1,3, and 4, and then were sacrificed at day 6. Expression of ICAM2 in spleen and liver macrophages, and expression of serum factor VII was measured by FACS analysis. The results indicated that serum factor VII expression was inhibited by factor VII siRNA, but that ICAM2 expression in liver and spleen macrophages was not silenced (FIGs. 3A and 3B). The results indicated that macrophages absorbed the siRNA, but that there was no target gene silencing.

**Example 4.** SNALP (stable nucleic acid lipid particle) liposome formulations targeted siRNAs to leukocytes.

Cy3-labeled siRNA formulated in liposomes in SNALP liposomes (Tekmira Pharmaceuticals (British Columbia, Canada)) previously showed localization to macrophage-rich areas with DMA and DAP formulations in rat. These siRNAs were therefore tested for gene silencing in macrophages.

CD45 and ICAM2 siRNAs were formulated with the following four different SNALP liposomes:

DLinDMA:DSPC:Chol:PEG-DMG (40: 10:40:10 mole ratio)
DLinDAP:DSPC:Chol:PEG-DMG (40:10:40:10 mole ratio)
DODMA:DSPC:Chol:PEG-DMG (25:20:45:10 mole ratio)
DLinDMA:Chol:PEG-DMG (50:40:10 mole ratio)

The liposome-formulated siRNAs were administered intravenously and intraperitoneally.

No silencing was observed in the spleen using these formulations.

**Example 5.** Splenic LNP-SNALP localization suggests leukocytic uptake.

Cy3-SNALP-CD45 was administered to mice intravenously and intraperitoneally. After 1.5 hr., uptake of the siRNA was primarily into "red pulp," a highly vascular tissue of the spleen containing macrophages, fibroblasts, erythrocytes and leukocytes. After 4 hours, the siRNA was still localized primarily to red pulp, but began to migrate into the marginal zone of the spleen, which is mostly populated with lymphocytes. After 10 hours, siRNA uptake was primarily in white pulp, which is lymphoid tissue that includes (i) a germinal center containing B lymphocytes, and (ii) the marginal zone. After 24 hours, siRNA uptake was observed primarily in white pulp and the germinal center.

**Example 6.** LNP08 (XTC) formulated CD45 siRNAs silenced CD45 expression in leukocytes in the peritoneal cavity of mice.

Naïve C57BL/6 mice (n=3) were injected with an LNP08 formulation containing either CD45 siRNA or Luc siRNA at 3 mg/kg, by intravenous or intraperitoneal injection. Three days post injection, leukocytes were analyzed from spleen, bone marrow, peritoneal cavity, Peyer's Patches, and liver. Leukocytes were stained with antibodies for combinations of the cell surface markers CD45, GR-1, CD11b (Mac1), CD11c, CD45, NK1.1, CD19, and TCR-beta.

CD45 was observed to be downregulated in $CD11b^+$ and $CD11c^+$ cells (in macrophages and dendritic cells) in the peritoneal cavity following either i.v. or i.p. injection of CD45 siRNA (FIGs. 4A and 4B). The silencing activity observed following administration of the siRNA by i.v. was surprising, as those of skill in the art have generally found that administration of siRNA by i.v. does not result in efficient gene silencing.

LNP08 formulated CD45 and luciferase siRNAs were both taken up by bone marrow leukocytes when administered by i.p. and i.v. (FIGs. 5A and 5B), and CD45 siRNAs were able to silence gene expression by both routes of administration (FIG. 5C). Again, it was particularly surprising that administration of the siRNA by i.v. was effective to down regulate gene expression.

A mild effect on CD45 expression in lymphocytes in the peritoneal cavity was also observed, including in B cells, NK (natural killer) cells, and T cells, following administration of siRNAs injection by i.p., and in B cells and NK cells following injection by i.v. (FIG. 6). Again, it was surprising to see down regulation of gene expression following administration by i.v.

In splenic cells, CD45 siRNAs decreased expression in B cell lymphocytes following i.p. injection, and in CD11b+ leukocytes following i.p. injection (FIGs. 7A and 7B).

CD45 siRNAs did not effect CD45 gene expression in B cells, NK cells, T cells or $CD11b^+$ $GR\text{-}1^+$ cells in Peyer's Patches (FIG. 8A), nor in leukocytes of the liver (FIG. 8B).

Lipid A formulations, which contain the lipid XTC, were also tested to determine a correlation between formulation uptake and silencing. Mice were injected with Lipid A formulations containing either CD45 (GFP) or luciferase siRNA by i.v. at 3 mg/kg, n=3 mice. CD45 and GFP are high abundance and very stable proteins. Three days post-injection, leukocytes were analyzed from spleen, bone marrow, peritoneal cavity, Peyer's Patches, liver and lymph nodes. Leukocyte subpopulations were assayed for silencing at the protein level by flow cytometry.

All live cells were gated for analysis. Distinct populations were identified based on cell surface markers and gated separately. Mean fluorescence intensity (MFI) of CD45 was determined for each population, and the percent knockdown was calculated by taking the percent difference in MFI between siRNA treated and control animals.

CD45 silencing was observed most strongly in macrophages and dendritic cells in the peritoneal cavity (FIG. 9). Weaker silencing was observed in the spleen, bone marrow and liver, and no significant knockdown was observed in lymphocytes (T cells, B cells, and natural killer cells). ApoE-/- mice showed the same knockdown in splenic and peritoneal cavity myeloid cells as wildtype mice. The results shown in FIG. 9 were averaged across four independent experiments.

FACS (fluorescence activated cell sorting) analysis indicated uptake of the lipid A-formulated CD45 siRNAs by macrophages and dendritic cells of the peritoneal cavity (FIGs. 10A and 10B). CD45 silencing was observed in peritoneal leukocytes 72 hours after injection (FIG. 10C), and similar results were seen with GFP siRNA in GFP transgenic mice.

In dose response experiments, both macrophage and dendritic cell silencing was observed at 0.3 mg/kg, but not at 0.1 mg/kg (FIG. 11C). FIGs. 11A and 11B indicate that there was greater uptake of the siRNAs at the higher dosage levels.

In another set of experiment, Lipid A formulations encapsulating Alexa 647 labeled siRNA were injected i.v. at 1 mg/kg (n=3 mice per group). FACS was used to measure the uptake of the lipids by macrophages, monocytes, B cells and T cells in the peritoneal cavity, bone marrow, spleen, periaortic lymph nodes and blood. The results are shown in FIG. 12. The periaortic nymph node showed less uptake than bone marrow.

The results of the study indicated that lipid A formulations were efficiently taken up by blood monocytes, and maximal uptake was achieved by 15 minutes. Blood monocytes may migrate to the peritoneal cavity after LNP uptake (see FIG. 13). Spleen macrophages showed lower uptake than seen in blood, and high uptake was observed in myeloid cells in the peritoneal cavity, although the kinetics of uptake were slower than that observed for the spleen and the blood monocytes. The high uptake observed in the peritoneal cavity is consistent with the high silencing observed in the peritoneal cavity.

**Example 7.** LNP09-formulated siRNAs silenced gene expression in leukocytes of the spleen, blood, and peritoneal cavity.

Earlier experiments showed that by 72 hours post-administration, most macrophages that demonstrate silencing by the lipid-formulated dsRNAs are located in the peritoneal cavity. Further studies were therefore designed to address the question of whether lipid-formulated dsRNAs are targeted to the cells of the peritoneal cavity, or whether cells located elsewhere take up the dsRNA first, and then the cells migrate to the peritoneal cavity.

Naïve C57BL/6 mice (n=3) were injected with LNP09- (XTC-) formulated CD45 dsRNA or Luciferase dsRNA. Injections were performed intravenously at 3 mg/kg. Leukocytes (including macrophages and monocytes) were isolated from spleen, peripheral blood, bone marrow and the peritoneal cavity 15 minutes, 1 hour, and 2 hours post administration, and the cells were cultured in vitro for 72 hours without any additional activating stimuli. Cells were then collected and CD45 levels were quantified by flow cytomometry. Leukocytes were stained with antibodies for combinations of surface markers: CD45, GR-1, CD11b (Mac1), and CD11c. The results are depicted in FIGs. 14A to 14D.

FIG. 14A shows that leukocytes isolated from bone marrow did not exhibit any silencing activity following administration of CD45 dsRNAs. FIG. 14B shows that leukocytes isolated from spleen tissue demonstrated an increase in silencing over the first hour and maintained this level of silencing through the second hour. FIG. 14D shows that leukocytes isolated from the peritoneal cavity demonstrated a CD45 gene silencing effect that increased over the period of two hours. In contrast, FIG. 14C shows that leukocytes in the blood stream experienced an initial gene silencing effect but fewer cells were identified that had CD45 silencing at later time points.

These experiments revealed that silencing occurs in peripheral leukocytes (in leukocytes in the bloodstream and spleen), and reaches 50-60% silencing, which is comparable to the effect seen in the peritoneal cavity by three days post-injection ex *vivo.*

The results indicated that peripheral leukocytes can be successfully targeted with siRNA containing LNP formulations. The peritoneal cavity may be either a migratory site and/or a later liposomal migration path.

**Example 8.** Lipid T-formulated CD45 siRNAs silenced CD45 expression in leukocytes in the peritoneal cavity of mice.

Naïve C57BL/6 mice (n=3) were injected with a lipid formulation containing either CD45 siRNA (AD3215) or Luc siRNA at 3 mg/kg, by i.v. or i.p. injection. The formulation included Lipid T, DSPC, Cholesterol and PEG in the following mol%:

| Lipid T | DSPC | Cholesterol | PEG | siRNA | Lipid T/siRNA | Total Lipid /siRNA |
|---|---|---|---|---|---|---|
| 50.0 | 7.5 | 37.5 | 5.0 | 3.2 | 4.75 | 7.03 |

AD3215 siRNA has sense and antisense strands, respectively, as indicated below:

| Strand ID | SEQ ID NO: | Sense strand (5'to 3') | Strand ID | SEQ ID NO: | Anti-sense strand (5'to 3') |
|---|---|---|---|---|---|
| A22825 | 1 | cuGGcuGAAuuucAGAGcATsT | A22826 | 2 | UGCUCUGAAAUUcAGCcAGTsT |

Three days post injection, leukocytes were analyzed from spleen, bone marrow, peritoneal cavity, Peyer's Patches, and liver. Leukocytes were stained with antibodies for combinations of the cell surface markers CD45, GR-1, CD11b (Mac1), CD11c, CD45, NK1.1, CD19, and TCR-beta. CD11b is a myeloid cell marker abundant on macrophages; CD11c is a myeloid cell marker found at high density on dendritic cells as well as other myeloid cells; GR-1 is a granulocyte marker; CD19 is a B-cell marker, TCR-beta is a T cell marker, and NK1.1 is a marker for natural killer cells.

Silencing by CD45 siRNAs was observed in macrophages and dendritic cells of the peritoneal cavity (FIGs. 15A and 15B), while CD45 in lymphocytes was not observed (FIG. 16). Again, it was particularly surprising to observe gene silencing activity following administration of the siRNA by i.v. injection.

CD45 siRNAs were also taken up by bone marrow leukocytes following administration by i.p. or i.v. (FIGs. 17A and 17B), and the siRNAs were effective to silence gene expression of leukocytes by either route of administration (FIG.

17C). Again, it was particularly surprising to observe gene silencing activity following administration of the siRNA by i.v. injection.

CD45 siRNAs were also tested for an effect on CD45 gene expression in leukocytes of the liver (FIG. 18A), spleen (FIG. 18B), or in Peyer's patch lymphocytes (FIG. 18C).

In a second set of experiments, the dsRNA formulated into LNP12, the lipid formulation containing Lipid T (TechG1), was administered by i.v. injection of naïve C57BL/6 mice (n=3) as described above. Three days post-injection, leukocytes were analyzed from spleen, bone marrow, peritoneal cavity, liver, and lymph node. Leukocyte subpopulations were assayed for silencing at the protein level by flow cytometry. All live cells were gated for analysis. Distinct populations were identified based on cell surface markers and gated separately. The mean fluorescence intensity (MFI) of CD45 was determined for each population, and the percent knock-down was calculated as the percent difference in MFI between siRNA treated and control mice.

These experiments revealed ~90% knockdown in macrophages of the peritoneal cavity (FIGs. 19A and 19B). Improved silencing was also observed in the macrophages and dendritic cells of the spleen (FIGs. 20A and 20B). The lipid formulations containing lipid T (e.g., LNP12) were observed to more effectively silence activity in the spleen than formulations containing Lipid A (XTC) or Lipid M (MC3).

The IC50 values for non-targeted liposomes in naive mice as determined from a single bolus dose administered i.v. is shown in the Table below. Maximal silencing was observed observed in the peritoneal cavity. Lipid T has proven to be the most efficacious lipid component to date for leukocyte silencing (IC50 = 0.3 mg/kg).

| LNP-Formulation | IC50 |
|---|---|
| Lipid A (LNP09: Lipid A/DSPC/Chol/PEG-DMG 50/10/38.5/1.5)) | 0.3-0.5 mg/kg |
| Lipid M (LNP11: Lipid M/DSPC/Chol/PEG-DMG 50/10/38.5/1.5) | ~1 mg/kg |
| Lipid T (LNP12: Lipid T/DSPC/Chol/PEG-DMG 50/10/38.5/1.5) | < 0.3 mg/kg |

**Example 9.** Optimization of formulations containing lipid A for enhanced immune cell targeting

To identify liposomal formulations with increased delivery of agents to immune cells, various lipid particles were formulated containing siRNAs targeting Factor VII (FVII), a liver-specific gene and CD45 (EC 3.1.34) in immune cells by siRNAs. A total of eight formulations with varying amounts of Lipid A, DSPC, Cholesterol and a PEG-lipid (either C14-PEG, which is PEG-dimyristoylglycerol (PEG-DMG), or C18-PEG, which is PEG-distyryl glycerol (PEG-DSG); in both cases, the average molecular weight of the PEG moiety is about 2,000) containing either CD45 siRNA or Luc/Factor VII (9:1) siRNA were tested by administration in naive C57BL/6 mice at a volume of 3mg/kg by i.v. (N=3). A lower amount of Factor VII siRNA was used since the base formulation containing lipid A is 10x more active for liver silencing than for leukocyte silencing. Luc siRNA was included in the Factor VII formulation to achieve the same total dose of siRNA as in the CD45 siRNA formulation. Three days after injection, leukocytes were collected from the peritoneal cavity and Factor VII was quantified from serum. Leukocytes were stained with antibodies for a combination of surface markers including CD45, GR-1, CD11b (Mac1) as a Macrophage specific marker, and CD11c as a dendritic cell (DC) marker.

The lipid A-containing formulations tested were:

| Formulation Lipid A: DSPC: Chol: PEG lipid (either C14 or C18) | Group | siRNA | Lipid/siRNA | d (nm) |
|---|---|---|---|---|
| C14(50/10/30/10) | A | 3215 | | |
| C14(50/10/30/10) | B | 1955/1661 | 14 | 55 |
| C18(50/10/30/10) | C | 3215 | | |
| C18(50/10/30/10) | D | 1955/1661 | 14 | 50 |
| C14(50/10/38.5/1.5) | E | 3215 | | |
| C14(50/10/38.5/1.5) | F | 1955/1661 | 10 | 75 |
| C18(50/10/38.5/1.5) | G | 3215 | | |
| C18(50/10/38.5/1.5) | H | 1955/1661 | 10 | 93 |

(continued)

| Formulation Lipid A: DSPC: Chol: PEG lipid (either C14 or C18) | Group | siRNA | Lipid/siRNA | d (nm) |
|---|---|---|---|---|
| 30/30/30/10-C14 | J | 3215 | | |
| 30/30/30/10-C14 | K | 1955/1661 | 24 | 67 |
| 30/30/30/10-C18 | L | 3215 | | |
| 30/30/30/10-C18 | M | 1955/1661 | 24 | 66 |
| 30/30/38.5/1.5-C14 | N | 3215 | | |
| 30/30/38.5/1.5-C14 | O | 1955/1661 | 18 | 117 |
| 30/30/38.5/1.5-C18 | P | 3215 | | |
| 30/30/38.5/1.5-C18 | Q | 1955/1661 | 18 | 116 |

SiRNAs 3215,1955 and 1661 target CD45, luciferase (Luc) and Factor VII, respectively.

Silencing of CD45 in Mac1+macrophages or CD11c+dendritic cells (DCs) is shown in FIG. 21A. Silencing of FVII in liver is shown in FIG. 21B. Correlation plots for CD45 and FVII silencing are shown in FIGs. 22A and 22B.

In macrophages, some formulations showed strong silencing of CD45 (e.g., N/O or E/F>G/H>P/Q>J/K). Similarly, some formulations showed strong silencing of CD45 in dendritic cells (e.g., E/F>G/H>N/O>P/Q>J/K).

In conclusion, formulations such as E/F, G/H, J/K, N/O, and P/Q showed strong silencing in immune cells (both macrophages and dendritic cells). In some formulations (e.g., N/O, and P/Q), there appeared to be more selective silencing in immune cells when compared with the liver.

**Example 10.** Preparation of various sized liposomes

In order to test whether liposomal formulations having different particle sizes have an effect in specific immune cell targeting, new methods were developed to make liposome particles of different sizes. The following procedure was based on the idea that liposomal particles, in the absence of agents that prevent fusion (e.g., PEG-lipids) can be made to undergo fusion reactions under certain conditions. By closely monitoring the progress of such fusion reaction, liposomes of large sizes can be reproducibly prepared.

Liposomes were prepared by adding sodium acetate buffer (0.3M, pH5.2) to a Lipid premix solution. The lipid premix solution (20.4 mg/ml total lipid concentration containing Lipid A/ cholesterol/ DSPC=50:10:30 molar ratios in ethanol) was prepared from each lipid stock solution. This lipid premix solution contained no PEG-lipids.

After addition of the sodium acetate buffer to the Lipid premix solution, the mixture was hydrated at a molar ratio of acetate to Lipid A of 0.5 (the resulting mixture had an ethanol concentration of about 97%). The lipids were subsequently hydrated by combining the mixture with 1.85 volumes of citrate buffer (10 mM, pH 3.0) with vigorous stirring. Subsequently, liposome solution was incubated at 37°C to induce fusion. Aliquots were removed at various times.

To investigate changes in liposome size during incubation, aliquots of the liposome solution were collected and diluted (1:500) to measure their sizes. Liposome particle size (d, in nm) and polydispersity indices (PDI) of liposomes were measured using the Zetasizer nano ZS (Malvern Instruments, Worcestershire, UK). The size of the liposomes grew as a function of time (FIG. 23A). Certain parameters were found to affect the rate of increase in the diameter of the liposomes, including temperature, sodium concentration, and pH. For example, liposome growth was faster at higher temperatures. In contrast, lower concentrations of sodium were found to reduce the rate of aggregation and liposome growth: at sodium concentrations above 100 mM, the liposomes aggregated too quickly to monitor increases in size, whereas decreasing the sodium concentration as is used here allowed the fusion reaction to proceed in a more controlled way.

Random fusion of liposomal particles in the fusion reaction would be expected to result in a steady increase in size distribution as the fusion reaction progresses. Surprisingly, while the size of liposomes steadily increased as a function of time (FIG. 23A), the polydispersity index (PDI) of the liposome remained low (FIG. 23B), indicating that the size distribution of the liposomes remained fairly uniform in spite of the increase in size due to fusion events. Therefore, the size distribution profiles were mostly parallel shifted (see, for example, FIG. 23C).

To investigate whether addition of PEG-lipids could serve to quench fusion and maintain liposomes at that size, aliquots of liposomes in a fusion reaction were removed at various times (t=0 to 150 min) after initiation of the fusion reaction and mixed with an aqueous PEG lipid solution (stock= 10 mg/mL PEG-C14 in 35% (v/v) ethanol) at a final PEG molar concentration of 3.5% of total lipid with vigorous stirring. Results showed that, upon addition of PEG-lipids, the liposomes maintained the size with apparently no significant additional fusion events, effectively quenching further growth

of the liposomes.

Following addition of the PEG lipids, the empty liposomes were loaded with siRNAs by addition of a half volume of an siRNA solution (stock= 1.5 mg/mL siRNA in 35% ethanol), followed by incubation for 30 min at 37°C. The mixture was subsequently dialyzed overnight in PBS. As a result, the different sized liposomes were obtained with low polydispersity index. Using this method, liposomes of particle size of ~200 nm, and some greater than 300 nm or even greater than 600 nm were easily generated.

These results indicated that the PEG-lipids can serve to effectively quench growth of liposome size in the fusion reaction. Therefore, liposomes of various sizes can be conveniently obtained by means of performing a fusion reaction in a mixture devoid of components such as the PEG-lipids that prevent fusion, followed by subsequent addition of a PEG-lipid after the fusion reaction is permitted to continue until the desired liposome size is reached. The reaction can be easily monitored for size and size distribution (e.g., by measuring PDI), and quenched by addition of reagents which inhibit further fusion (e.g., PEG-lipids), or by dilution. The liposomes obtained using this method are surprisingly uniform in size, as evidenced by the relatively low PDI values.

Table 4. Size measurements of various sized liposomes.

| | Time (min) | Peak 1 | PDI |
|---|---|---|---|
| | | d.nm | |
| Blue | 0 | 105 | 0.037 |
| Red | 10 | 199 | 0.052 |
| Black | 60 | 326 | 0.256 |
| Green | 150 | 654 | 0.126 |

**Example 11.** Optimization of the size of lipid particles for enhanced immune cell targeting

To test the ability of liposomal formulations having different particle sizes to selectively target immune cells, various lipid particles were formulated containing siRNAs targeting Factor VII (FVII), a liver-specific gene or CD45 (EC 3.1.34) present in immune cells, using a method essentially as described above in Example 9, using either PEG-C14(PEG-DMG) or PEG-C18 (PEG-DSG). A total of eight pairs of lipid particles were prepared. These lipid particles varied in either the nature and/or amount of composition in the lipid formulation or the particle size. Lipid particles containing either CD45 siRNA or Luc/Factor VII (9:1) siRNA were tested by administration in naïve C57BL/6 mice at a volume of 3mg/kg by i.v. (N=3). siRNAs 3215,1955 and 1661 target CD45, luciferase (Luc) and Factor VII, respectively. A lower amount of Factor VII siRNA was used since the base Lipid A-containing formulation is 10x more active for liver silencing than for leukocyte silencing. Luc siRNA was included in the Factor VII formulation to achieve the same total dose of siRNA as in the CD45 siRNA formulation.

Three days after injection, leukocytes were collected from the peritoneal cavity and spleen; Factor VII was quantified from serum using a chromogenic assay (Coaset Factor VII, DiaPharma Group, OH or Biophen FVII, Aniara Corporation, OH) according to manufacturer protocols. Leukocytes were stained with antibodies for a combination of surface markers including CD45, GR-1, CD11b (Mac1) as a Macrophage specific marker, and CD11c as a dendritic cell (DC) marker. The formulations tested are as shown below in Table 5. Either C14-PEG (PEG-dimyristoylglycerol (PEG-DMG)) or C18-PEG (PEG-distyryl glycerol (PEG-DSG)) as indicated was used in the formulations. In both cases, the average molecular weight of the PEG moiety in the C14-PEG and C18-PEG is about 2,000.

**Table 5**

| **Formulation(Lipid A/DSPC/ Cholesterol/ PEG-lipid) Ratios in molar %** | **Group** | **siRNA** | **size, d (nm)** |
|---|---|---|---|
| C14(50/10/38.5/1.5) large | R | 3215 | 355 |
| C14(50/10/38.5/1.5) large | S | 1955/1661 | 355 |
| C14(50/10138.5/1.5) medium | T | 3215 | 188 |
| C14(50/10/38.5/1.5) medium | U | 1955/1661 | 199 |
| C14(50/10/38.5/1.5) | E | 3215 | 75 |
| C14(50/10/38.5/1.5) | F | 1955/1661 | 75 |
| C18(40/20/38.5/1.5) | AA | 3215 | 79 |

(continued)

| Formulation(Lipid A/DSPC/ Cholesterol/ PEG-lipid) Ratios in molar % | Group | siRNA | size, d (nm) |
|---|---|---|---|
| C18(40/20/38.5/1.5) | BB | 1955/1661 | 80 |
| C14(40/20/38.5/1.5) | CC | 3215 | 77 |
| C14(40/20/38.5/1.5) | DD | 1955/1661 | 80 |
| C18(30/30/38.5/1.5) | P | 3215 | 116 |
| C18(30/30/38.5/1.5) | Q | 1955/1661 | 118 |
| C18(30/30/38.5/1.5) large | V | 3215 | 331 |
| C18(30/30/38.5/1.5) large | W | 1955/1661 | 330 |
| C18(30/30/38.5/1.5) medium | X | 3215 | 160 |
| C18(30/30/38.5/1.5) medium | Y | 1955/1661 | 180 |
| C18(30/30/38.5/1.5) | P | 3215 | 116 |
| C18(30/30/38.5/1.5) | Q | 1955/1661 | 118 |

The results are shown in FIGs. 24A-24D. FIG. 24A shows the silencing of FVII in liver. FIG. 24B shows the silencing of CD45 in peritoneal CD11c+ dendritic cells (DCs) or Mac1+ macrophages. FIG. 24C shows the silencing of CD45 in CD11c+ or Mac1+ splenocytes. FIG. 24D shows a correlation plot for CD45 silencing in macrophages and FVII silencing in liver.

As demonstrated in FIGs. 24A and 24D, lipid particles having the same formulation and differing only in particle size showed significantly different silencing of FVII. For example, formulation E/F showed stronger silencing of FVII than formulation T/U, which showed much stronger silencing of FVII than formulation R/S. As shown in FIGs. 24B to 24D, formulations having different particle size had much a less of an effect on the silencing of CD45. Larger sized liposomal formulations did not drastically diminish silencing in leukocytes, but appeared to significantly diminish silencing in liver cells. As shown in FIG. 24D, formulations P/Q and R/S appeared to be more selective silencing in immune cells when compared with the liver.

In addition, as shown in FIGs. 24B and 24C, formulations CC/DD and E/F, which have similar particle size, showed similar silencing of CD45 in macrophages.

**Example 12**. Liposomal formulations silence gene expression in a dosage-dependent manner in primary macrophages *in vitro.*

LNP-01 exhibited silencing of CD45 in primary macrophages in a dosage dependent manner *in vitro* (FIG. 25A). The $IC_{50}$ value was determined to be ~100 nM. The silencing of CD45 in primary macrophages using an LNP08 formulation *in vitro* was also dosage-dependent (FIGs. 25B). The $IC_{50}$ value using the LNP08 formulation was determined to be ~5 nM.

LNP08 formulations also demonstrated dosage dependent CD45 silencing in *vivo* in macrophages and dendritic cells of the peritoneal cavity (FIG. 26). No *in vivo* systemic silencing was observed with the lipid formulations LNP-01, DODMA, or DLinDMA, despite the accumulation of siRNA in cells.

**Example 13.** Lipid M-formulated siRNAs (formulated with MC3) exhibited a less steep dose-response than lipid A- (XTC-) formulated siRNAs. and a lower IC50.

The results of a dose response experiment are shown in FIGs. 27A-C. A less steep dose-response was observed with the lipid M-formulated siRNAs than with the lipid A- (XTC-)formulated siRNAs. The lipid M-formulated siRNAs also exhibited a lower IC50, and less maximal silencing. FACS analysis indicating uptake of the lipid M-formulated siRNAs into macrophages and dendritic cells is shown in FIGs. 27A and 27B. Silencing data is presented in FIG. 27C. Silencing was dose dependent. There was almost no silencing observed in dendritic cells below a dose of 3 mg/kg.

Lipid M (MC3) and structurally similar lipids are dislosed at least in PCT/US2009/063933, filed November 10, 2009; PCT/US2009/063931, filed November 10,2009; PCT/US2009/063927, filed November 10, 2009; PCT/US2010/22614, filed January 29,2010; U.S.S.N 61/185,800, filed June 10, 2009; and U.S.S.N. 61/299291, filed January 28, 2010. The contents of each of these applications are incorporated by reference herein in their entirety for all purposes.

**Example 14.** Silencing was enhanced by multi-dosing regimens.

To determine whether silencing could be improved and whether leukocytes in places other than the peritoneal cavity could be more efficiently reached, multiple doses of LNP-siRNA were administered according to the following protocol. Naive C57BL/6 mice were injected with lipid A- (XTC-) or lipid M- (MC3-) containing formulations for three consecutive days or once at 1 mg/kg, by i.v. (n=2). Three days after the last injection, leukocytes and lymphocytes were analyzed from peritoneal cavity, spleen, bone marrow, liver, and blood. The results are shown in FIGs. 28A to 28D.

Some improvement in silencing in peritoneal cavity monocytes and in B cells was observed as a result of multidosing. Improved silencing in the splenic dendrocytes was observed with both lipid A- and lipid M-containing formulations (FIGs. 28A and 28B). Also, the multidosing resulted in the first detectable reliable silencing in bone marrow macrophages, dendritic cells and B cells with a 3X dose of lipid A (FIG. 28C). Thus, a multidosing regimen may provide additional target organs for leukocyte silencing as well as reach more cell types.

Having thus described several aspects of at least one embodiment of this invention, it is to be appreciated that various alterations, modifications, and improvements will readily occur to those skilled in the art. Such alterations, modifications, and improvements are intended to be part of this disclosure, and are intended to be within the spirit and scope of the invention. Accordingly, the foregoing description is by way of example only.

ITEMS

1. A method of delivering a nucleic acid-based agent to an immune cell, comprising providing a nucleic acid-based agent complexed with a formulation comprising a sterol; a neutral lipid; a PEG or a PEG-modified lipid; and a cationic lipid selected from the group consisting of :

(i) a cationic lipid having the structure of formula (I)

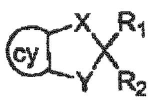 (I),

salts or isomers thereof, wherein:

cy is optionally substituted cyclic, optionally substituted heterocyclic or heterocycle, optionally substituted aryl or optionally substituted heteroaryl;
$R_1$ and $R_2$ are each independently for each occurrence optionally substituted $C_{10}$-$C_{30}$ alkyl, optionally substituted $C_{10}$-$C_{30}$ alkenyl, optionally substituted $C_{10}$-$C_{30}$ alkynyl, optionally substituted $C_{10}$-$C_{30}$ acyl or -linker-ligand;
X and Y are each independently O or S, alkyl or N(Q); and
Q is H, alkyl, acyl, ω-aminoalkyl, ω-(substituted)aminoalky, ω-phosphoalkyl or ω-thiophosphoalkyl;

(ii) a cationic lipid having the structure of formula (II)

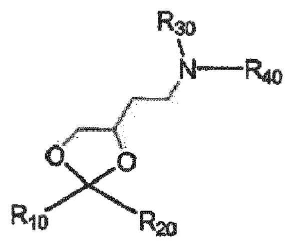 (II),

where $R_{10}$ and $R_{20}$ are independently alkyl, alkenyl or alkynyl, each can be optionally substituted, and $R_{30}$ and $R_{40}$ are independently lower alkyl or $R_{30}$ and $R_{40}$ can be taken together to form an optionally substituted heterocyclic ring;
(iii) a cationic lipid having the structure

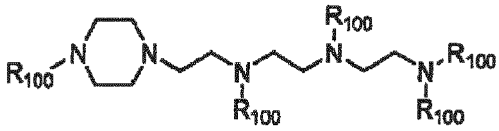
formula (III)

or

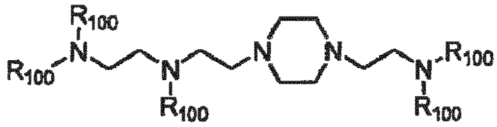

formula (IV),

wherein each R is independently H, alkyl,

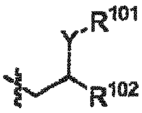, or 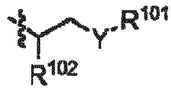 ;

provided that at least one R is

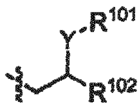, or 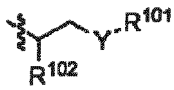 ;

wherein $R^{100}$, for each occurrence, is independently H, $R^{103}$,

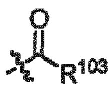, 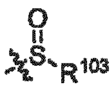, 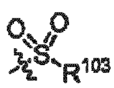, 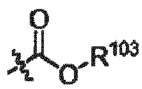,

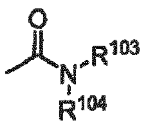, or 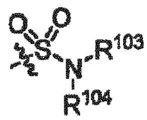 ;

wherein $R^{103}$ is optionally substituted with one or more substituent;

$R^{102}$, for each occurrence, is independently, alkyl, alkenyl, alkynyl, heteroalkyl, heteroalkenyl, or heteroalkynyl; each of which is optionally substituted with one or more substituent;

$R^{103}$, for each occurrence, is independently, alkyl, alkenyl, alkynyl, heteroalkyl, heteroalkenyl, or heteroalkynyl; each of which is optionally substituted with one or more substituent;

Y, for each occurrence, is independently O, $NR^{104}$, or S;

$R^{104}$, for each occurrence is independently H alkyl, alkenyl, alkynyl, heteroalkyl, heteroalkenyl, or heteroalkynyl; each of which is optionally substituted with one or more substituent; and

(iv) a cationic lipid having the structure

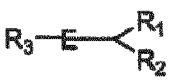 formula (V),

wherein $R_1$ and $R_2$ are each independently for each occurrence optionally substituted $C_{10}$-$C_{30}$ alkyl, optionally substituted $C_{10}$-$C_{30}$ alkoxy, optionally substituted $C_{10}$-$C_{30}$ alkenyl, optionally substituted $C_{10}$-$C_{30}$ alkenyloxy, optionally substituted $C_{10}$-$C_{30}$ alkynyl, optionally substituted $C_{10}$-$C_{30}$ alkynyloxy, or optionally substituted $C_{10}$-$C_{30}$ acyl;

E is -O-, -S-, -N(Q)-, -C(O)O-, -OC(O)-, -C(O)-, -N(Q)C(O)-, -C(O)N(Q)-, -N(Q)C(O)O-, -OC(O)N(Q)-, S(O), -N(Q)S(O)$_2$N(Q)-, -S(O)$_2$-, -N(Q)S(O)$_2$-, -SS-,-O-N=, =N-O-, -C(O)-N(Q)-N=, -N(Q)-N=, -N(Q)-O-, -C(O)S-, arylene, heteroarylene, cyclalkylene, or heterocyclylene; and

Q is H, alkyl, ω-aminoalkyl, ω-(substituted)aminoalkyl, ω-phosphoalkyl or ω-thiophosphoalkyl; and

$R_3$ is H, optionally substituted $C_1$-$C_{10}$ alkyl, optionally substituted $C_2$-$C_{10}$ alkenyl, optionally substituted $C_2$-$C_{10}$ alkynyl, optionally substituted alkylheterocycle, optionally substituted heterocyclealkyl, optionally substituted alkylphosphate, optionally substituted phosphoalkyl, optionally substituted alkylphosphorothioate, optionally substituted phosphorothioalkyl, optionally substituted alkylphosphorodithioate, optionally substituted phosphorodithioalkyl, optionally substituted alkylphosphonate, optionally substituted phosphonoalkyl, optionally substituted amino, optionally substituted alkylamino, optionally substituted di(alkyl)amino, optionally substituted aminoalkyl, optionally substituted alkylaminoalkyl, optionally substituted di(alkyl)aminoalkyl, optionally substituted hydroxyalkyl, optionally substituted polyethylene glycol (PEG, mw 100-40K), optionally substituted mPEG (mw 120-40K), optionally substituted heteroaryl, optionally substituted heterocycle, or linker-ligand.

2. A method of item 1, wherein the formulation comprises 10-75% of cationic lipid of formula (I), (II), (III) or mixtures thereof, 0.5-50% of the neutral lipid, 5-60% of the sterol, and 0.1-20% of the PEG or PEG-modified lipid.

3. The method of item 1, wherein the nucleic acid-based agent is an RNA-based construct.

4. The method of item 1, wherein the nucleic acid-based agent is a double-stranded RNA (dsRNA).

5. The method of item 4, wherein the dsRNA targets a gene expressed in an immune cell.

6. The method of item: 1, wherein the immune cell is in the peritoneal cavity or bone marrow of a human.

7. The method of item 1, wherein the immune cell is a leukocyte.

8. The method of item 1, wherein the immune cell is a macrophage, dendritic cell, a monocyte, a neutrophil, a B cell, T cell, or natural killer (NK) cell.

9. The method of item 1, wherein the immune cell is a lymphocyte.

10. The method of item 1, wherein the delivery is performed *in vitro* or *in vivo.*

11. The method of item 1, wherein the nucleic acid-based agent is delivered to an immune cell of a subject by intravenous or intraperitoneal injection.

12. The method of item 1, wherein the nucleic acid-based agent has an average particle size of at least 100 nm.

13. A method of treating a subject having an autoimmune disorder, comprising administering to the subject a dsRNA complexed with a formulation comprising a sterol; a neutral lipid; a PEG or a PEG-modified lipid; and a lipid selected from the group consisting of :

(i) a cationic lipid having the structure of formula (I)

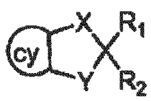 (I),

salts or isomers thereof, wherein:

cy is optionally substituted cyclic, optionally substituted heterocyclic or heterocycle, optionally substituted

aryl or optionally substituted heteroaryl;
$R_1$ and $R_2$ are each independently for each occurrence optionally substituted $C_{10}$-$C_{30}$ alkyl, optionally substituted $C_{10}$-$C_{30}$ alkenyl, optionally substituted $C_{10}$-$C_{30}$ alkynyl, optionally substituted $C_{10}$-$C_{30}$ acyl or -linker-ligand;
X and Y are each independently O or S, alkyl or N(Q); and
Q is H, alkyl, acyl, ω-aminoalkyl, ω-(substituted)aminoalky, ω-phosphoalkyl or ω-thiophosphoalkyl;

(ii) a cationic lipid having the structure of formula (II)

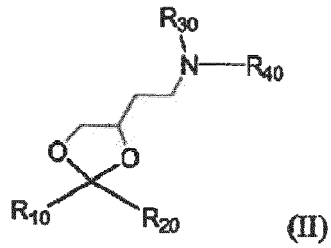
(II),

where $R_{10}$ and $R_{20}$ are independently alkyl, alkenyl or alkynyl, each can be optionally substituted, and $R_{30}$ and $R_{40}$ are independently lower alkyl or $R_{30}$ and $R_{40}$ can be taken together to form an optionally substituted heterocyclic ring;

(iii) a cationic lipid having the structure

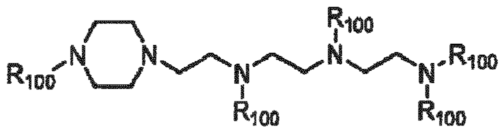
formula (III)

or

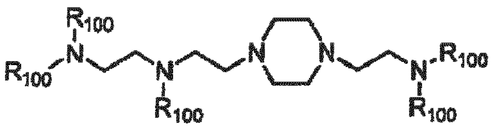
formula (IV),

wherein each R is independently H, alkyl,

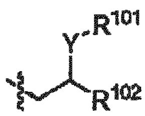, or 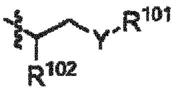;

provided that at least one R is

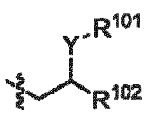, or 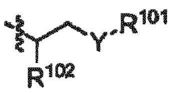;

wherein $R^{100}$, for each occurrence, is independently H, $R^{103}$,

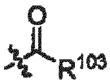, 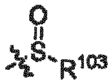, 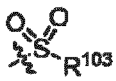, 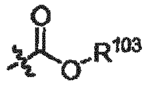,

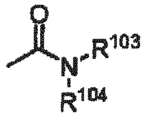, or 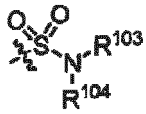;

wherein $R^{103}$ is optionally substituted with one or more substituent;
$R^{102}$, for each occurrence, is independently, alkyl, alkenyl, alkynyl, heteroalkyl, heteroalkenyl, or heteroalkynyl; each of which is optionally substituted with one or more substituent;
$R^{103}$, for each occurrence, is independently, alkyl, alkenyl, alkynyl, heteroalkyl, heteroalkenyl, or heteroalkynyl; each of which is optionally substituted with one or more substituent;
Y, for each occurrence, is independently O, $NR^{104}$, or S;
$R^{104}$, for each occurrence is independently H alkyl, alkenyl, alkynyl, heteroalkyl, heteroalkenyl, or heteroalkynyl; each of which is optionally substituted with one or more substituent; and

(iv) a cationic lipid having the structure

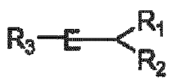 formula (V),

wherein $R_1$ and $R_2$ are each independently for each occurrence optionally substituted $C_{10}$-$C_{30}$ alkyl, optionally substituted $C_{10}$-$C_{30}$ alkoxy, optionally substituted $C_{10}$-$C_{30}$ alkenyl, optionally substituted $C_{10}$-$C_{30}$ alkenyloxy, optionally substituted $C_{10}$-$C_{30}$ alkynyl, optionally substituted $C_{10}$-$C_{30}$ alkynyloxy, or optionally substituted $C_{10}$-$C_{30}$ acyl;
E is -O-, -S-, -N(Q)-, -C(O)O-, -OC(O)-, -C(O)-, -N(Q)C(O)-, -C(O)N(Q)-, -N(Q)C(O)O-, -OC(O)N(Q)-, S(O), -N(Q)S(O)$_2$N(Q)-, -S(O)$_2$-, -N(Q)S(O)$_2$-, -SS-,-O-N=, =N-O-, -C(O)-N(Q)-N=, -N(Q)-N=, -N(Q)-O-, -C(O)S-, arylene, heteroarylene, cyclalkylene, or heterocyclylene; and
Q is H, alkyl, ω-aminoalkyl, ω-(substituted)aminoalkyl, ω-phosphoalkyl or ω-thiophosphoalkyl; and
$R_3$ is H, optionally substituted $C_1$-$C_{10}$ alkyl, optionally substituted $C_2$-$C_{10}$ alkenyl, optionally substituted $C_2$-$C_{10}$ alkynyl, optionally substituted alkylheterocycle, optionally substituted heterocyclealkyl, optionally substituted alkylphosphate, optionally substituted phosphoalkyl, optionally substituted alkylphosphorothioate, optionally substituted phosphorothioalkyl, optionally substituted alkylphosphorodithioate, optionally substituted phosphorodithioalkyl, optionally substituted alkylphosphonate, optionally substituted phosphonoalkyl, optionally substituted amino, optionally substituted alkylamino, optionally substituted di(alkyl)amino, optionally substituted aminoalkyl, optionally substituted alkylaminoalkyl, optionally substituted di(alkyl)aminoalkyl, optionally substituted hydroxyalkyl, optionally substituted polyethylene glycol (PEG, mw 100-40K), optionally substituted mPEG (mw 120-40K), optionally substituted heteroaryl, optionally substituted heterocycle, or linker-ligand.

14. The method of item 13, wherein the nucleic acid-based agent is an RNA-based construct.

15. The method of item 13, wherein the nucleic acid-based agent is a double-stranded RNA (dsRNA).

16. The method of item 13, wherein the subject has arthritis.

17. The method of item 13, wherein the dsRNA complexed with the formulation is administered by intravenous injection.

18. The method of item 12, wherein the dsRNA complexed with the formulation is administered by intraperitoneal injection.

19. A method of preparing a liposome, the methods comprising:

providing a mixture comprising a sterol, a neutral lipid, and a cationic lipid, wherein the mixture is substantially free of a PEG or PEG-modified lipid;
maintaining the mixture under conditions to allow the formation of liposomes, wherein the average diameter of the liposomes is at least 100 nm;
adding to said mixture a PEG or PEG-modified lipid; thereby forming the liposome.

20. The method of item 19, further comprising incorporating a nucleic acid into the liposome.

21. The method of item 19, the pH of the mixture is acidic.

22. The method of item 19, wherein the mixture comprises sodium.

23. The method of item 22, wherein the sodium concentration is about 10 mM.

24. The method of item 19, wherein the sterol is cholesterol.

25. The method of item 19, wherein the neutral lipid is DSPC.

26. The method of item 19, wherein the cationic lipid is selected from a lipid of any of formula I, II, III, or IV.

27. The method of item 19, wherein the cationic lipid is Lipid A.

28. The method of item 19, comprising adding to said mixture a PEG-modified lipid.

29. The method of item 19, wherein the PEG-modified lipid is selected from the group consisting of PEG-DSG, PEG-DMG, PEG-CerC14 or PEG-CerC18.

30. The method of item 19, wherein the average diameter of the liposomes is at least 150 nm.

31. The method of item 19, wherein the liposomes have a polydispersity index (PDI) of less than 0.4.

32. The method of item 30 wherein the the liposomes have a polydispersity index (PDI) of less than 0.4.

33. A product made by the method of item 19.

**Items**

1. A nucleic acid-based agent complexed with a formulation comprising a sterol; a neutral lipid; a PEG or a PEG-modified lipid; and a cationic lipid having the structure of formula (II)

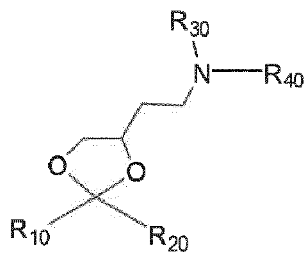

(II),

where $R_{10}$ and $R_{20}$ are independently alkyl, alkenyl or alkynyl, each can be optionally substituted, and $R_{30}$ and $R_{40}$ are independently lower alkyl or $R_{30}$ and $R_{40}$ can be taken together to form an optionally substituted heterocyclic ring; for use in delivering a nucleic acid-based agent to an immune cell.

2. The complexed nucleic acid-based agent for use of item 1, wherein the formulation comprises 10-75% of the cationic lipid of formula (II), 0.5-50% of the neutral lipid, 5-60% of the sterol, and 0.1-20% of the PEG or PEG-modified lipid.

3. The complexed nucleic acid-based agent for use of item 1, wherein the nucleic acid-based agent is an RNA-based construct.

4. The complexed nucleic acid-based agent for use of item 1, wherein the nucleic acid-based agent is a double-stranded RNA (dsRNA).

5. The complexed nucleic acid-based agent for use of item 4, wherein the dsRNA targets a gene expressed in an immune cell, selected from a leukocyte and a lymphocyte.

6. The complexed nucleic acid-based agent for use of item 1, wherein the delivery is performed *in vitro* or *in vivo.*

7. The complexed nucleic acid-based agent for use of item 4, which is for use in treating a subject having an autoimmune disorder.

8. The complexed nucleic acid-based agent for use of item 7, wherein the subject has arthritis.

## Claims

**1.** A nucleic acid-based agent complexed with a formulation comprising a sterol; a neutral lipid; a PEG or a PEG-modified lipid; and a cationic lipid selected from the group consisting of:

(iii) a cationic lipid having the structure

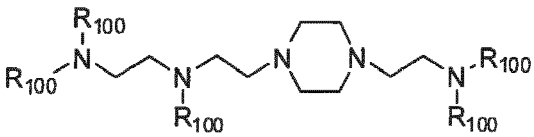

formula (IV),

wherein each R is independently H, alkyl,

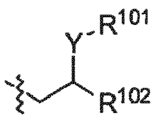

, or

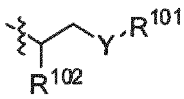

;

provided that at least one R is

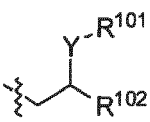

, or

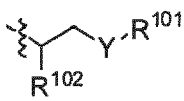

; wherein $R^{100}$,

for each occurrence, is independently H, $R^{103}$,

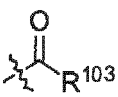

,

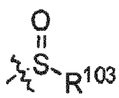

,

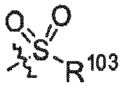, 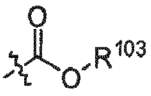, 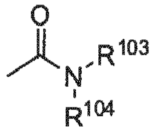, or 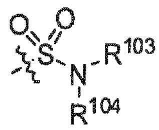 ;

wherein $R^{103}$ is optionally substituted with one or more substituent;
$R^{102}$, for each occurrence, is independently, alkyl, alkenyl, alkynyl, heteroalkyl, heteroalkenyl, or heteroalkynyl; each of which is optionally substituted with one or more substituent;
$R^{103}$, for each occurrence, is independently, alkyl, alkenyl, alkynyl, heteroalkyl, heteroalkenyl, or heteroalkynyl; each of which is optionally substituted with one or more substituent;
Y, for each occurrence, is independently O, $NR^{104}$, or S;
$R^{104}$, for each occurrence is independently H, alkyl, alkenyl, alkynyl, heteroalkyl, heteroalkenyl, or heteroalkynyl; each of which is optionally substituted with one or more substituent; and

(iv) a cationic lipid having the structure

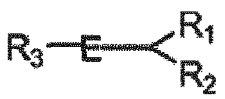 formula (V),

wherein $R_1$ and $R_2$ are each independently for each occurrence optionally substituted $C_{10}$-$C_{30}$ alkyl, optionally substituted $C_{10}$-$C_{30}$ alkoxy, optionally substituted $C_{10}$-$C_{30}$ alkenyl, optionally substituted $C_{10}$-$C_{30}$ alkenyloxy, optionally substituted $C_{10}$-$C_{30}$ alkynyl, optionally substituted $C_{10}$-$C_{30}$ alkynyloxy, or optionally substituted $C_{10}$-$C_{30}$ acyl;
E is -O-, -S-, -N(Q)-, -C(O)O-, -OC(O)-, -C(O)-, -N(Q)C(O)-, -C(O)N(Q)-, -N(Q)C(O)O-, -OC(O)N(Q)-, S(O), -N(Q)S(O)$_2$N(Q)-, -S(O)$_2$-, -N(Q)S(O)$_2$-, -SS-, -O-N=, =N-O-, -C(O)-N(Q)-N=, -N(Q)-N=, -N(Q)-O-, -C(O)S-, arylene, heteroarylene, cycloalkylene, or heterocyclylene; and
Q is H, alkyl, ω-aminoalkyl, ω-(substituted)aminoalkyl, ω-phosphoalkyl or ω-thiophosphoalkyl; and
$R_3$ is H, optionally substituted $C_1$-$C_{10}$ alkyl, optionally substituted $C_2$-$C_{10}$ alkenyl, optionally substituted $C_2$-$C_{10}$ alkynyl, optionally substituted alkylheterocycle, optionally substituted heterocyclealkyl, optionally substituted alkylphosphate, optionally substituted phosphoalkyl, optionally substituted alkylphosphorothioate, optionally substituted phosphorothioalkyl, optionally substituted alkylphosphorodithioate, optionally substituted phosphorodithioalkyl, optionally substituted alkylphosphonate, optionally substituted phosphonoalkyl, optionally substituted amino, optionally substituted alkylamino, optionally substituted di(alkyl)amino, optionally substituted aminoalkyl, optionally substituted alkylaminoalkyl, optionally substituted di(alkyl)aminoalkyl, optionally substituted hydroxyalkyl, optionally substituted polyethylene glycol (PEG, mw 100-40K), optionally substituted mPEG (mw 120-40K), optionally substituted heteroaryl, optionally substituted heterocycle, or linker-ligand;

for use in delivering a nucleic acid-based agent to an immune cell.

**2.** The complexed nucleic acid-based agent for use of claim 1, wherein the formulation comprises 10-75% of cationic lipid of formula (IV), (V), or mixtures thereof, 0.5-50% of the neutral lipid, 5-60% of the sterol, and 0.1-20% of the PEG or PEG-modified lipid.

**3.** The complexed nucleic acid-based agent for use of claim 1, wherein the nucleic acid-based agent is an RNA-based construct.

**4.** The complexed nucleic acid-based agent for use of claim 1, wherein the nucleic acid-based agent is a double-stranded RNA (dsRNA).

**5.** The complexed nucleic acid-based agent for use of claim 4, wherein the dsRNA targets a gene expressed in an immune cell.

**6.** The complexed nucleic acid-based agent for use of claim 5, wherein the immune cell is in the peritoneal cavity or

bone marrow of a human.

**7.** The complexed nucleic acid-based agent for use of claim 5, wherein the immune cell is a leukocyte, a macrophage, dendritic cell, a monocyte, a neutrophil, a B cell, T cell, natural killer (NK) cell, or a lymphocyte.

**8.** The complexed nucleic acid-based agent for use of claim 1, wherein the delivery is performed *in vivo.*

**9.** The complexed nucleic acid-based agent for use of claim 1, wherein the nucleic acid-based agent is delivered to an immune cell of a subject by intravenous or intraperitoneal injection.

**10.** The complexed nucleic acid-based agent for use of claim 1, wherein the nucleic acid-based agent has an average particle size of at least 100 nm.

**11.** The complexed nucleic acid-based agent for use of claim 4, which is for use in a method of treating a subject having an autoimmune disorder.

**12.** The complexed nucleic acid-based agent for use of claim 11, wherein the subject has arthritis.

**13.** The complexed nucleic acid-based agent for use of claim 11, wherein the dsRNA complexed with the formulation is administered by intravenous injection or by intraperitoneal injection.

FIG. 1A

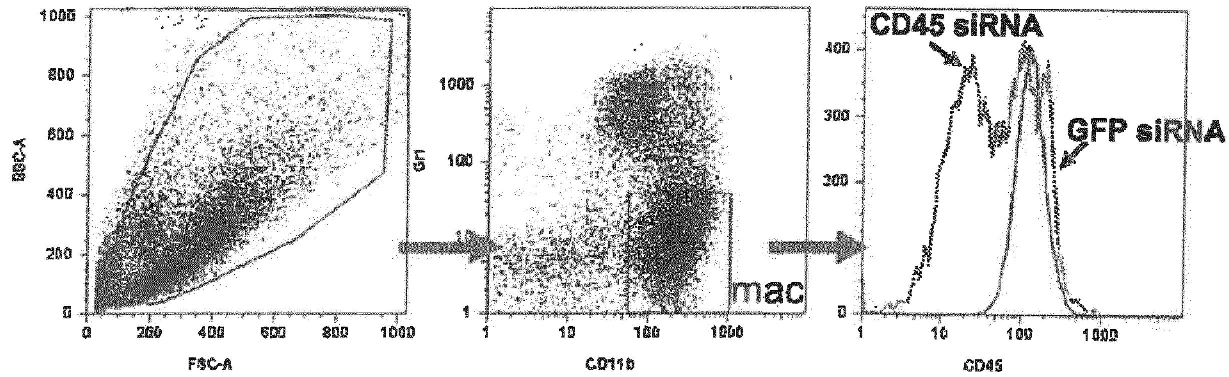
SSC-A
FSC-A
Gr1
CD11b
mac
CD45 siRNA
GFP siRNA
CD45

FIG. 1B

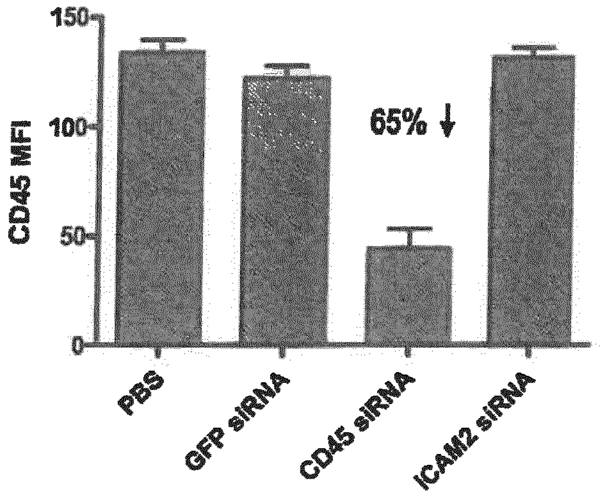
CD45 MFI
65% ↓
PBS
GFP siRNA
CD45 siRNA
ICAM2 siRNA

FIG. 2

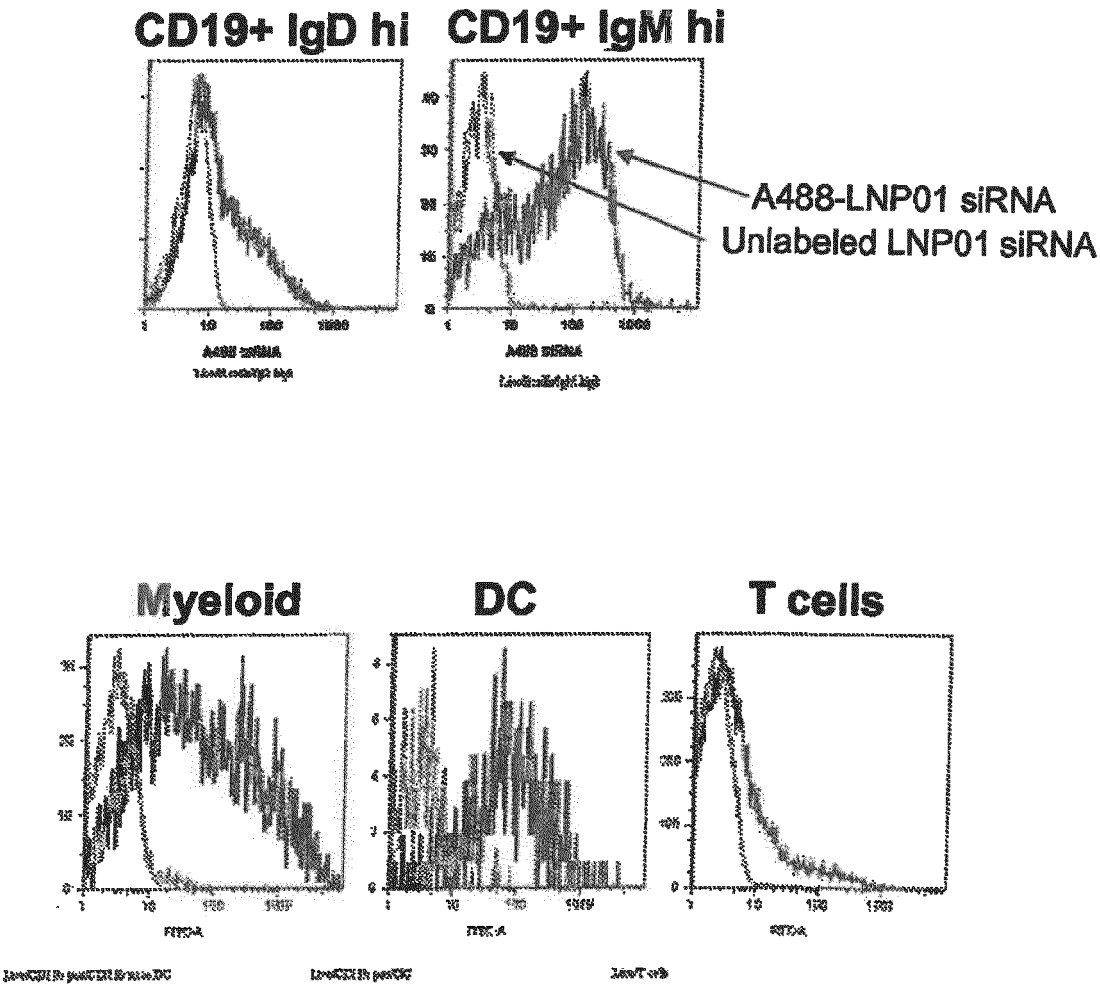
CD19+ IgD hi
CD19+ IgM hi
A488-LNP01 siRNA
Unlabeled LNP01 siRNA
Myeloid
DC
T cells

FIG. 3A

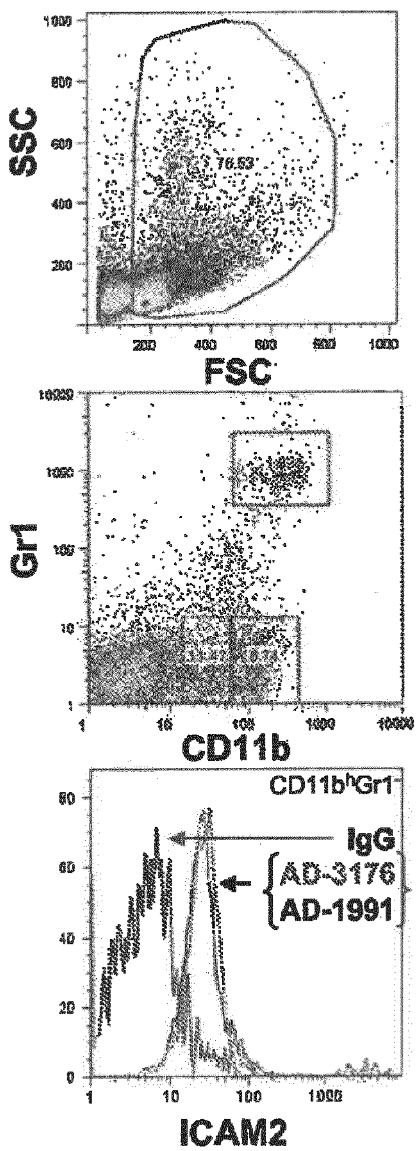
SSC
FSC
Gr1
CD11b
CD11b$^{h}$Gr1$^{-}$
IgG
AD-3176
AD-1991
ICAM2

FIG. 3B

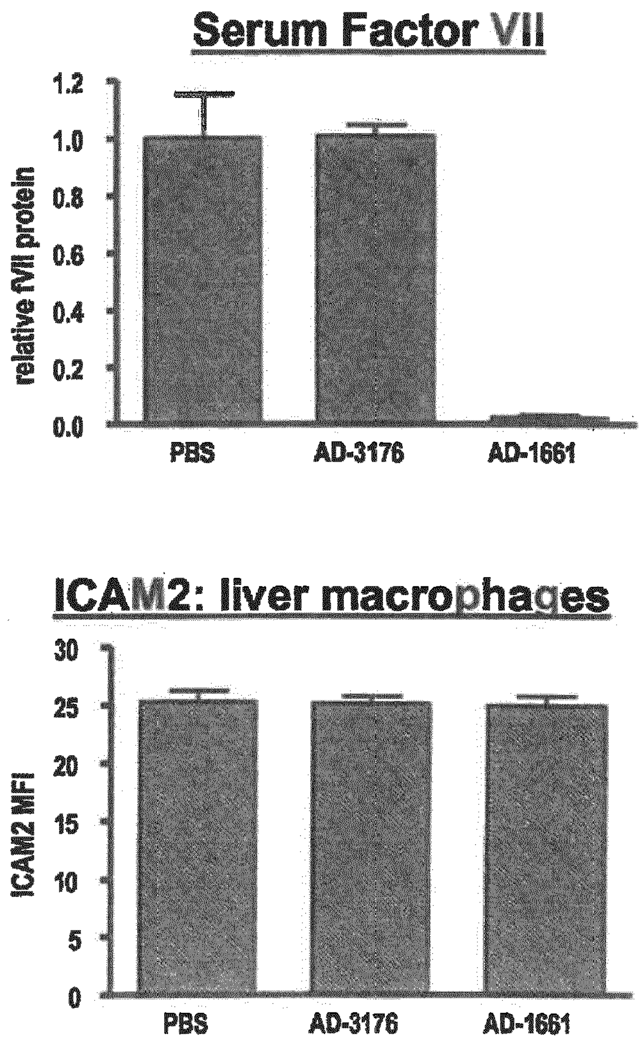
Serum Factor VII
relative fVII protein
1.2
1.0
0.8
0.6
0.4
0.2
0.0
PBS
AD-3176
AD-1661
ICAM2: liver macrophages
ICAM2 MFI
30
25
20
15
10
5
0
PBS
AD-3176
AD-1661

FIG. 4A

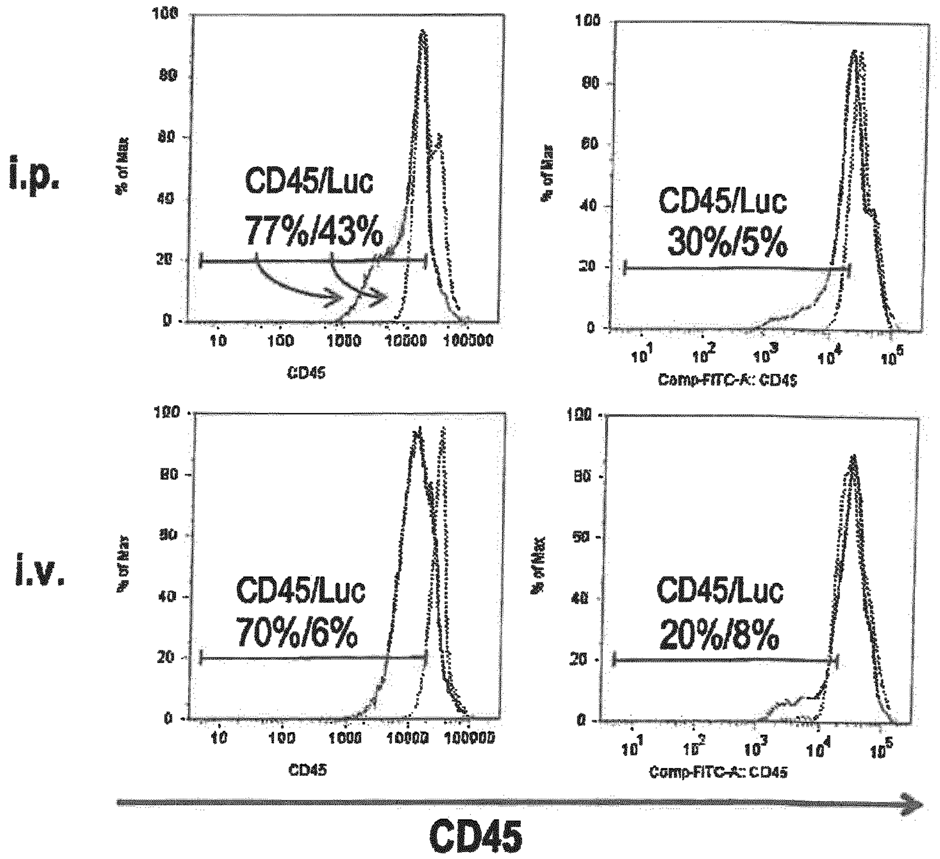
i.p.
CD45/Luc
77%/43%
CD45/Luc
30%/5%
i.v.
CD45/Luc
70%/6%
CD45/Luc
20%/8%
% of Max
CD45
Comp-FITC-A: CD45
CD45

FIG. 4B

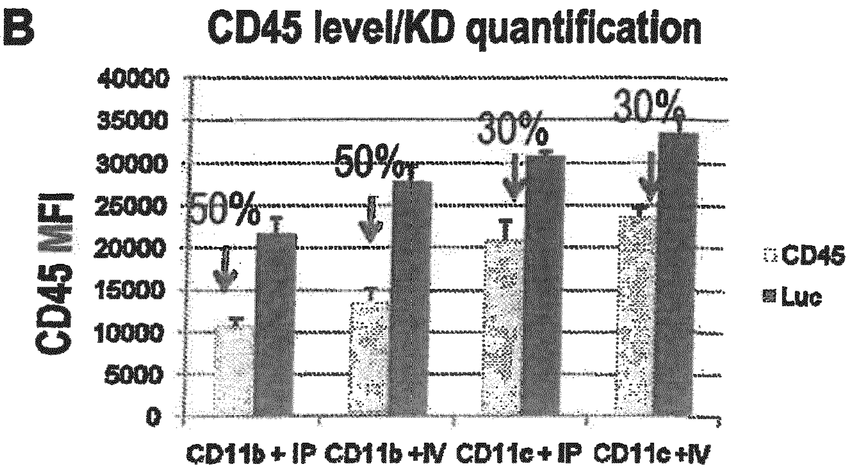
CD45 level/KD quantification
CD45 MFI
40000
35000
30000
25000
20000
15000
10000
5000
0
50%
50%
30%
30%
CD45
Luc
CD11b + IP
CD11b +IV
CD11c + IP
CD11c +IV

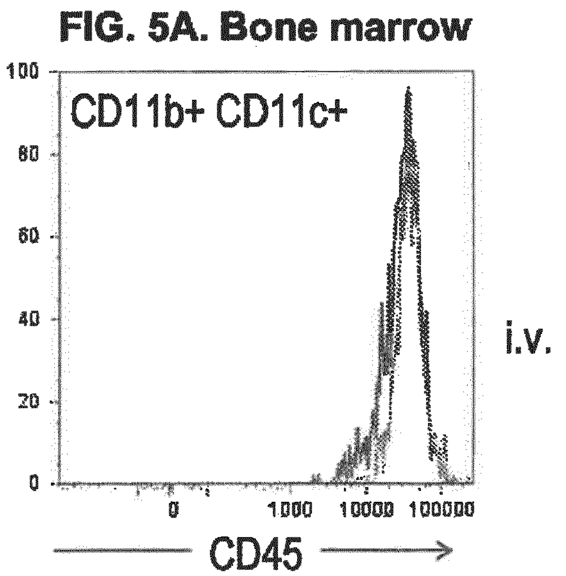
FIG. 5A. Bone marrow
CD11b+ CD11c+
100
80
60
40
20
0
0
1000
10000
100000
CD45
i.v.

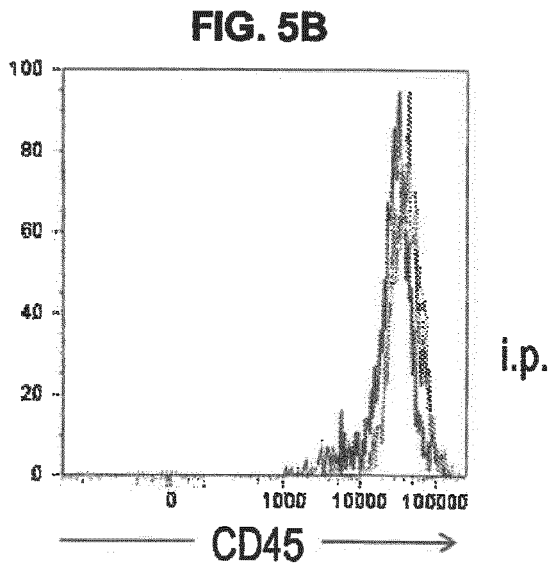
FIG. 5B
100
80
60
40
20
0
0
1000
10000
100000
CD45
i.p.

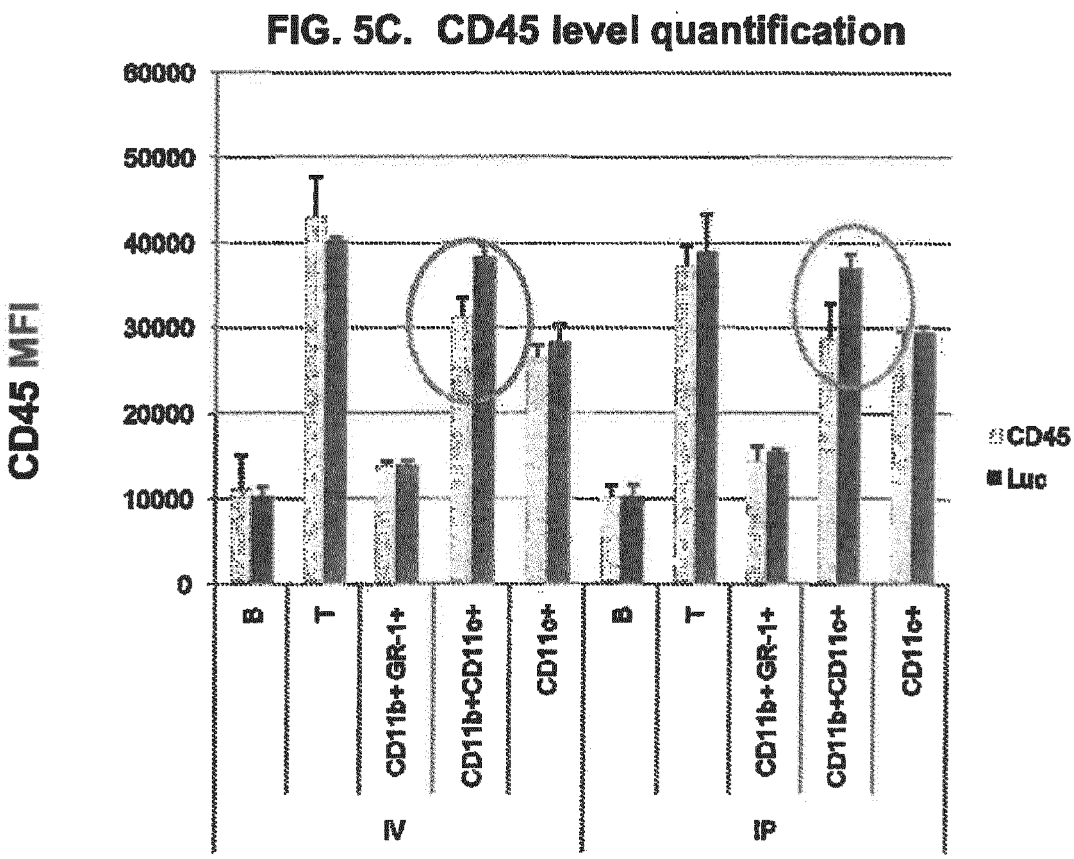
FIG. 5C. CD45 level quantification
CD45 MFI
60000
50000
40000
30000
20000
10000
0
B
T
CD11b+GR-1+
CD11b+CD11c+
CD11c+
IV
IP
CD45
Luc

FIG. 6

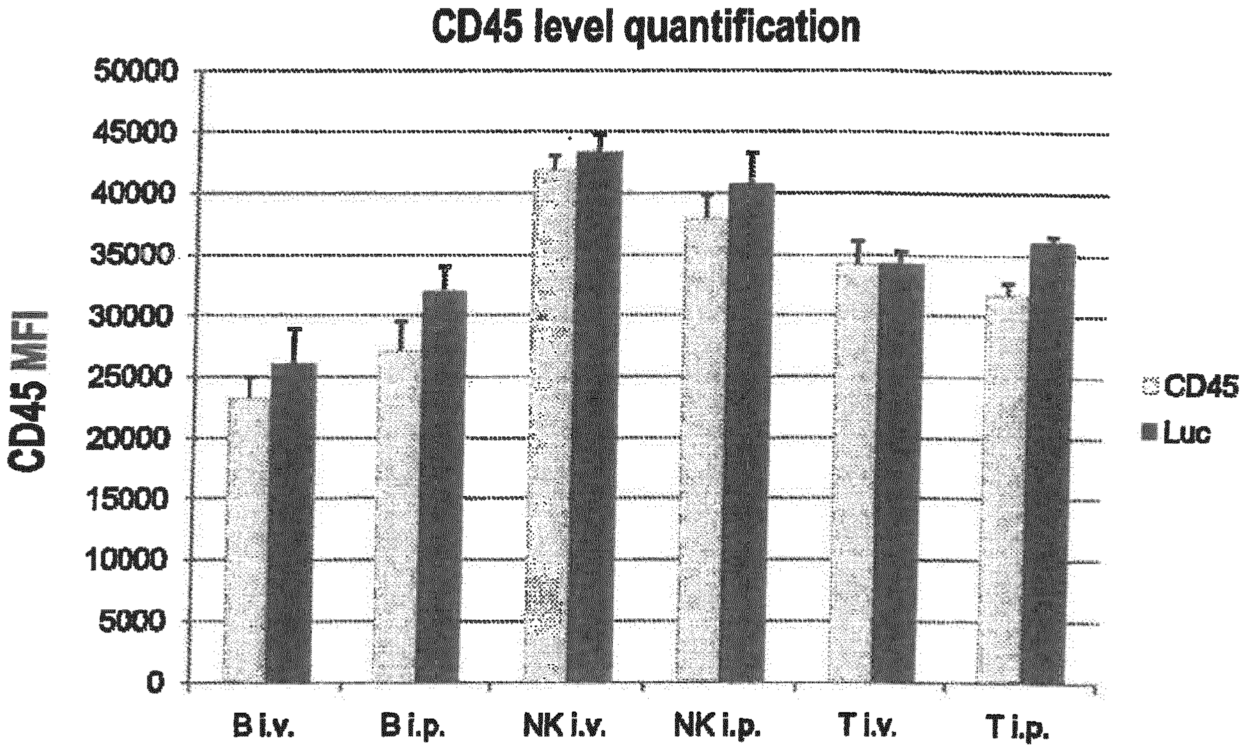
CD45 level quantification
CD45 MFI
50000
45000
40000
35000
30000
25000
20000
15000
10000
5000
0
B i.v.
B i.p.
NK i.v.
NK i.p.
T i.v.
T i.p.
CD45
Luc

FIG. 7A

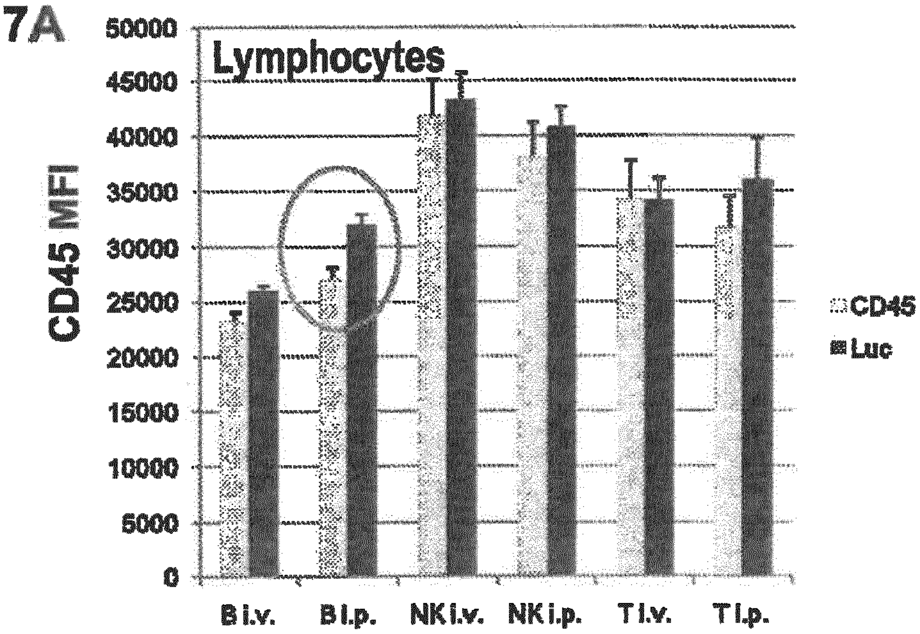
Lymphocytes
CD45 MFI
50000
45000
40000
35000
30000
25000
20000
15000
10000
5000
0
B i.v.
B i.p.
NK i.v.
NK i.p.
T i.v.
T i.p.
CD45
Luc

FIG. 7B

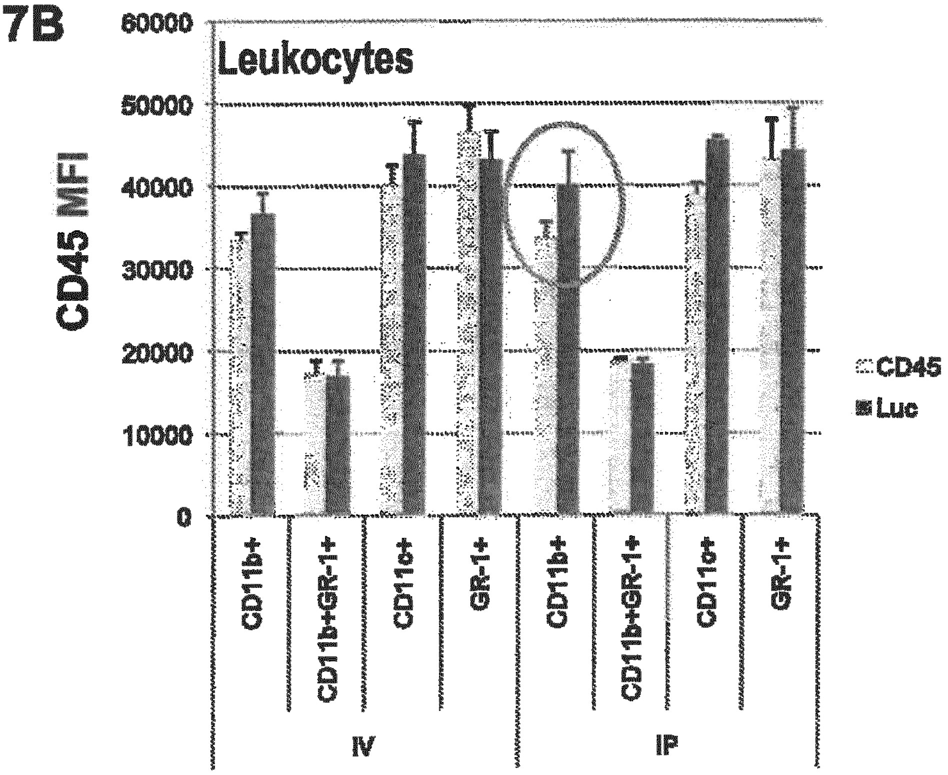
Leukocytes
CD45 MFI
60000
50000
40000
30000
20000
10000
0
CD11b+
CD11b+GR-1+
CD11c+
GR-1+
CD11b+
CD11b+GR-1+
CD11c+
GR-1+
IV
IP
CD45
Luc

FIG. 8A. Peyer's Patches

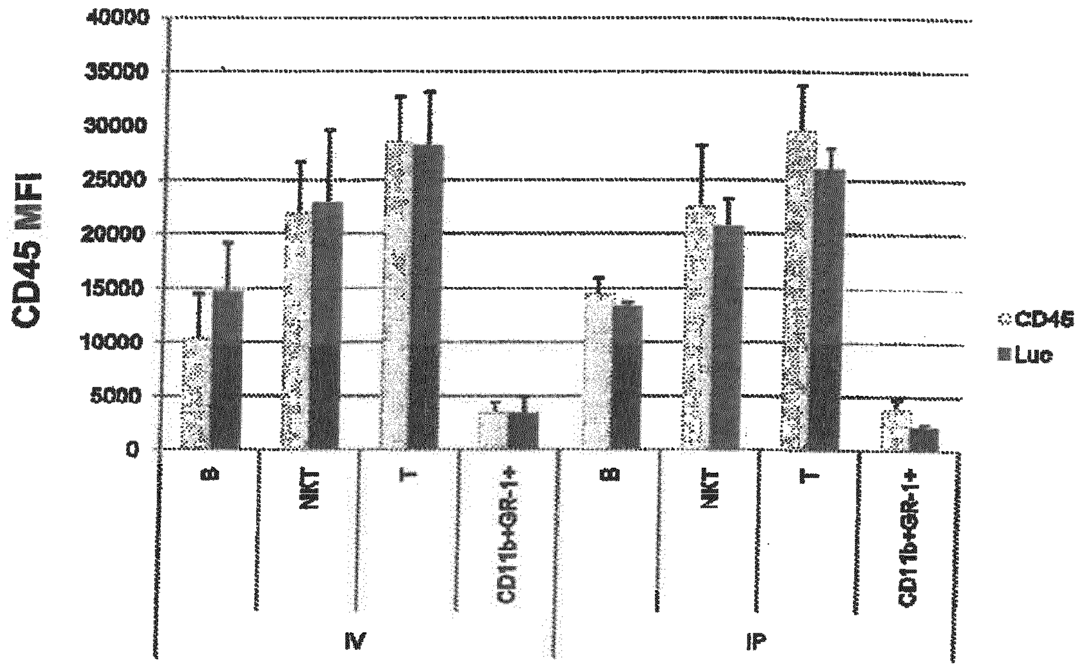


FIG. 8B. Liver

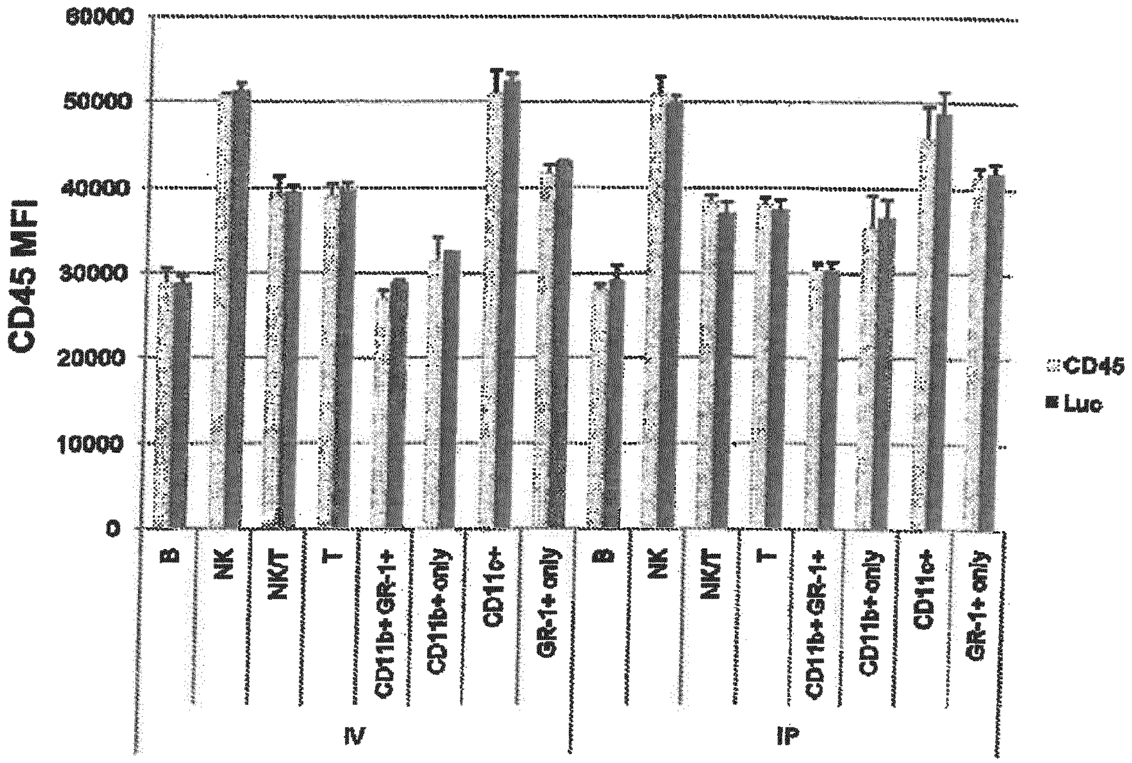

FIG. 9

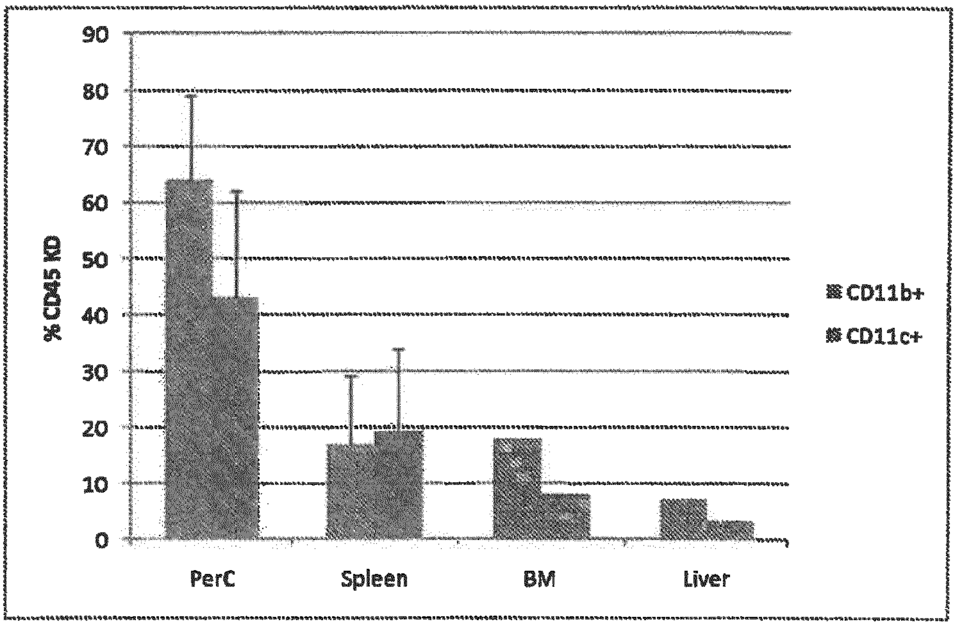
90
80
70
60
50
40
30
20
10
0
% CD45 KD
CD11b+
CD11c+
PerC
Spleen
BM
Liver

FIG. 10A. Peritoneal cavity

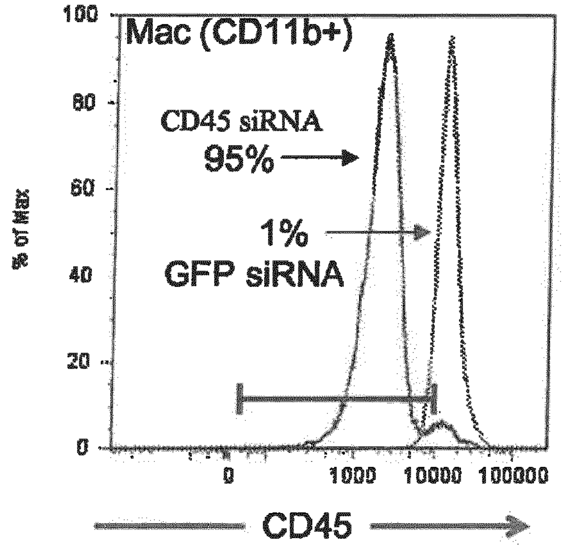


FIG. 10B

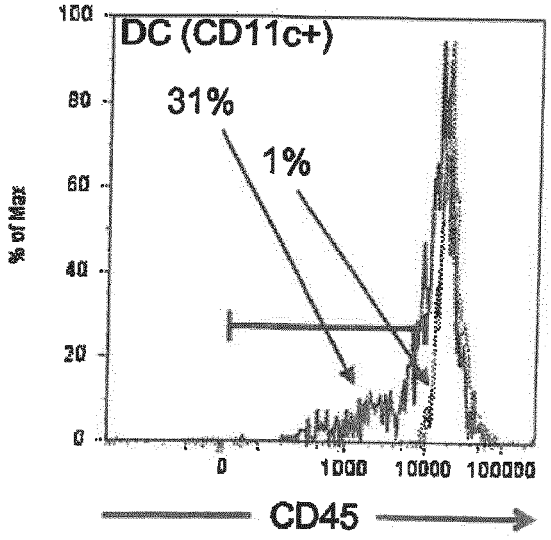


FIG. 10C

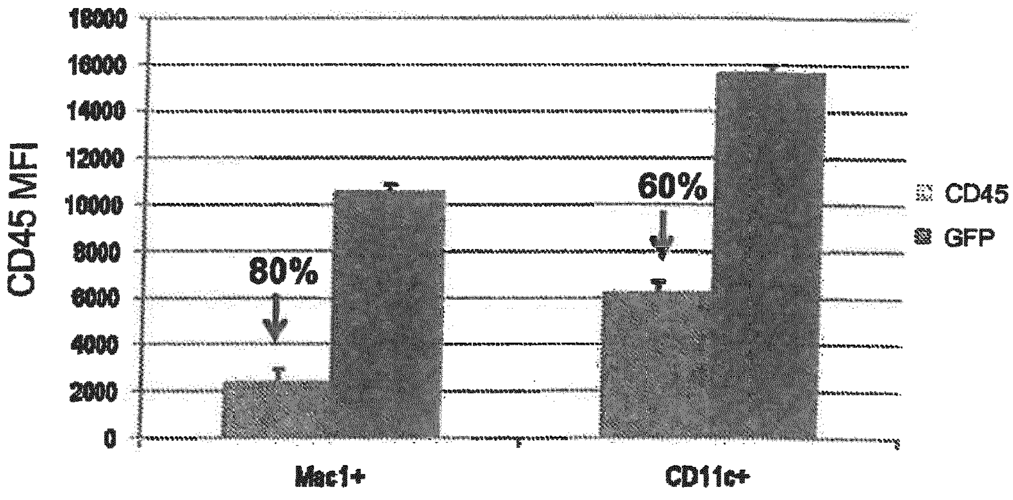

FIG. 11A. Peritoneal cavity

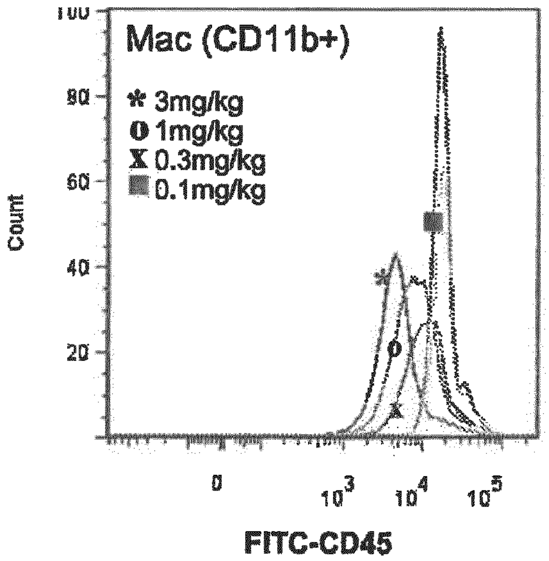


FIG. 11B

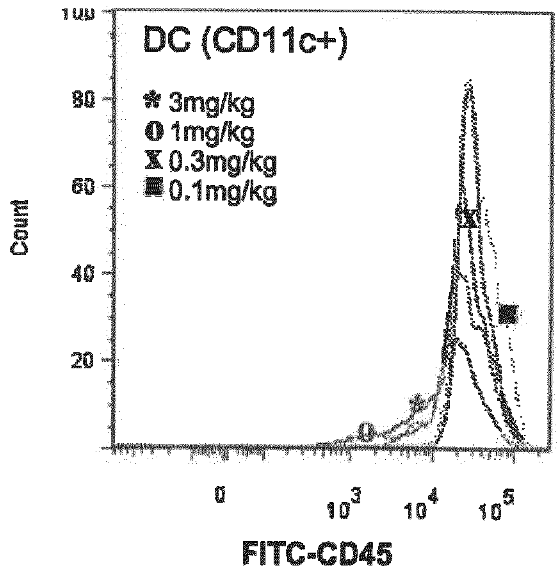


FIG. 11C

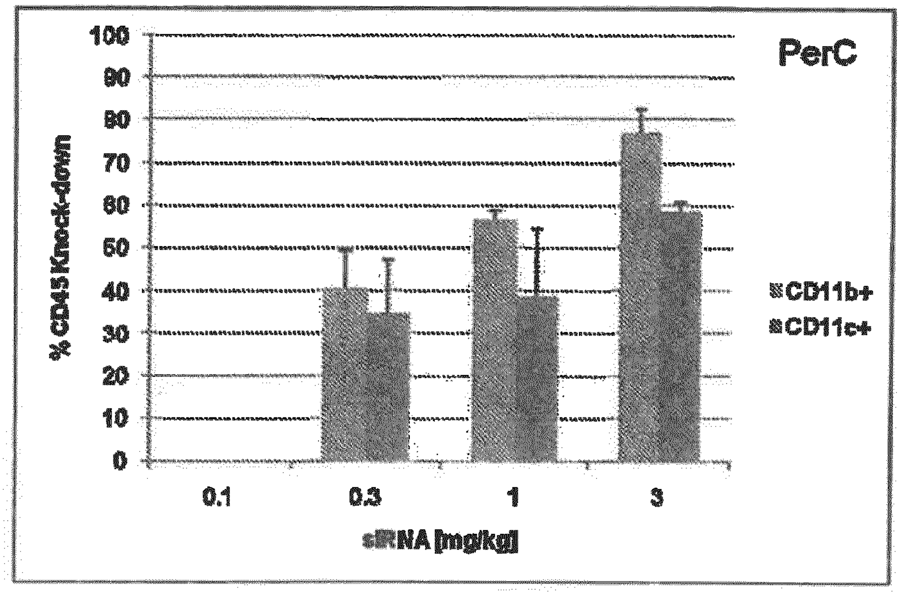

FIG. 12

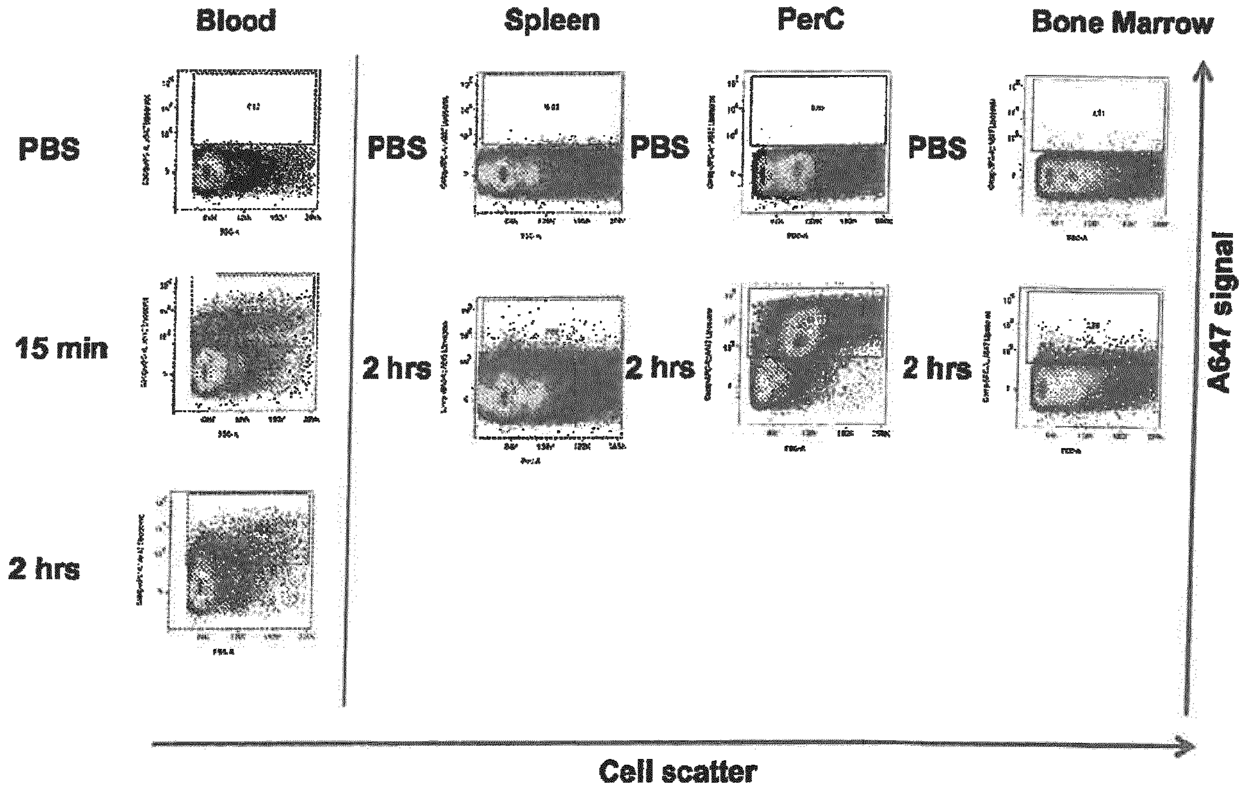
Blood
Spleen
PerC
Bone Marrow
PBS
15 min
2 hrs
PBS
2 hrs
PBS
2 hrs
PBS
2 hrs
A647 signal
Cell scatter

FIG. 13

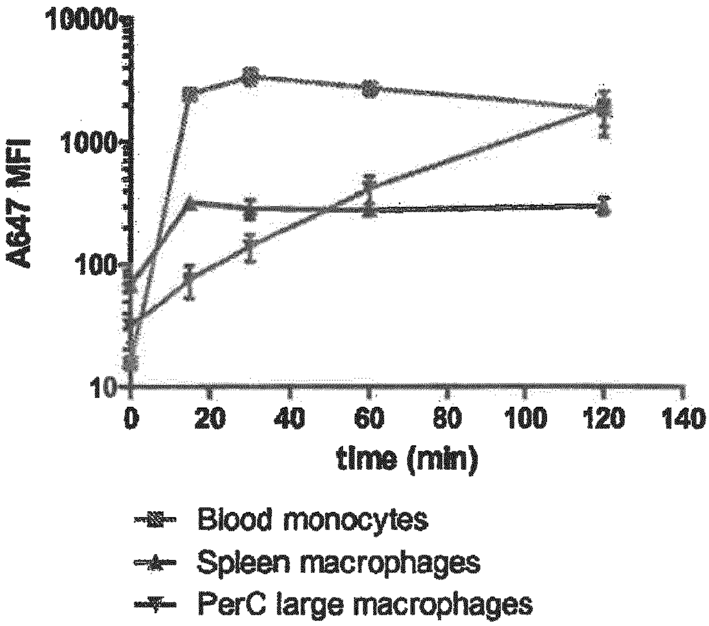
10000
1000
100
10
A647 MFI
0
20
40
60
80
100
120
140
time (min)
Blood monocytes
Spleen macrophages
PerC large macrophages

FIG. 14A. Bone Marrow

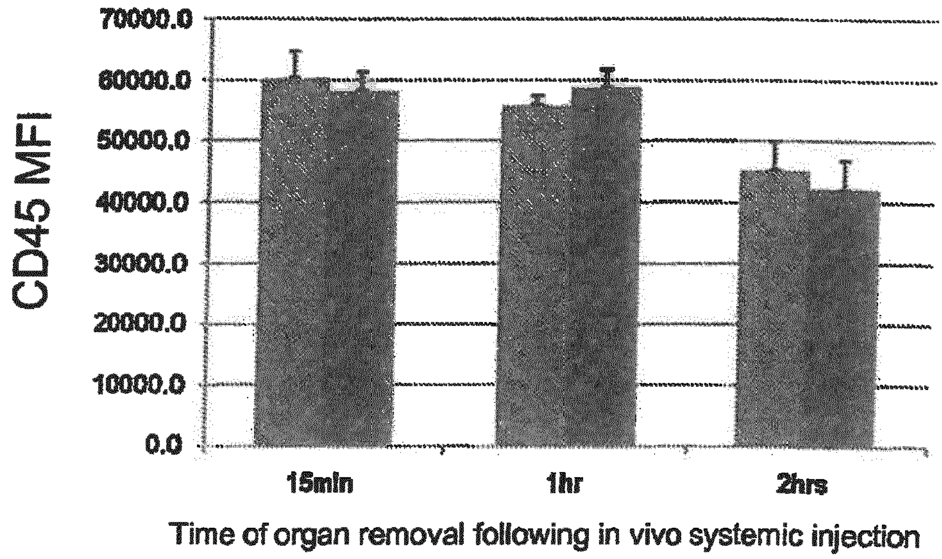


FIG. 14B. Spleen

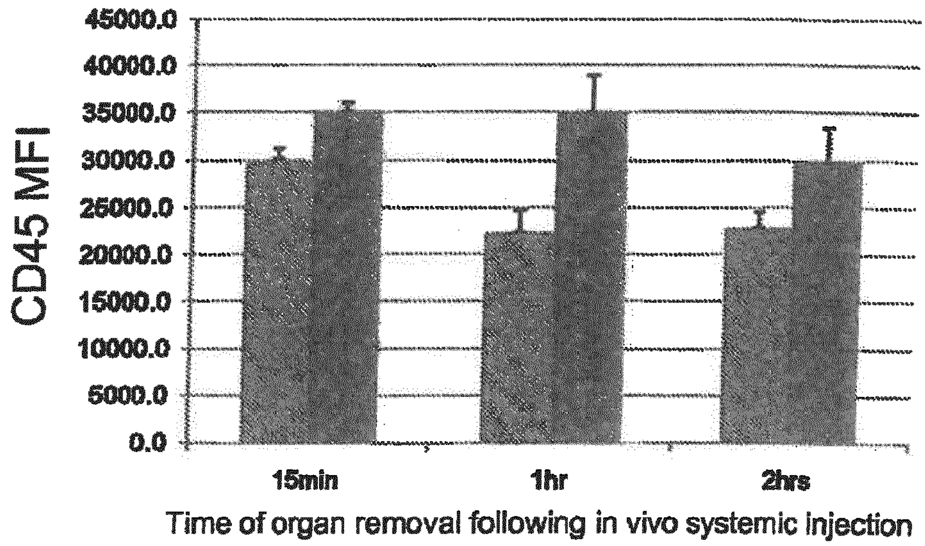

FIG. 14C. Blood

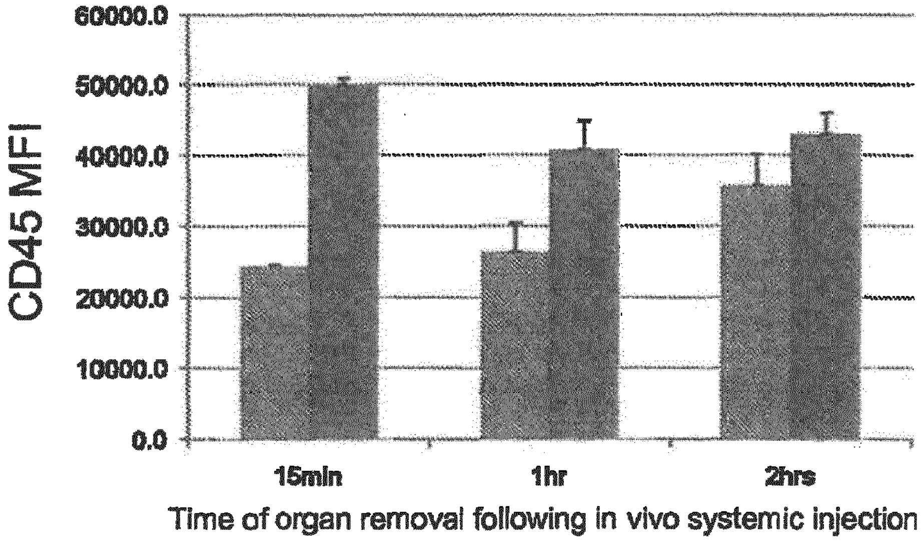


FIG. 14D. Peritoneal Cavity

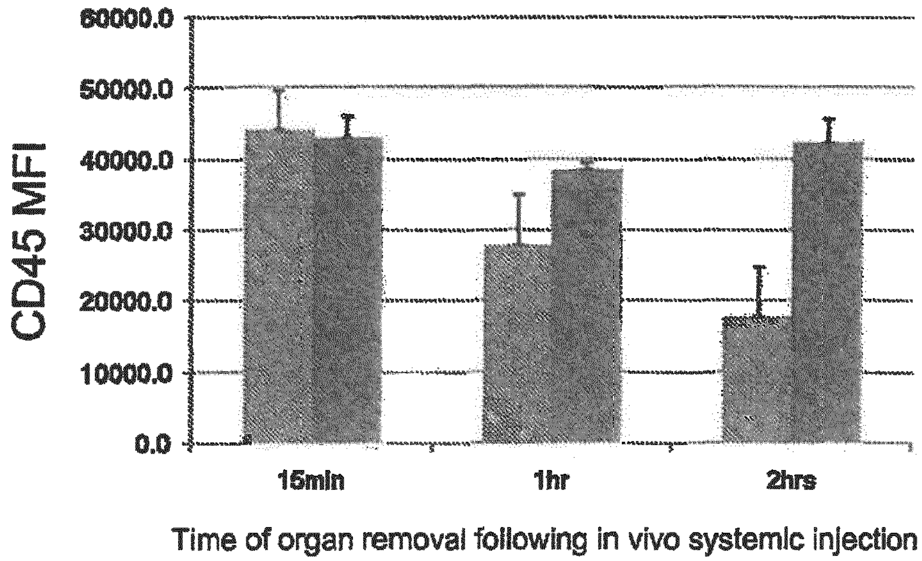

FIG. 15A

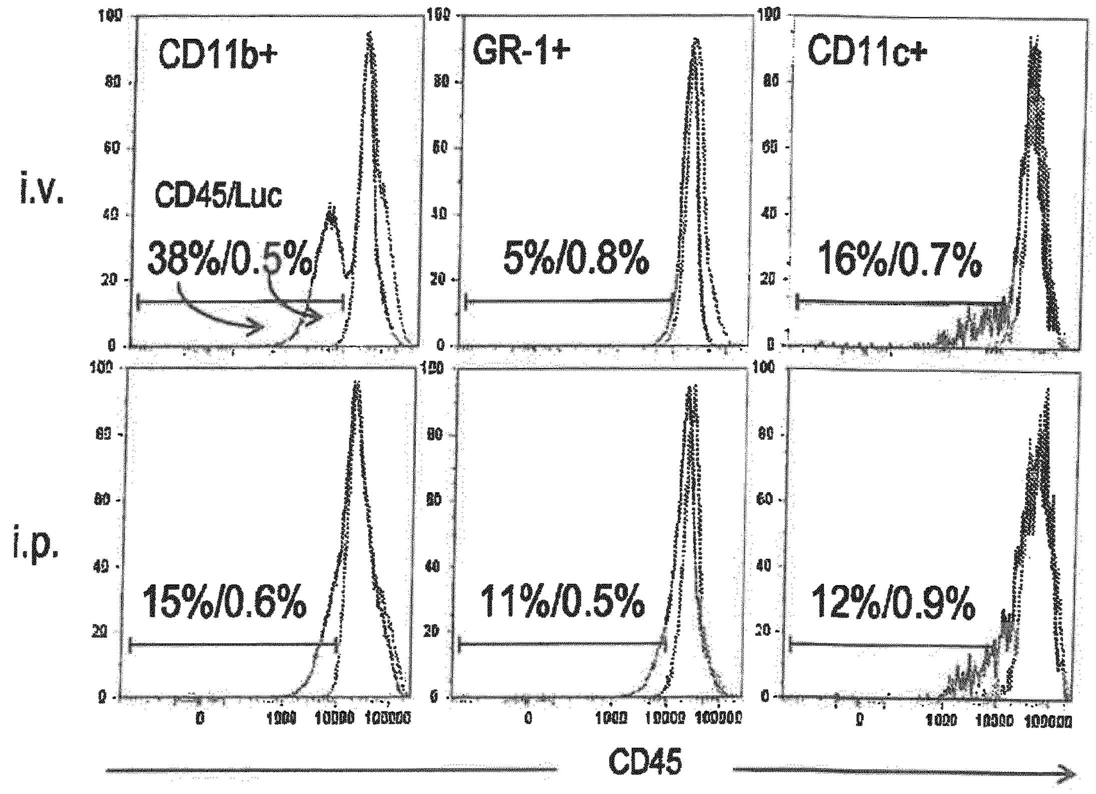


FIG. 15B

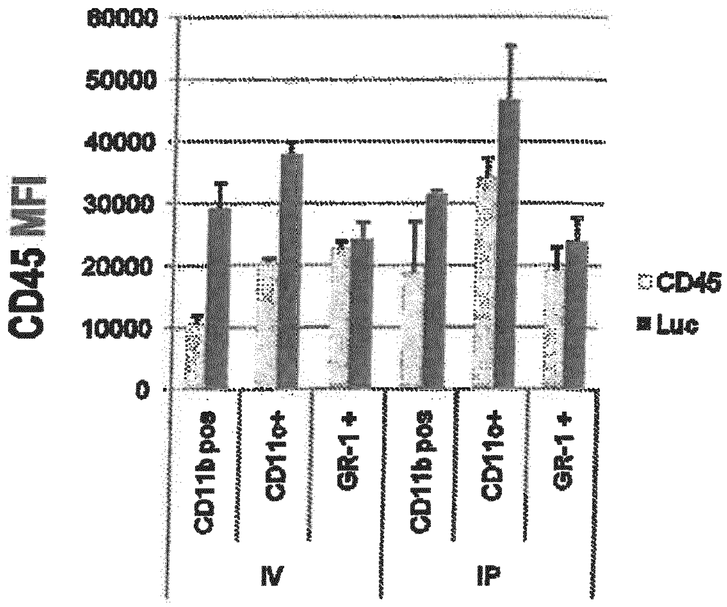

FIG. 16. Lymphocytes

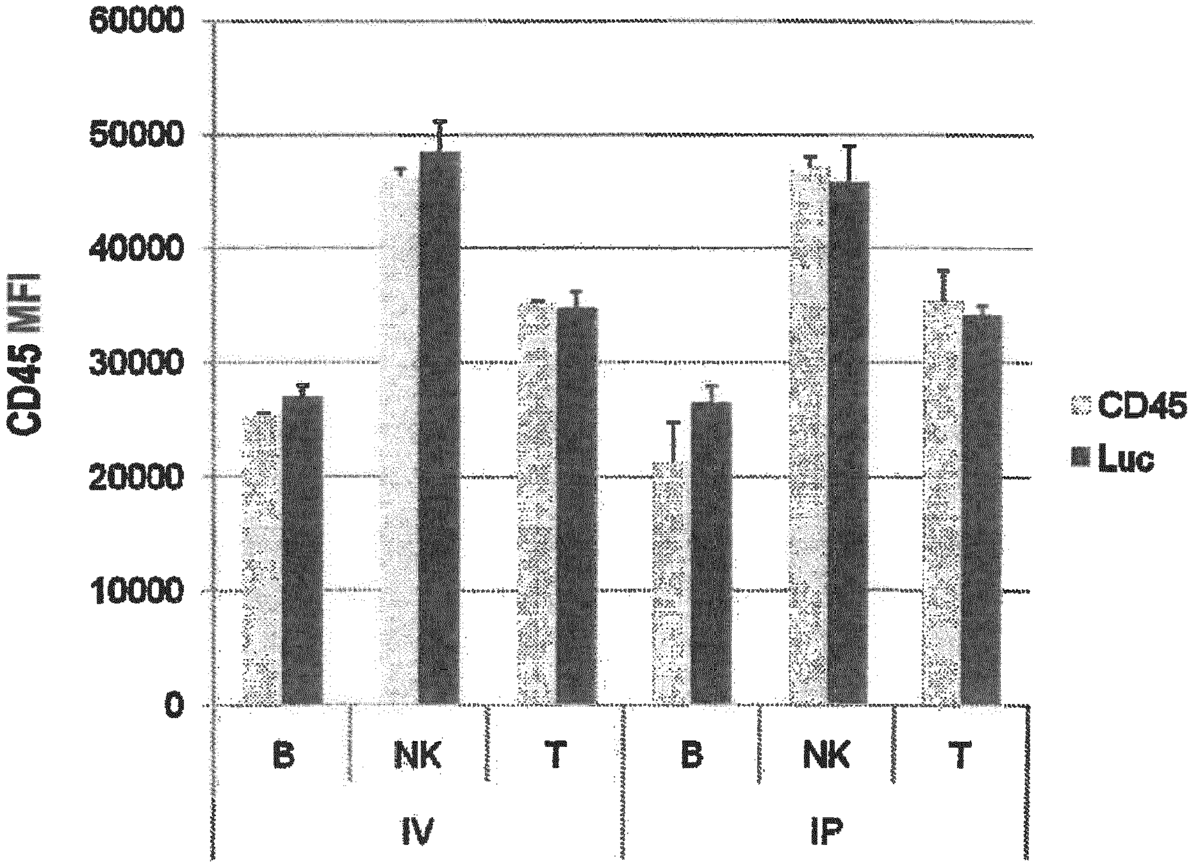

FIG. 17A

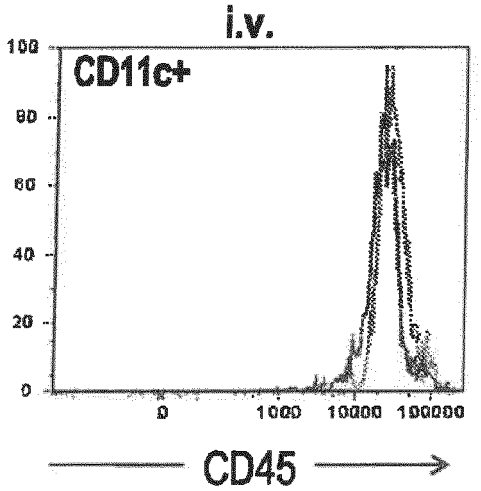


FIG. 17B

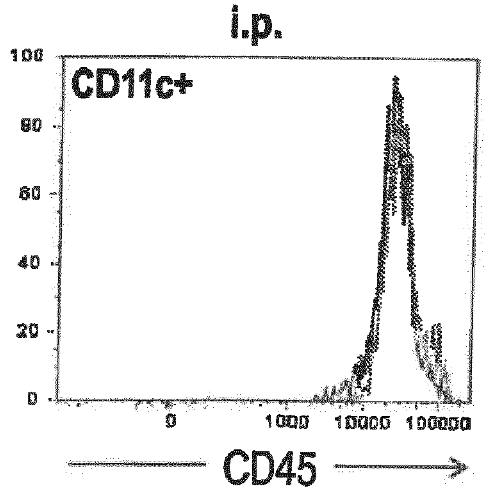


FIG. 17C

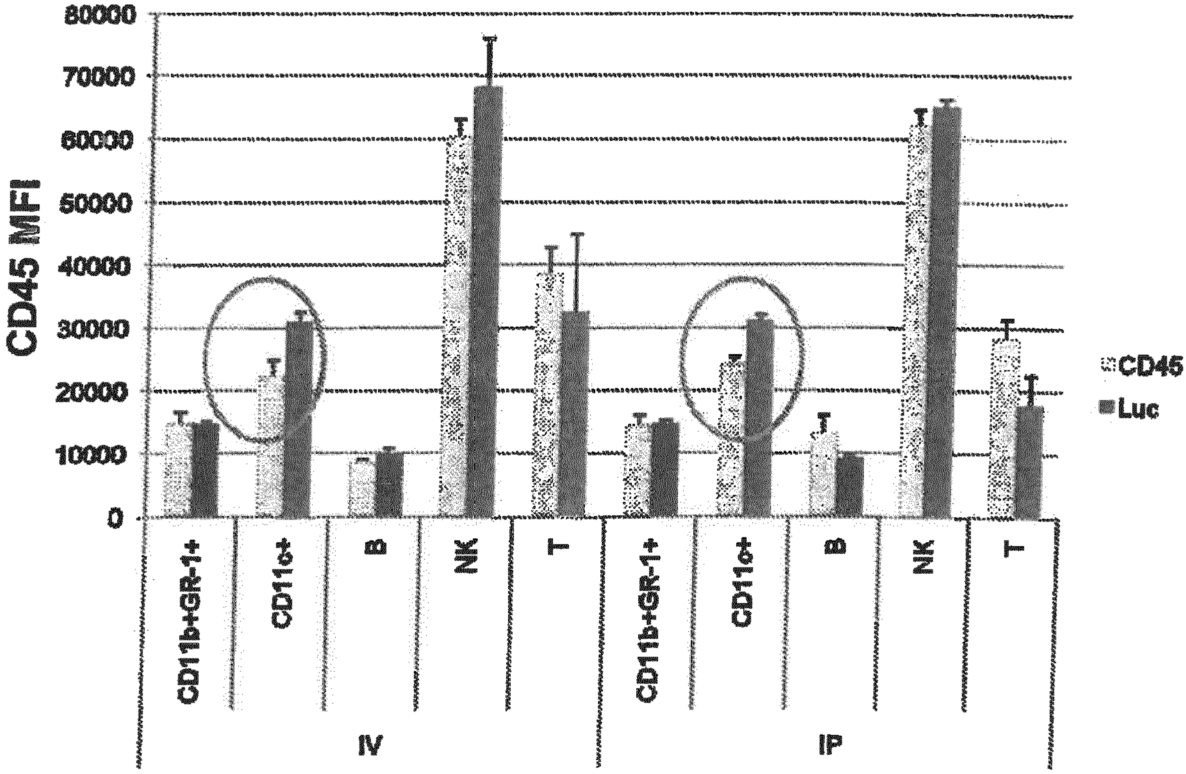

FIG. 18A. Liver

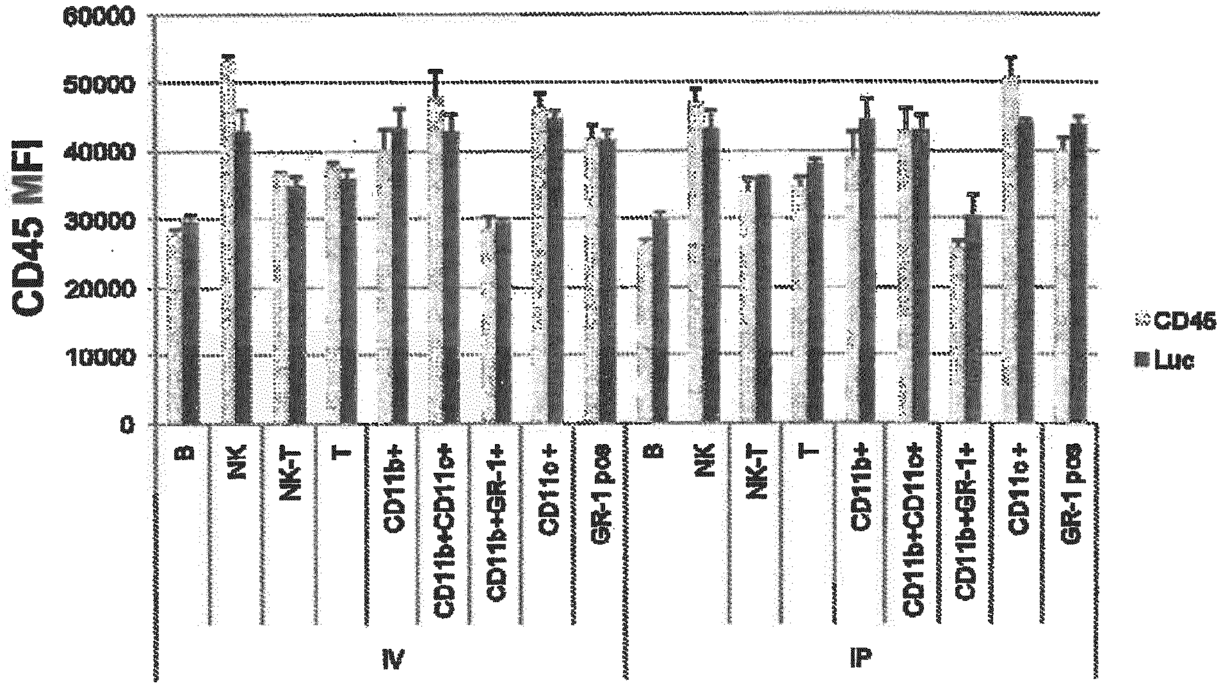


FIG. 18B. Spleen

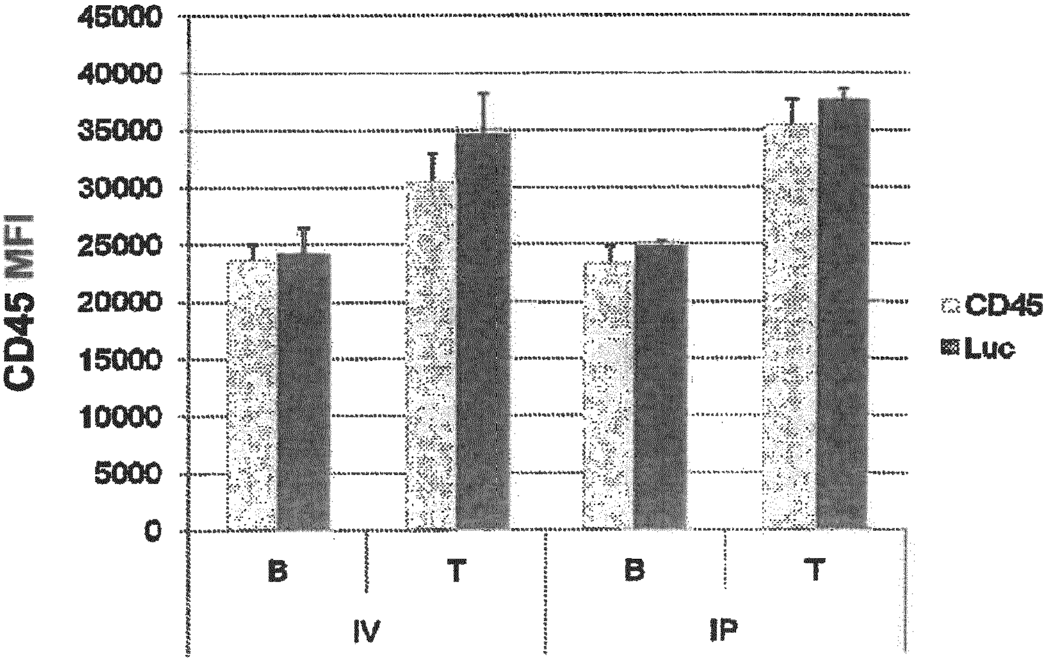

FIG. 18C. Peyer's Patch Lymphocytes

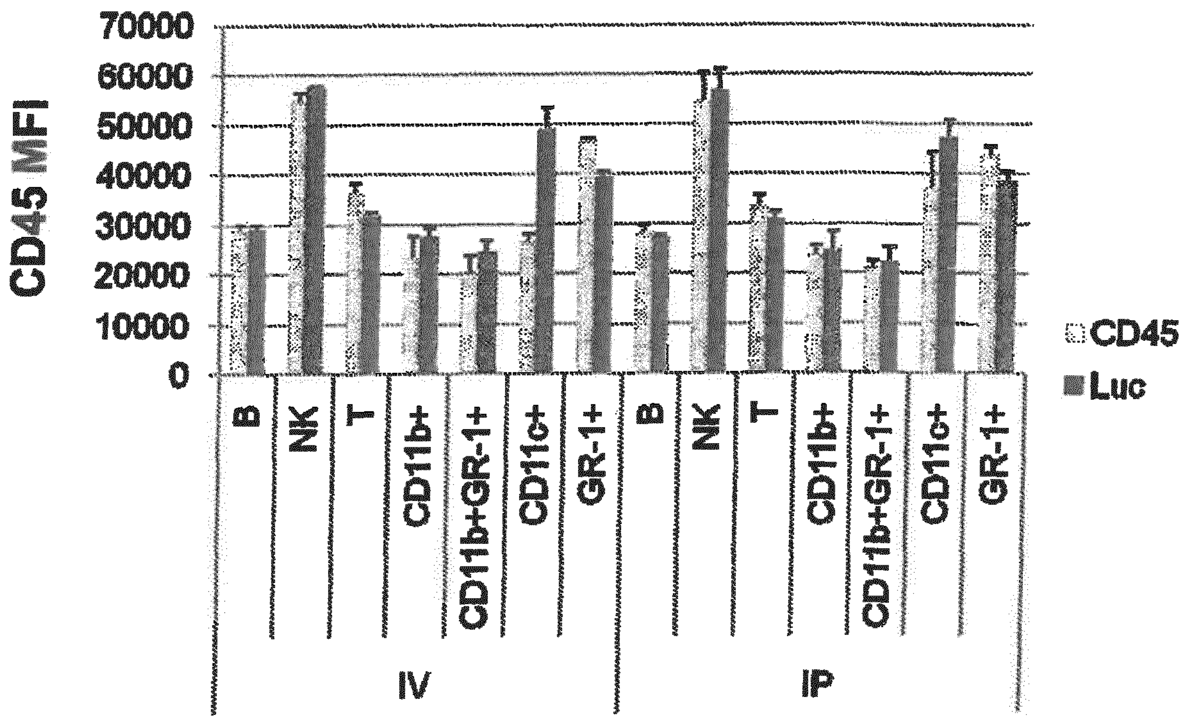

FIG. 19A. Peritoneal Cavity

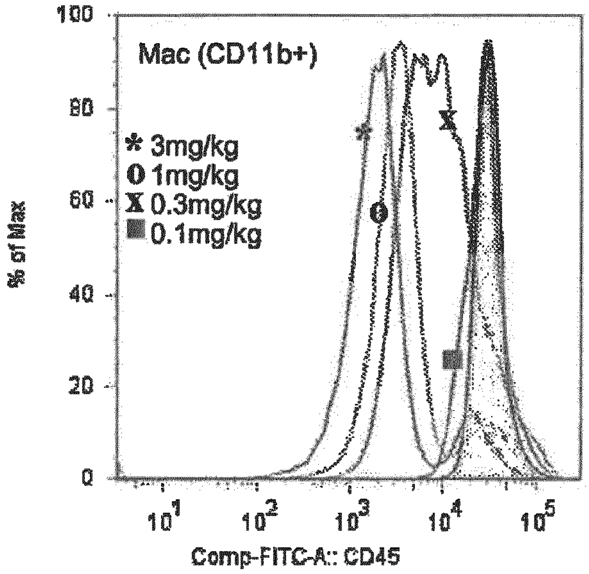


FIG. 19B. Peritoneal Cavity

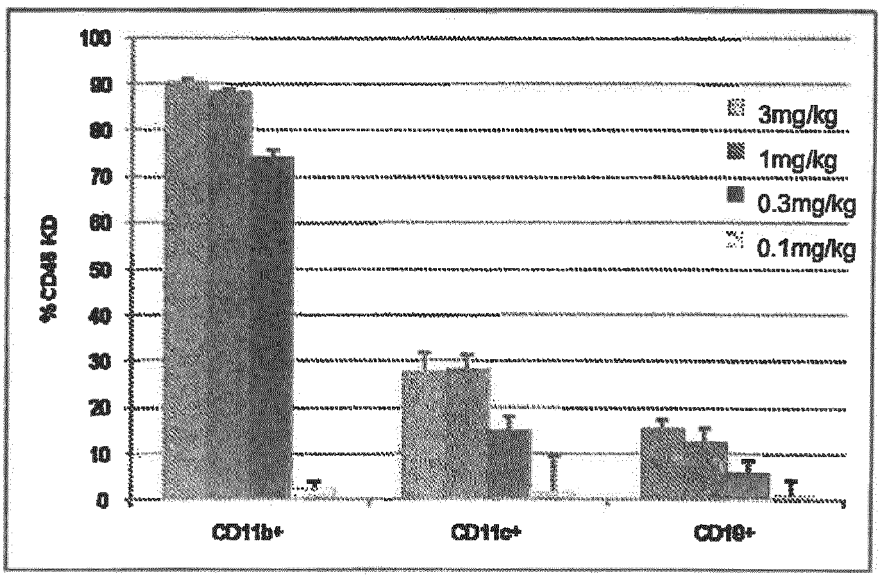

FIG. 20A. Spleen

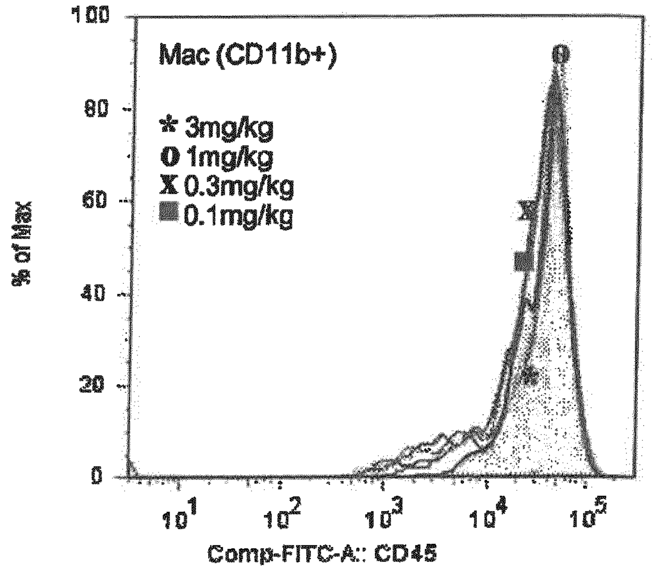


FIG. 20B. Spleen

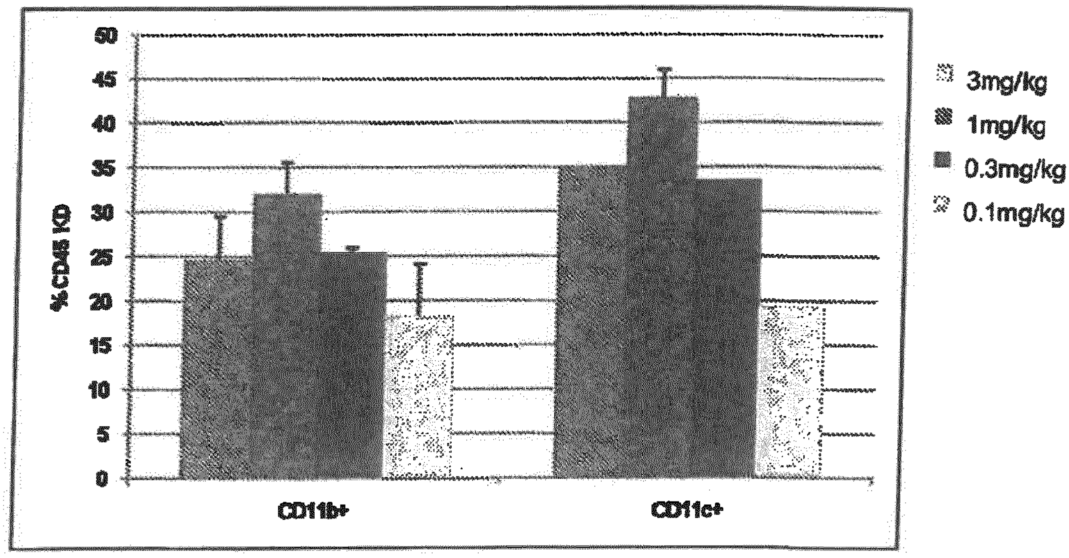

FIG. 21A

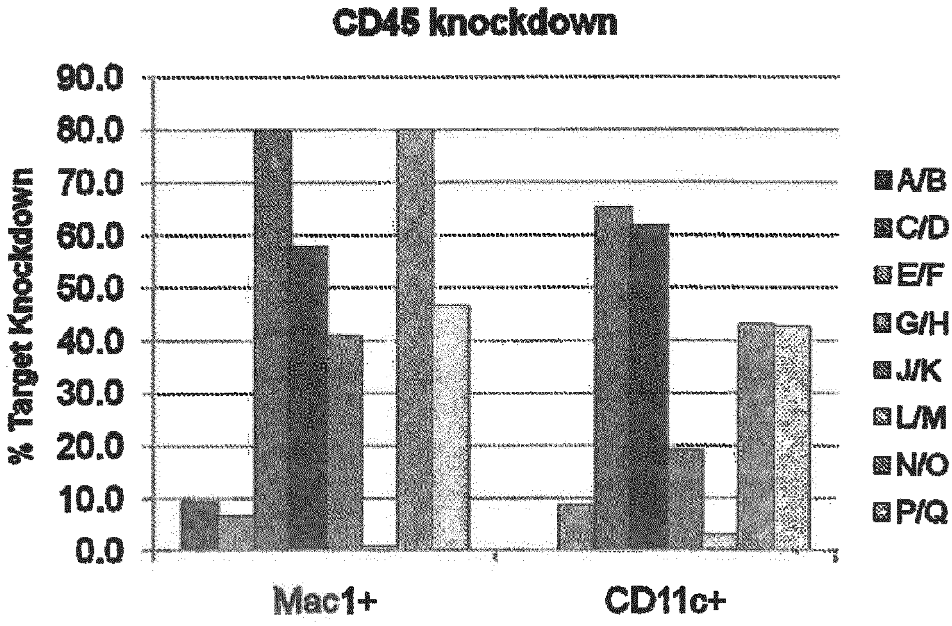


FIG. 21B

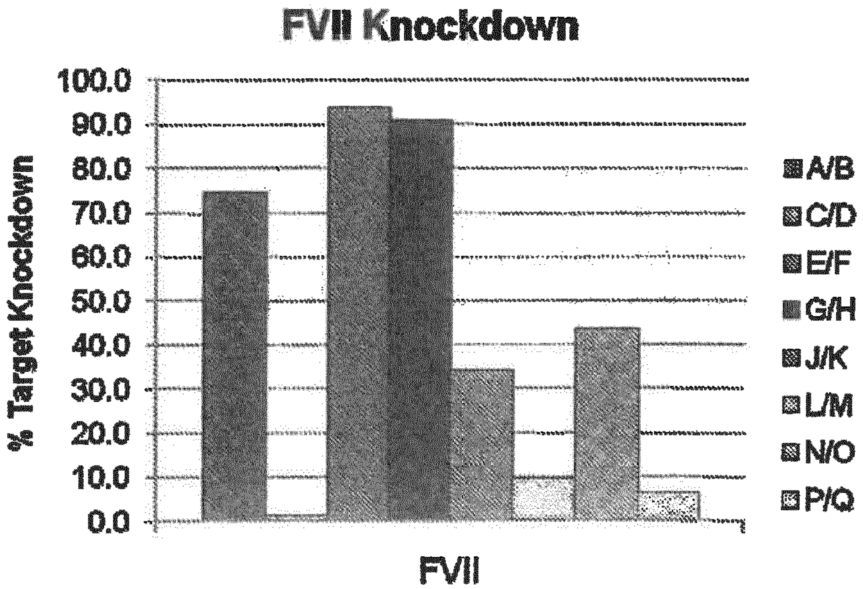

FIG. 22A

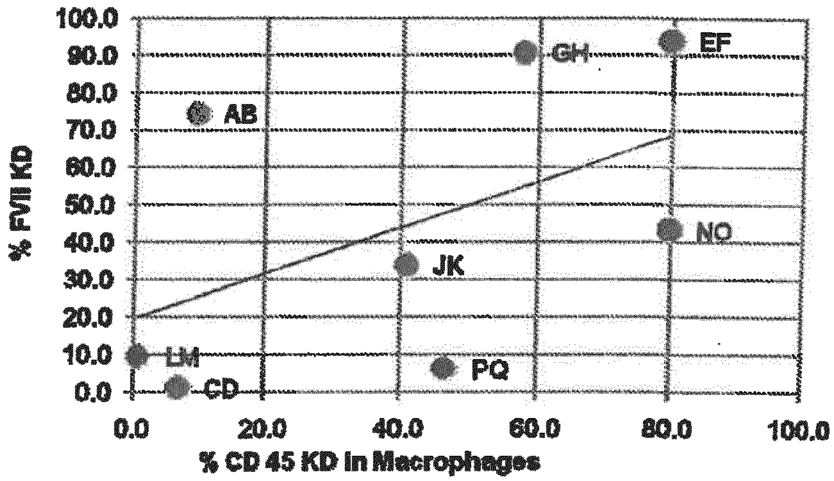


FIG. 22B

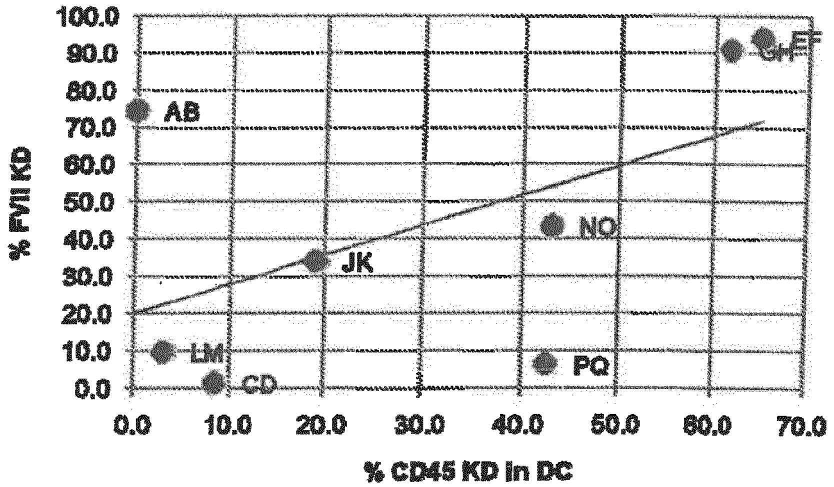

FIG. 23A

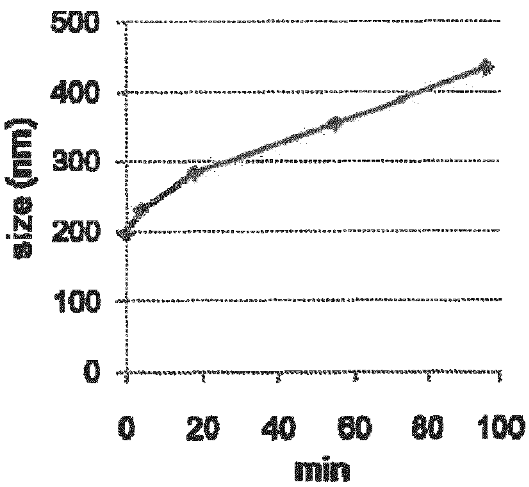


FIG. 23B

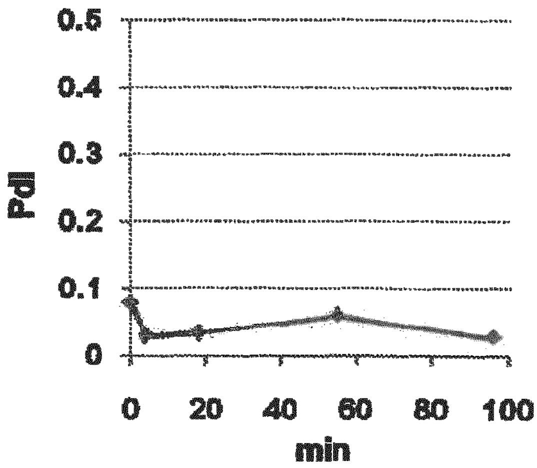


FIG. 23C

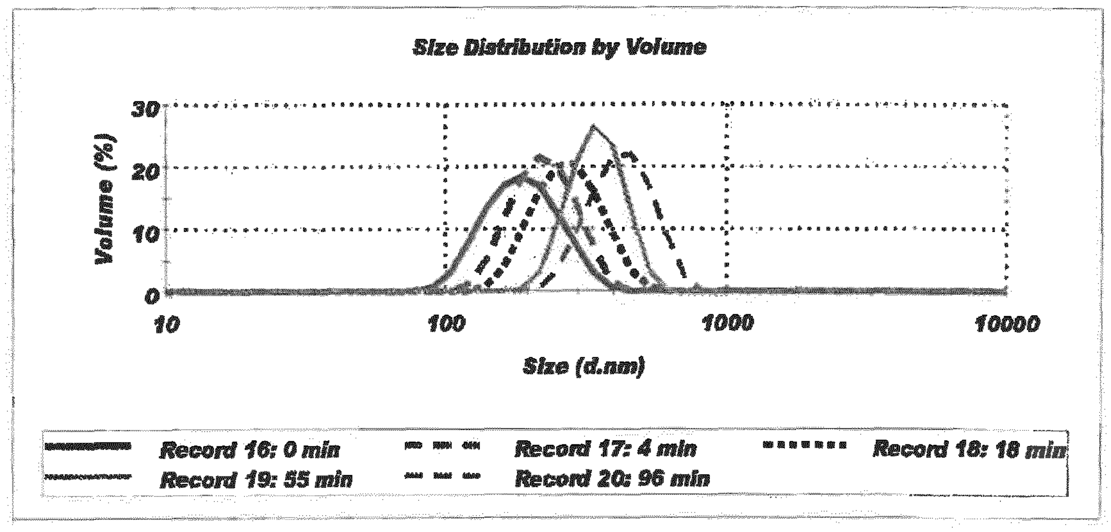

FIG. 24A

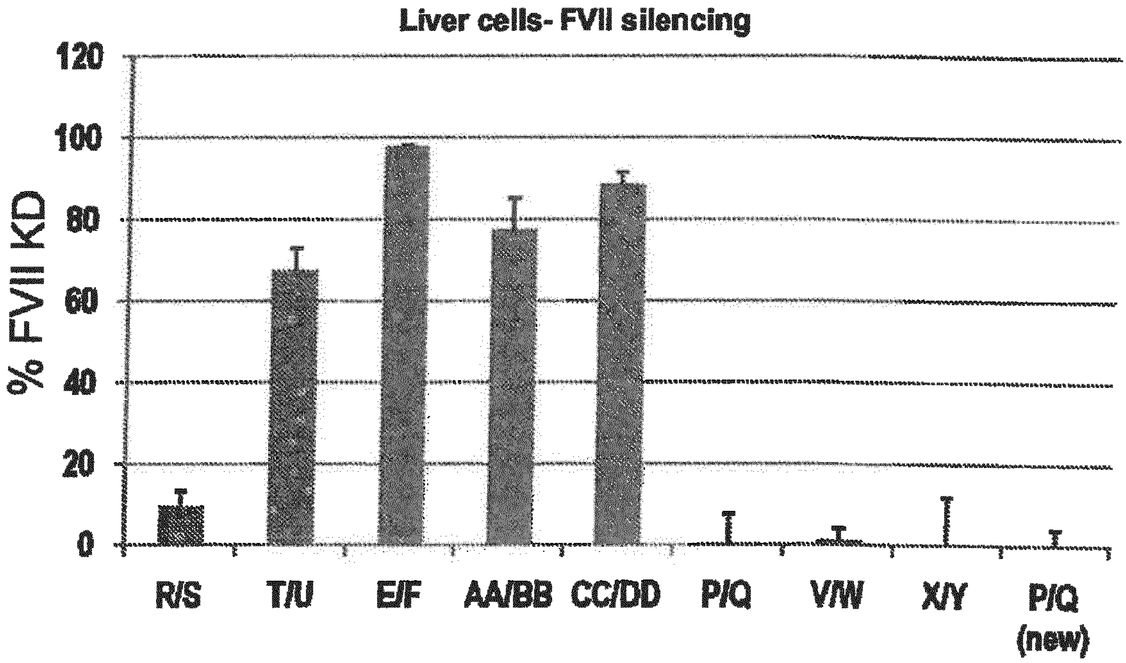


FIG. 24B

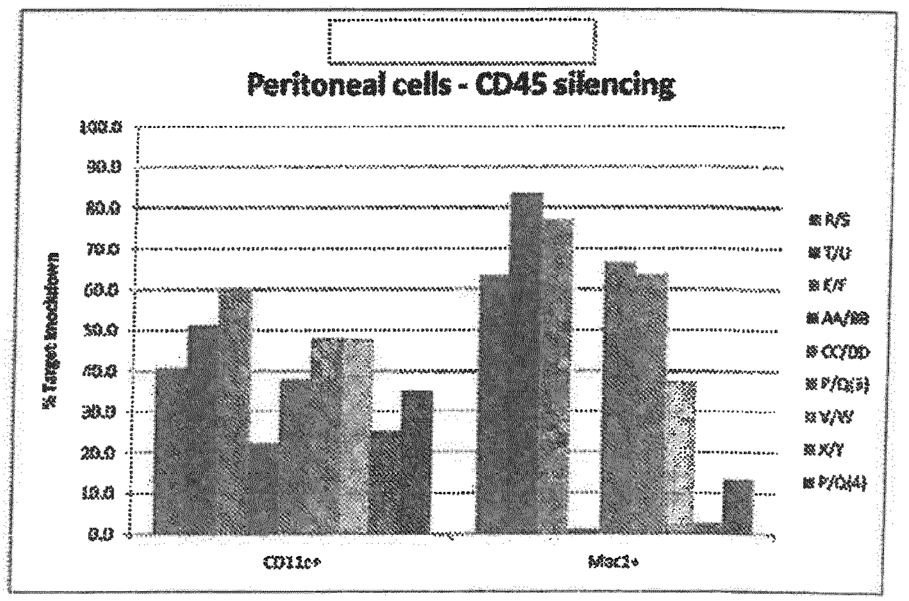

FIG. 24C

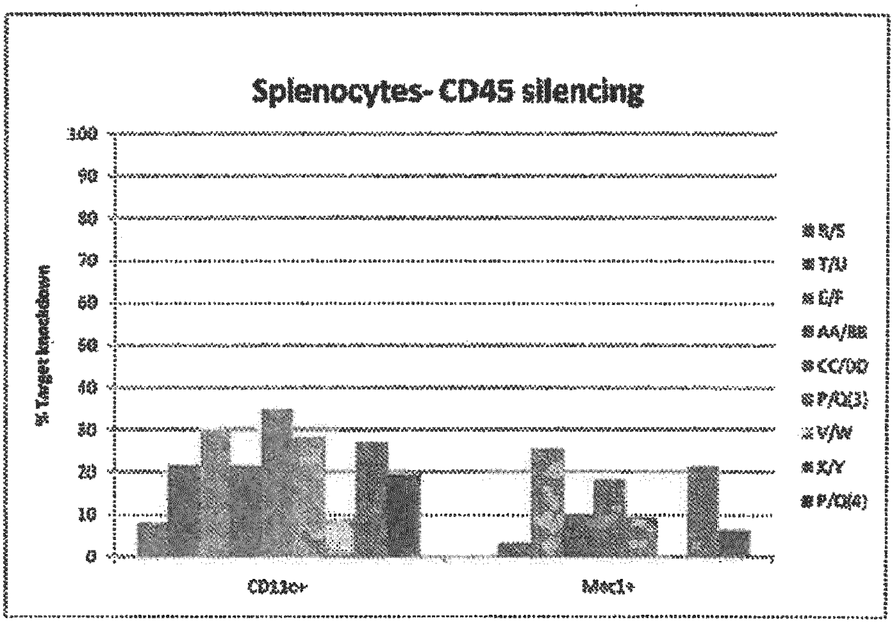


FIG. 24D

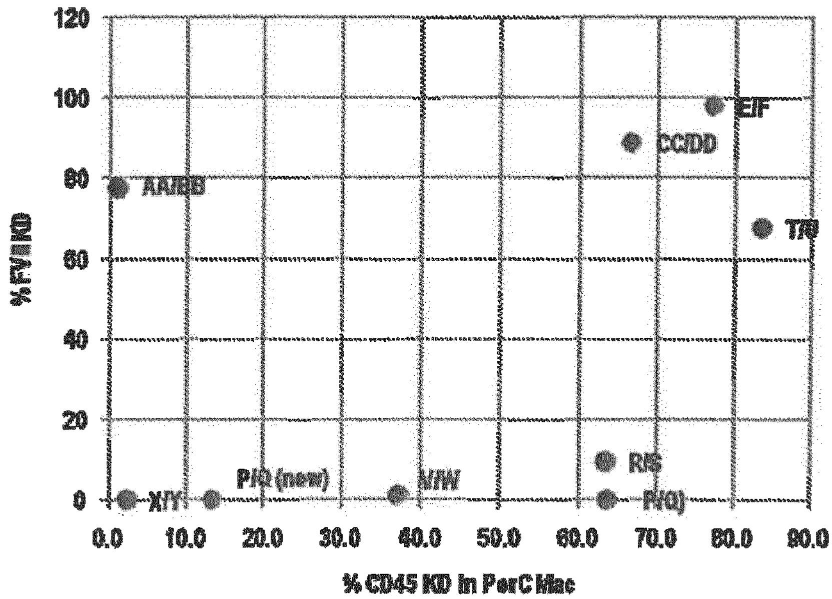

FIG. 25A

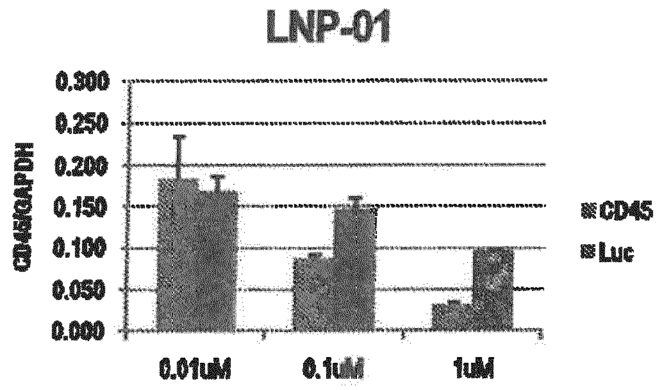


FIG. 25B

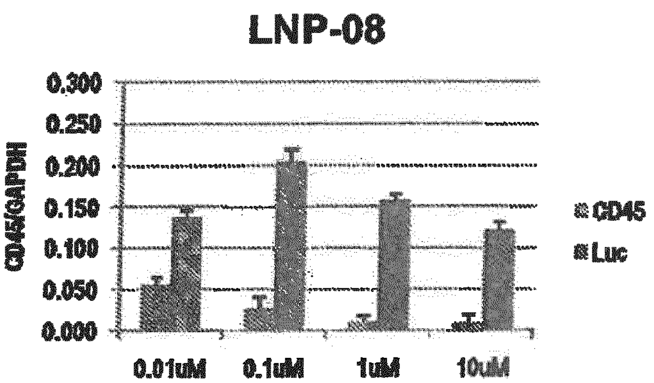

FIG. 26

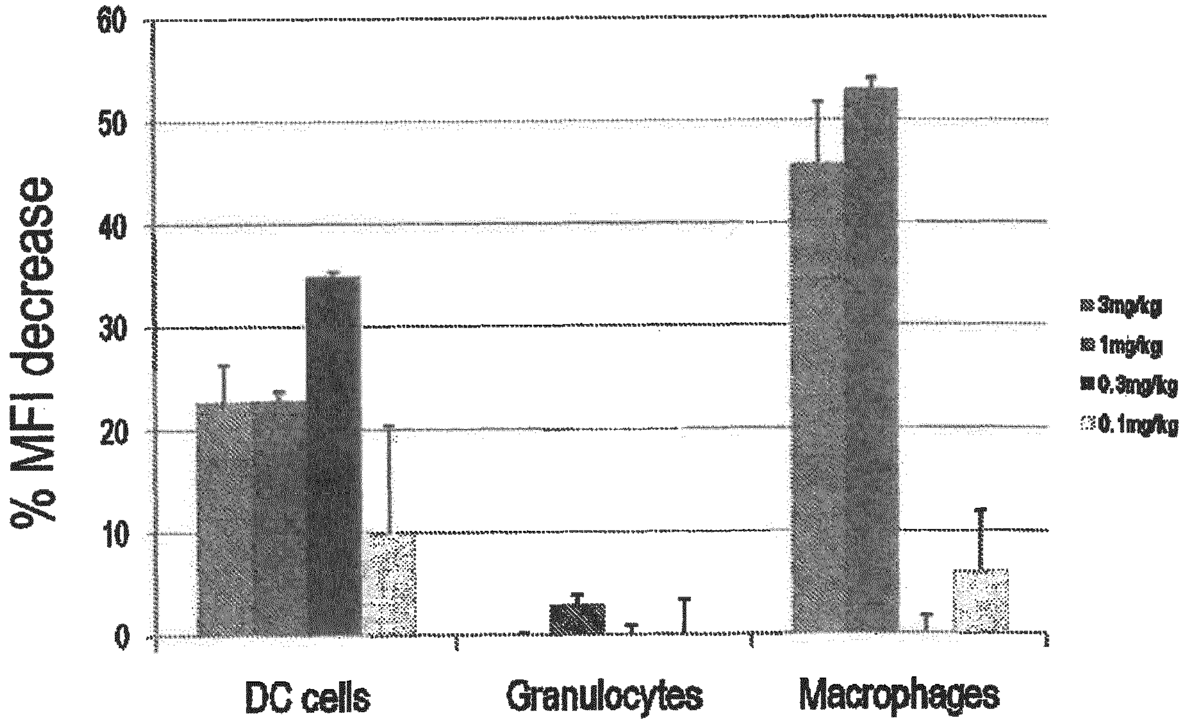
60
50
40
30
20
10
0
% MFI decrease
DC cells
Granulocytes
Macrophages
3mg/kg
1mg/kg
0.3mg/kg
0.1mg/kg

# FIG. 27A

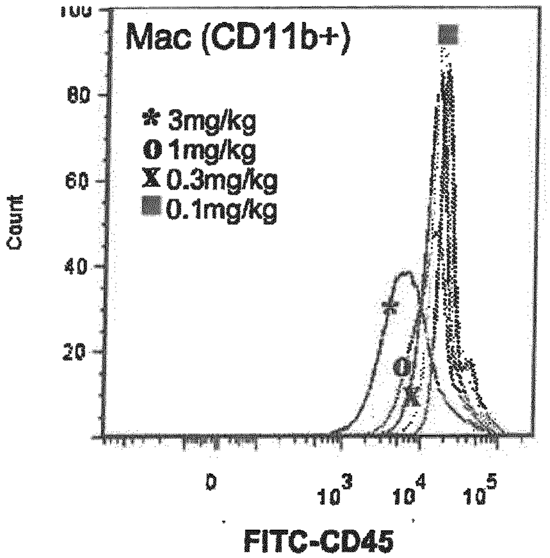


# FIG. 27B

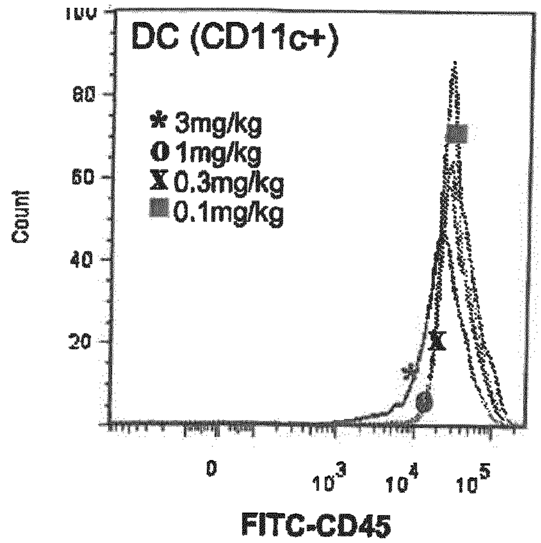


# FIG. 27C

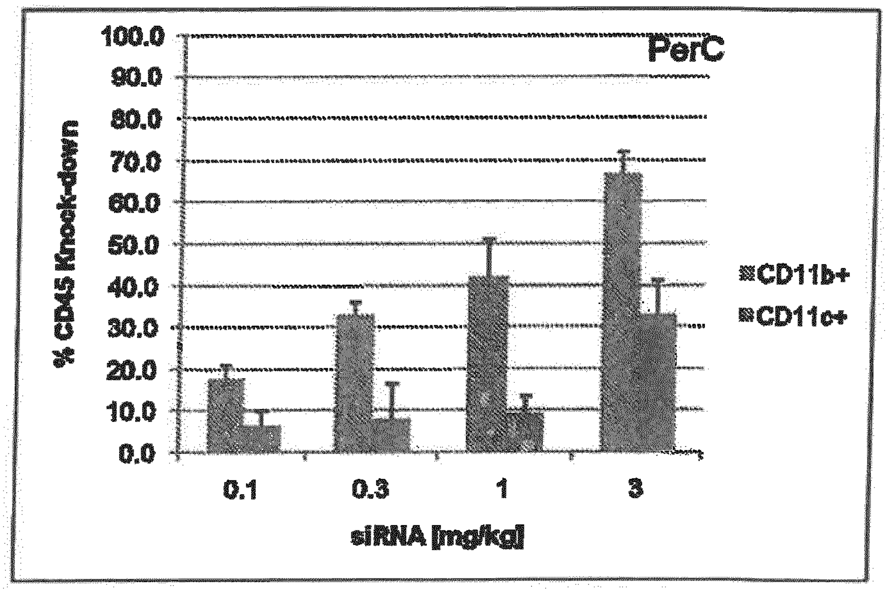

FIG. 28A. Peritoneal Cavity

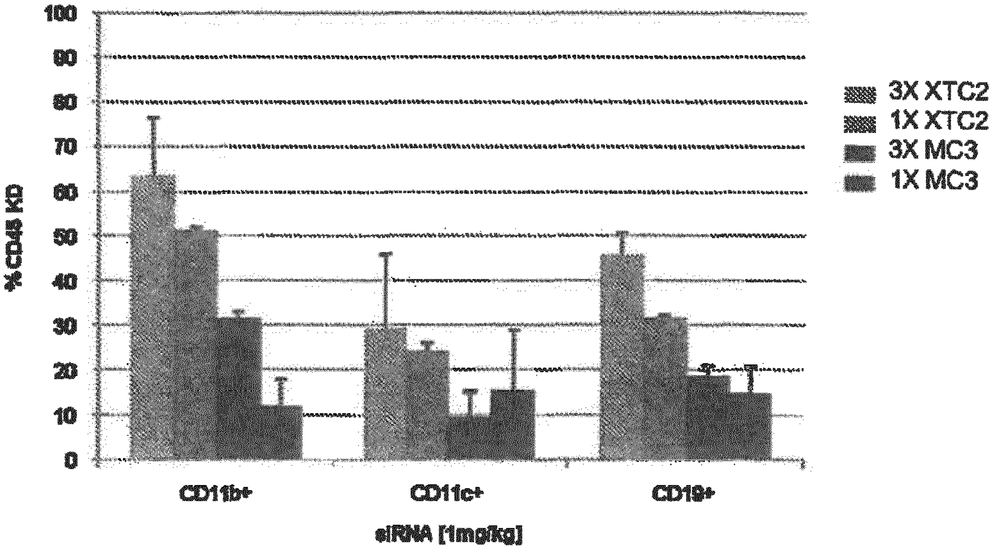


FIG. 28B. Spleen

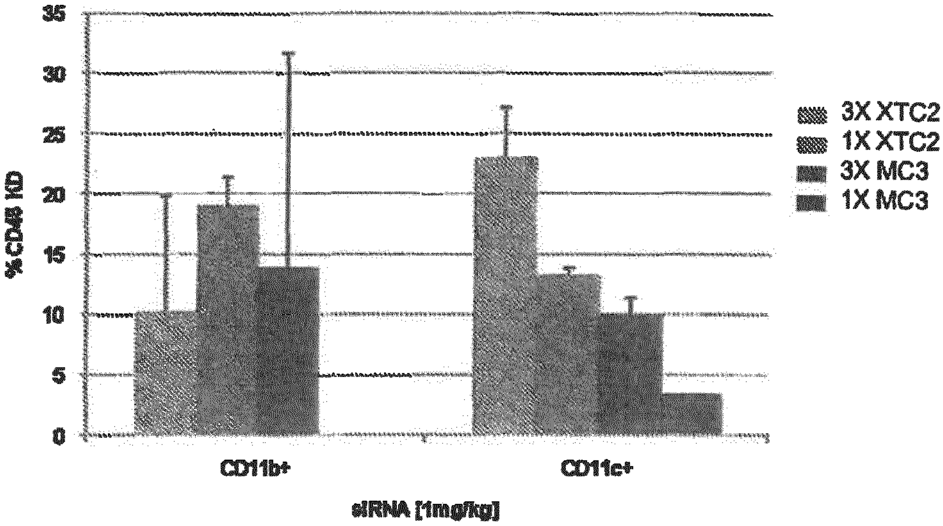

FIG. 28C. Bone Marrow

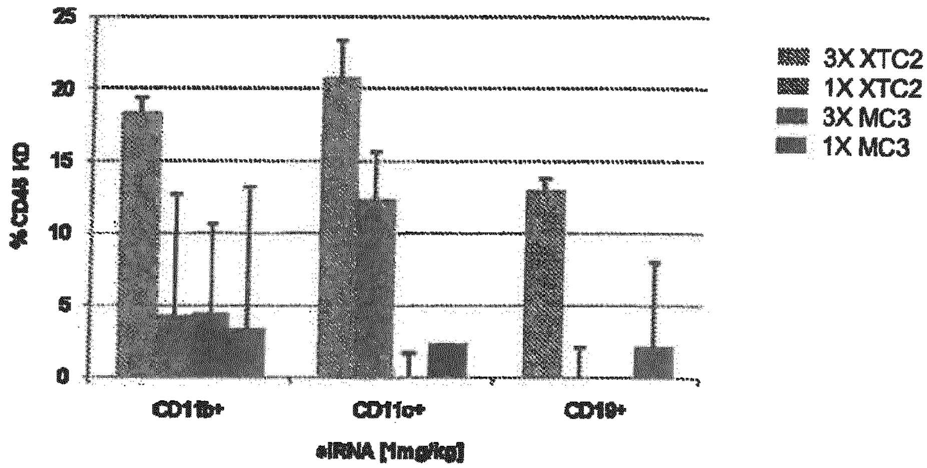


FIG. 28D. Liver

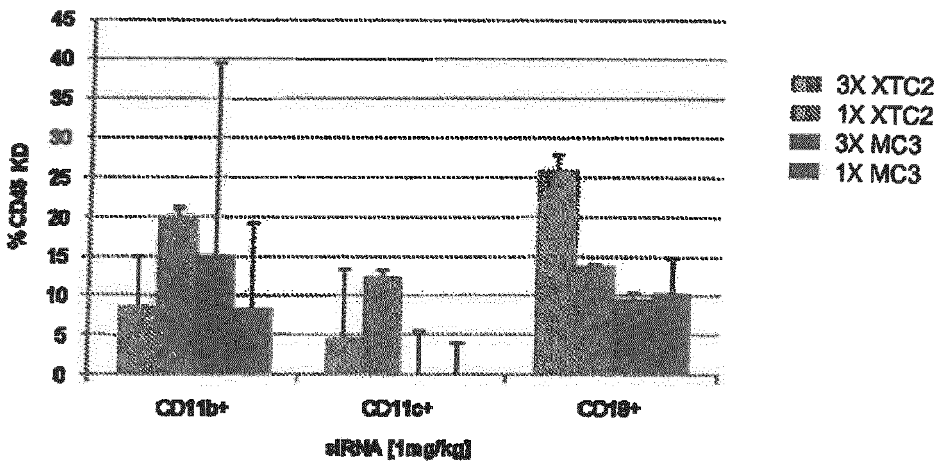

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61175777
- US 61234045
- US 61242761
- US 61251991
- US 61258848
- US 61185800 B
- US 2009063933 W
- US 2009063931 W
- US 2009063927 W
- US 201022614 W
- US 61299291 B
- US 5008050 A
- US 4927637 A
- US 4737323 A
- US 6534018 B
- US 6855277 B
- US 20070042031 A
- US 61171439 B
- US 61156851 B
- US 61175770 B
- US 61225898 B
- US 5286634 A, Stadler
- US 3993754 A, Rahman
- US 4145410 A, Sears
- US 4235871 A, Papahadjopoulos
- US 4224179 A, Schneider
- US 4522803 A, Lenk
- US 4588578 A, Fountain
- US 5739119 A
- US 5759829 A
- US 5801154 A
- US 5789573 A
- US 5718709 A
- US 5610288 A
- US 5747470 A
- US 5591317 A
- US 5783683 A
- EP 0360257 A
- US 5631359 A
- US 4987071 A
- WO 9323569 A
- WO 9402595 A
- WO 9207065 A
- WO 9315187 A
- WO 9103162 A
- WO 92110298 A
- US 5334711 A
- WO 9413688 A
- US 379343
- US 649527
- WO 02069369 A
- WO 0115726 A
- US 6406705 B
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5587361 A
- US 5625050 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5470967 A
- US 5489677 A
- US 5541307 A
- US 5561225 A
- US 5596086 A
- US 5602240 A
- US 5610289 A
- US 5608046 A
- US 5618704 A
- US 5623070 A

- US 5663312 A
- US 5633360 A
- US 5677437 A
- US 5677439 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5681941 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5700920 A
- US 5539082 A
- US 5714331 A
- US 5719262 A
- US 6320017 B
- US 5885613 A
- US 5820873 A
- US 5534499 A
- US 08486214 B
- US 486214
- US 4957773 A
- US 4603044 A
- US 5013556 A
- US 6027726 A
- US 5032401 A
- US 5607677 A
- US 20050281781 A
- US 20060240093
- US 20070135372 A
- US 61045228 B
- US 31529899
- US 88682997
- US 09108673 B
- US 09256515 B
- US 09082624 B
- US 09315298 B
- WO 2008042973 A


**Non-patent literature cited in the description**


- *Biochim Biophys Acta.,* 19 October 1979, vol. 557 (1), 9-23
- *Biochim Biophys Acta,* 02 October 1980, vol. 601 (3), 559-7
- *Biochim Biophys Acta,* 13 June 1986, vol. 858 (1), 161-8
- *Biochim. Biophys. Acta,* 1985, vol. 812, 55-65
- *Pharmaceuticals Research,* March 2005, vol. 22 (3), 362-372
- **LOVE et al.** Lipid-like materials for low-dose, in vivo gene silencing. *Proc Natl Acad Sci U S A.,* 2010, vol. 107, 1864-9
- **GREEN, T.W.** PROTECTIVE GROUPS IN ORGANIC SYNTHESIS. Wiley-Interscience, 1999
- **KUNKEL et al.** *Brit. Med Bull.,* 1989, vol. 45 (3), 630-643
- **GOODFELLOW.** *Nature,* 1989, vol. 341, 102-103
- **BENNETT et al.** *Mol. Pharm.,* 1992, vol. 41, 1023-1033
- **ZHU et al.** *Science,* 1993, vol. 261, 209-211
- **HYDE et al.** *Nature,* 1993, vol. 362, 250-256
- **BRIGHAM et al.** *Am. J. Med. Sci.,* 1989, vol. 298, 278-281
- **STRAUBRINGER et al.** METHODS IN ENZYMOLOGY. Academic Press, 1983, vol. 101, 512-527
- **MANNINO et al.** *Biotechniques,* 1988, vol. 6, 682-690
- **NICOLAU et al.** *Crit. Rev. Ther. Drug Carrier Syst.,* 1989, vol. 6, 239-271
- **BEHR.** *Acc. Chem. Res.,* 1993, vol. 26, 274-278
- **BRIGHAM et al.** *Am. J. Sci.,* 1989, vol. 298 (4), 278-281
- **CULVER.** Human Gene Therapy. MaryAnn Liebert, Inc, 1994, 70-71
- The Merck Manual of Diagnosis and Therapy. 1987, 1206-1228

- The Merck Manual of Diagnosis and Therapy. 1987, 2499-2506, 46-49
- **DE FOUGEROLLES, A. et al.** *Nature Reviews,* 2007, vol. 6, 443-453
- **LAMBERTON, J.S. ; CHRISTIAN, A.T.** *Molecular Biotechnology,* 2003, vol. 24, 111-119
- **ELSHABIR, S.M. et al.** *Nature,* 2001, vol. 411, 494-498
- **CAPLEN, N. et al.** *Proc. Natl. Acad. Sci. USA,* 2001, vol. 98, 9746-9747
- **BROWN, D. et al.** *TechNotes,* vol. 9 (1), 1-7
- **ELSHABIR, S.M. et al.** *EMBO,* 2001, vol. 20, 6877-6888
- **ELSHABIR, S. et al.** *Nature,* 2001, vol. 411, 494-498
- **ELSHABIR, S. et al.** *EMBO J.,* 2001, vol. 20, 6877-6888
- **PADDISON, P. et al.** *Genes Dev.,* 2002, vol. 16 (8), 948-58
- **PADDISON, P. et al.** *Proc. Natl. Acad. Sci. USA,* 2002, vol. 99 (3), 1443-1448
- **PADDISON et al.** *Genes & Dev.,* 2002, vol. 16 (8), 948-58
- **GRIFFITHS-JONES S ; GROCOCK RJ ; VAN DONGEN S ; BATEMAN A ; ENRIGHT AJ. NAR.** *miRBase: microRNA sequences, targets and gene nomenclature,* 2006, vol. 34, D140-D144
- **GRIFFITHS-JONES S. NAR.** *The microRNA Registry,* 2004, vol. 32, D109-D111
- **JASKULSKI et al.** *Science,* 10 June 1988, vol. 240 (4858), 1544-6
- **VASANTHAKUMAR ; AHMED.** *Cancer Commun.,* 1989, vol. 1 (4), 225-32
- **PERIS et al.** *Brain Res Mol Brain Res,* 15 June 1998, vol. 57 (2), 310-20
- **ALTSCHUL et al.** *Nucleic Acids Res.,* 1997, vol. 25 (17), 3389-402
- **KIM ; CECH.** *Proc Nat1 Acad Sci U S A,* December 1987, vol. 84 (24), 8788-92
- *Forster and Symons, Cell.,* 24 April 1987, vol. 49 (2), 211-20
- **CECH et al.** *Cell,* December 1981, vol. 27 (3), 487-96
- **MICHEL ; WESTHOF.** *J Mol Biol.,* 05 December 1990, vol. 216 (3), 585-610
- **REINHOLD-HUREK ; SHUB.** *Nature,* 14 May 1992, vol. 357 (6374), 173-6
- **ROSSI et al.** *Nucleic Acids Res.,* 11 September 1992, vol. 20 (17), 4559-65
- **HAMPEL ; TRITZ.** *Biochemistry,* 13 June 1989, vol. 28 (12), 4929-33
- **HAMPEL et al.** *Nucleic Acids Res.,* 25 January 1990, vol. 18 (2), 299-304
- **PERROTTA ; BEEN.** *Biochemistry,* 01 December 1992, vol. 31 (47), 11843-52
- **GUERRIER-TAKADA et al.** *Cell,* December 1983, vol. 35 (3), 849-57
- **SAVILLE ; COLLINS.** *Cell,* 18 May 1990, vol. 61 (4), 685-96
- **SAVILLE ; COLLINS.** *Proc Natl Acad Sci U S A.,* 01 October 1991, vol. 88 (19), 8826-30
- **COLLINS ; OLIVE.** *Biochemistry,* 23 March 1993, vol. 32 (11), 2795-9
- **RANEY et al.** *Journal of Pharmacology and Experimental Therapeutics,* 2001, vol. 298, 1185-1192
- **KURRECK, J.** Antisense technologies. Improvement through novel chemical modifications. *Eur J Biochem,* 2003, vol. 270, 1628-44
- **KURRECK.** *Eur. J. Biochem.,* 2003, vol. 270, 1628-44
- **HALL et al.** *Nucleic Acids Res.,* 2004, vol. 32, 5991-6000
- **ZHANG et al.** *Curr. Top. Med. Chem.,* 2006, vol. 6, 893-900
- Antisense Research and Applications. CRC Press, 1993, 276-278
- **MANOHARAN.** *Curr. Opin. Chem. Biol.,* 2004, vol. 8, 570-9
- **HOLEN et al.** *Nucleic Acids Res,* 2003, vol. 31, 2401-7
- **PRAKASH et al.** *J. Med. Chem,* 2005, vol. 48, 4247-53
- **MARTIN et al.** *Helv. Chim. Acta,* 1995, vol. 78, 486-504
- **NIELSEN et al.** *Science,* 1991, vol. 254, 1497-1500
- **FELGNER.** *Scientific American*
- **SAPRA, P. ; ALLEN, TM.** *Prog. Lipid Res.,* 2003, vol. 42 (5), 439-62
- **ABRA, RM et al.** *J. Liposome Res.,* 2002, vol. 12, 1-3
- **ALLEN et al.** *Biochimica et Biophysica Acta,* 1995, vol. 1237, 99-108
- **DEFREES et al.** *Journal of the American Chemistry Society,* 1996, vol. 118, 6101-6104
- **BLUME et al.** *Biochimica et Biophysica Acta,* 1993, vol. 1149, 180-184
- **KLIBANOV et al.** *Journal of Liposome Research,* 1992, vol. 2, 321-334
- **ZALIPSKY.** *Bioconjugate Chemistry,* 1993, vol. 4, 296-299
- **ZALIPSKY.** *FEBS Letters,* 1994, vol. 353, 71-74
- **ZALIPSKY.** Stealth Liposomes. CRC Press, 1995
- **KLIBANOV et al.** Journal of Liposome Research. 1992, vol. 2, 321-334
- **KIRPOTIN et al.** *FEBS Letters,* 1996, vol. 388, 115-118
- **RENNEISEN et al.** *J. Bio. Chem.,* 1990, vol. 265, 16337-16342
- **LEONETTI et al.** *Proc. Natl Acad. Sci. (USA),* 1990, vol. 87, 2448-2451

- **HEATH.** Covalent Attachment of Proteins to Liposomes, 149 Methods in Enzymology. Academic Press, Inc, 1987, 111-119
- **HEINJE, G.** Sequence Analysis in Molecular Biology. Academic Press, 1987
- Sequence Analysis Primer. M. Stockton Press, 1991